# EUROPEAN PATENT APPLICATION

(11) **EP 4 711 372 A1**
(43) Date of publication of application: **18.03.2026**
(21) Application number: 24802953.0
(22) Date of filing: 07.05.2024
(51) Int. Cl.: C07D 519/00, A61K 31/4375, A61P 1/00

(54) **CRYSTAL FORM OF HETEROCYCLIC COMPOUND, SALT THEREOF, CRYSTAL FORM OF SALT THEREOF, AND USE**

(30) Priority: 08.05.2023 CN 202310513152; 23.04.2024 CN 202410496830
(71) Applicant: Hubei Bio-Pharmaceutical Industrial Technological Institute Inc., Wuhan, Hubei 430075 (CN)
(72) Inventor: ZHANG, Xuejun, Wuhan, Hubei 430075 (CN); ZANG, Yang, Wuhan, Hubei 430075 (CN); LI, Qun, Wuhan, Hubei 430075 (CN); ZONG, Qiao, Wuhan, Hubei 430075 (CN); WANG, Meng, Wuhan, Hubei 430075 (CN); QIAN, Lina, Wuhan, Hubei 430075 (CN)
(74) Representative: Haseltine Lake Kempner LLP
(86) International application number: PCT/CN2024/091386
(87) International publication number: WO 2024/230672

(57) **Abstract**

Disclosed in the present invention are a crystal form of a heterocyclic compound, a salt thereof, a crystal form of the salt thereof, and a use. Disclosed in the present invention are a pharmaceutically acceptable salt of a heterocyclic compound or a solvate thereof, wherein the heterocyclic compound is a compound represented by formula I-3A and/or a compound represented by formula I-3B. The crystal form of a heterocyclic compound, the salt thereof, and the crystal form of the salt thereof disclosed in the present invention can be used for drug preparation, and have wide application prospects.

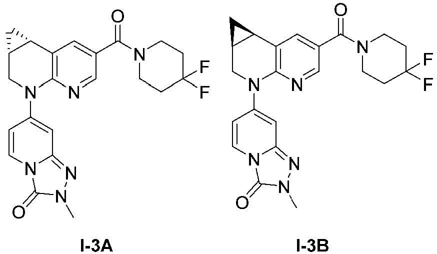

## Description

The present application claims the priority to Chinese patent application 2023105131520 filed on May 8, 2023. The present application claims the priority to Chinese patent application 2024104968301 filed on April 23, 2024. The contents of the above Chinese patent applications are incorporated herein by reference in their entirety.

### TECHNICAL FIELD

The present disclosure belongs to the field of medicinal chemistry, and specifically relates to a crystal form of a heterocyclic compound, a salt thereof, a crystal form of the salt thereof, and a use.

### BACKGROUND

The 15-hydroxyprostaglandin dehydrogenase (15-PGDH) gene spans approximately 31 kb on the 4q34-q35 of chromosome 4 and contains 7 exons with a molecular weight of 29 kD. 15-PGDH contains 266 amino acids and belongs to the short-chain dehydrogenases (SDR) family, which is a dimer composed of two identical subunits, but is believed to only have enzymatic activity when present as a monomer. 15-PGDH is a key enzyme in the degradation and inactivation of prostaglandins (PGs) and related eicosanoids, which is widely found in normal tissues such as lungs, kidneys, gastrointestinal tract, thyroid, prostate, and placenta in humans and mammals. On the one hand, 15-PGDH can catalyze the oxidation of active 15-hydroxyprostaglandin into 15-keto-prostaglandin with greatly reduced activity, and on the other hand, it can degrade a number of other non-prostaglandin polycyclic aromatic hydrocarbons in the presence of NAD⁺ coenzyme factors, reducing carcinogens and procarcinogens produced under physiological or pathological conditions through oxidative reactions.

Chinese patent application 202211449273.5 discloses a heterocyclic compound with 15-PGDH inhibitory activity:

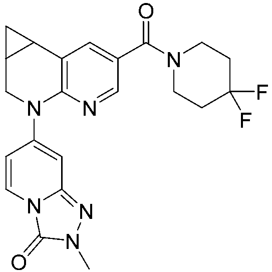

The heterocyclic compound, known under the chemical name of 7-(6-(4,4-difluoropiperidine-1-carbonyl)-1,1a,2,7b-tetrahydro-3*H*-cyclopropa[*c*][1,8]naphthyridin-3-yl)-2-methyl-[1,2,4]triazolo[4,3-*a*]pyridin-3(2*H*)-one (I-3), contains the following two isomers:

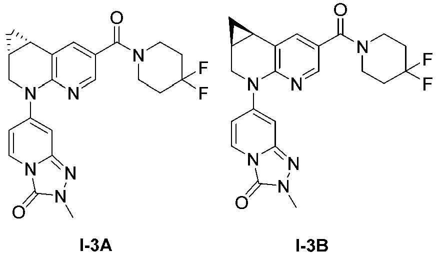

On the basis of the favorable biological activity of the compound, it is necessary to develop suitable salt forms and solid forms thereof to achieve improved druggability or other properties.

### SUMMARY

The present disclosure provides a crystal form of a heterocyclic compound, a salt thereof, a crystal form of the salt thereof, and a use. The salt of the heterocyclic compound as well as the solid form of the heterocyclic compound or the salt thereof provided by the present disclosure can be used for drug preparation and have good application prospects.

The present disclosure provides a pharmaceutically acceptable salt of a heterocyclic compound or a solvate thereof (which refers to a solvate of a pharmaceutically acceptable salt of a heterocyclic compound),
wherein the heterocyclic compound is a compound represented by formula I-3A and/or a compound represented by formula I-3B; 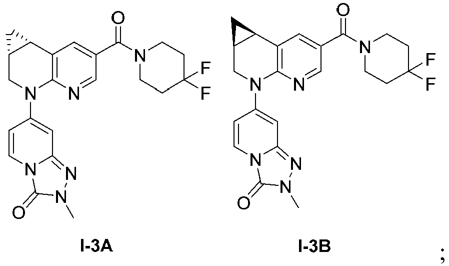
wherein the pharmaceutically acceptable salt is a salt formed by the heterocyclic compound with an acid; the acid is selected from the group consisting of hydrochloric acid, phosphoric acid, fumaric acid, tartaric acid, malic acid, ethanedisulfonic acid, *p-*toluenesulfonic acid, methanesulfonic acid, benzenesulfonic acid, and oxalic acid.

In a certain embodiment, in the solvate of the pharmaceutically acceptable salt of the heterocyclic compound, the solvent is one or two selected from the group consisting of isopropyl acetate, acetone, isopropanol, and n-heptane.

In a certain embodiment, the heterocyclic compound is a compound represented by formula I-3A and/or a compound represented by formula I-3B:

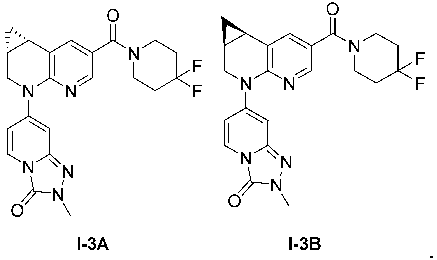

In a certain embodiment, when the heterocyclic compound is a compound represented by formula I-3A and a compound represented by formula I-3B, the heterocyclic compound is a compound represented by formula I-3:

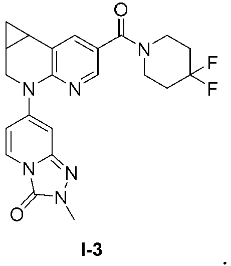

In a certain embodiment, the heterocyclic compound is a compound represented by formula I-3B:

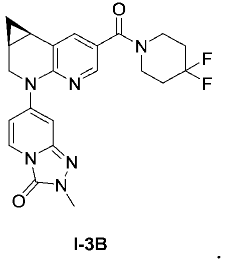

In a certain embodiment, in the pharmaceutically acceptable salt of the heterocyclic compound or the solvate thereof, the heterocyclic compound and the acid have a molar ratio of 1:(0.5 to 2); for example, 1:(0.5 to 1); for another example, 1:1, 1:0.9, or 1:0.5.

In a certain embodiment, in the solvate of the pharmaceutically acceptable salt of the heterocyclic compound, the heterocyclic compound and the solvent have a molar ratio of 1:(0.001 to 2); for example, 1:(0.001 to 1); for another example, 1:0.05, 1:0.9, 1:0.01, 1:0.04, 1:0.008, 1:0.03, 1:0.3, or 1:0.5.

In a certain embodiment, the pharmaceutically acceptable salt of the heterocyclic compound or the solvate thereof is:
an acetone solvate of a hydrochloride salt of the compound represented by formula I-3B, wherein hydrochloric acid, the compound represented by formula I-3B, and acetone have a molar ratio of 0.9:1:0.9;
or, a phosphate salt of the compound represented by formula I-3B, wherein phosphoric acid and the compound represented by formula I-3B have a molar ratio of 1:1;
or, an acetone solvate of a phosphate salt of the compound represented by formula I-3B, wherein phosphoric acid, the compound represented by formula I-3B, and acetone have a molar ratio of 1:1:0.05;
or, a fumarate salt of the compound represented by formula I-3B, wherein fumaric acid and the compound represented by formula I-3B have a molar ratio of 1:1;
or, an L-tartrate salt of the compound represented by formula I-3B, wherein tartaric acid and the compound represented by formula I-3B have a molar ratio of 1:1;
or, an L-malate salt of the compound represented by formula I-3B, wherein malic acid and the compound represented by formula I-3B have a molar ratio of 1:1;
or, an isopropanol solvate of an ethanedisulfonate salt of the compound represented by formula I-3B, wherein ethanedisulfonic acid, the compound represented by formula I-3B, and isopropanol have a molar ratio of 0.5:1:0.5;
or, an ethanedisulfonate salt of the compound represented by formula I-3B, wherein ethanedisulfonic acid and the compound represented by formula I-3B have a molar ratio of 0.5:1;
or, an ethanedisulfonate salt of the compound represented by formula I-3B, wherein ethanedisulfonic acid and the compound represented by formula I-3B have a molar ratio of 1:1;
or, an acetone/n-heptane solvate of an ethanedisulfonate salt of the compound represented by formula I-3B, wherein ethanedisulfonic acid, the compound represented by formula I-3B, acetone, and n-heptane have a molar ratio of 1:1:0.3:0.03;
or, a p-toluenesulfonate salt of the compound represented by formula I-3B, wherein p-toluenesulfonic acid and the compound represented by formula I-3B have a molar ratio of 1:1;
or, a methanesulfonate salt of the compound represented by formula I-3B, wherein methanesulfonic acid and the compound represented by formula I-3B have a molar ratio of 1:1;
or, an n-heptane solvate of a methanesulfonate salt of the compound represented by formula I-3B, wherein methanesulfonic acid, the compound represented by formula I-3B, and n-heptane have a molar ratio of 1:1:0.008;
or, a benzenesulfonate salt of the compound represented by formula I-3B, wherein benzenesulfonic acid and the compound represented by formula I-3B have a molar ratio of 1:1;
or, an isopropanol solvate of a benzenesulfonate salt of the compound represented by formula I-3B, wherein benzenesulfonic acid, the compound represented by formula I-3B, and isopropanol have a molar ratio of 1:1:0.01;
or, an oxalate salt of the compound represented by formula I-3B, wherein oxalic acid and the compound represented by formula I-3B have a molar ratio of 1:1.

In a certain embodiment, the solvate of the pharmaceutically acceptable salt of the heterocyclic compound is not a monohydrochloride monohydrate of the heterocyclic compound.

The present disclosure provides a crystal form A of the compound represented by formula I-3B, which has an X-ray powder diffraction pattern using Cu-Kα radiation and expressed by 20 angles comprising diffraction peaks at 15.2 ± 0.2°, 22.8 ± 0.2°, 19.1 ± 0.2°, 18.6 ± 0.2°, and 21.6 ± 0.2°;
the compound represented by formula I-3B has a structure of

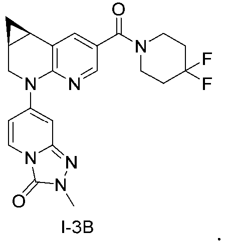

In a certain embodiment, the X-ray powder diffraction pattern for the crystal form A of the compound represented by formula I-3B, using Cu-Kα radiation and expressed by 20 angles, further comprises diffraction peaks at one or more of 23.1 ± 0.2°, 17.6 ± 0.2°, 11.9 ± 0.2°, 24.9 ± 0.2°, and 20.1 ± 0.2°.

In a certain embodiment, the X-ray powder diffraction pattern for the crystal form A of the compound represented by formula I-3B, using Cu-Kα radiation and expressed by 20 angles, comprises diffraction peaks at 15.2 ± 0.2°, 22.8 ± 0.2°, 19.1 ± 0.2°, 17.6 ± 0.2°, 21.6 ± 0.2°, 18.6 ± 0.2°, and 11.9 ± 0.2°.

In a certain embodiment, the X-ray powder diffraction pattern for the crystal form A of the compound represented by formula I-3B, using Cu-Kα radiation and expressed by 20 angles, comprises diffraction peaks at 15.2 ± 0.2°, 22.8 ± 0.2°, 19.1 ± 0.2°, 17.6 ± 0.2°, 21.6 ± 0.2°, 23.1 ± 0.2°, 18.6 ± 0.2°, 11.9 ± 0.2°, 24.9 ± 0.2°, and 20.1 ± 0.2°.

In a certain embodiment, the X-ray powder diffraction pattern for the crystal form A of the compound represented by formula I-3B, using Cu-Kα radiation and expressed by 20 angles, comprises diffraction peaks shown in Table 1.

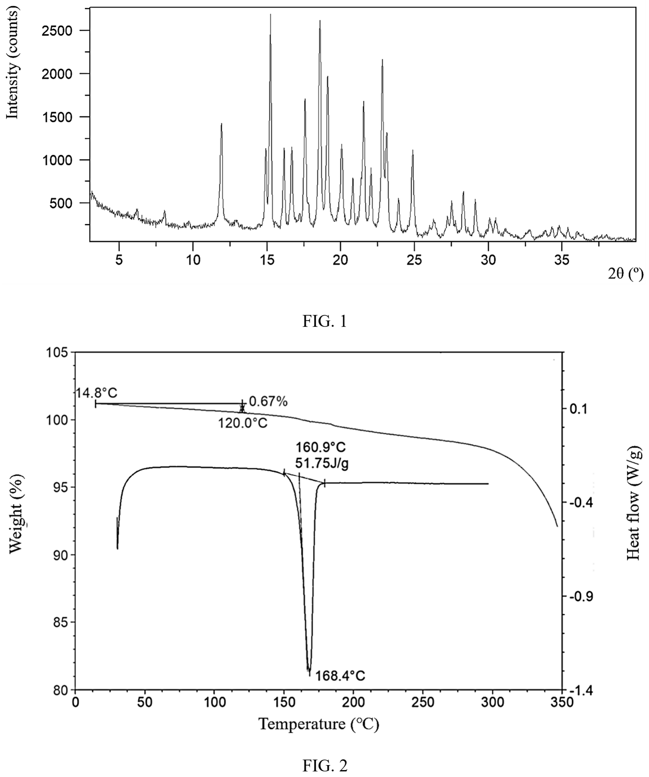

In a certain embodiment, the X-ray powder diffraction (XRPD) pattern for the crystal form A of the compound represented by formula I-3B using Cu-Kα radiation is substantially shown in FIG. 1.

In a certain embodiment, the crystal form A of the compound represented by formula I-3B has a differential scanning calorimetry (DSC) curve comprising an endothermic peak with an onset temperature at 160.9 ± 3°C, and/or the crystal form A of the compound represented by formula I-3B has a differential scanning calorimetry (DSC) curve comprising an endothermic peak with a peak temperature at 168.4 ± 3°C.

In a certain embodiment, the differential scanning calorimetry (DSC) curve for the crystal form A of the compound represented by formula I-3B is substantially shown in FIG. 2.

In a certain embodiment, the crystal form A of the compound represented by formula I-3B has a thermogravimetric analysis (TGA) curve with a weight loss of approximately 0.67% in the temperature range of 14.8 ± 3°C to 120 ± 3°C.

In a certain embodiment, the thermogravimetric analysis (TGA) curve for the crystal form A of the compound represented by formula I-3B is substantially shown in FIG. 2.

In a certain embodiment, the crystal form A of the compound represented by formula I-3B is a crystal form of an isopropyl acetate solvate of the compound represented by formula I-3B, wherein the compound represented by formula I-3B and the isopropyl acetate have a molar ratio of 1:0.04.

The present disclosure provides a crystal form B of the compound represented by formula I-3B, which has an X-ray powder diffraction pattern using Cu-Kα radiation and expressed by 20 angles comprising diffraction peaks at 18.5 ± 0.2°, 24.8 ± 0.2°, 21.6 ± 0.2°, 20.9 ± 0.2°, and 14.4 ± 0.2°.

In a certain embodiment, the X-ray powder diffraction pattern for the crystal form B of the compound represented by formula I-3B, using Cu-Kα radiation and expressed by 20 angles, further comprises diffraction peaks at one or more of 14.9 ± 0.2°, 19.2 ± 0.2°, 17.7 ± 0.2°, 17.1 ± 0.2°, and 15.2 ± 0.2°.

In a certain embodiment, the X-ray powder diffraction pattern for the crystal form B of the compound represented by formula I-3B, using Cu-Kα radiation and expressed by 20 angles, comprises diffraction peaks at 18.5 ± 0.2°, 24.8 ± 0.2°, 21.6 ± 0.2°, 20.9 ± 0.2°, 14.4 ± 0.2°, 14.9 ± 0.2°, and 19.2 ± 0.2°.

In a certain embodiment, the X-ray powder diffraction pattern for the crystal form B of the compound represented by formula I-3B, using Cu-Kα radiation and expressed by 20 angles, comprises diffraction peaks at 18.5 ± 0.2°, 24.8 ± 0.2°, 21.6 ± 0.2°, 20.9 ± 0.2°, 14.4 ± 0.2°, 14.9 ± 0.2°, 19.2 ± 0.2°, 17.7 ± 0.2°, 17.1 ± 0.2°, and 15.2 ± 0.2°.

In a certain embodiment, the X-ray powder diffraction pattern for the crystal form B of the compound represented by formula I-3B, using Cu-Kα radiation and expressed by 20 angles, comprises diffraction peaks shown in Table 2.

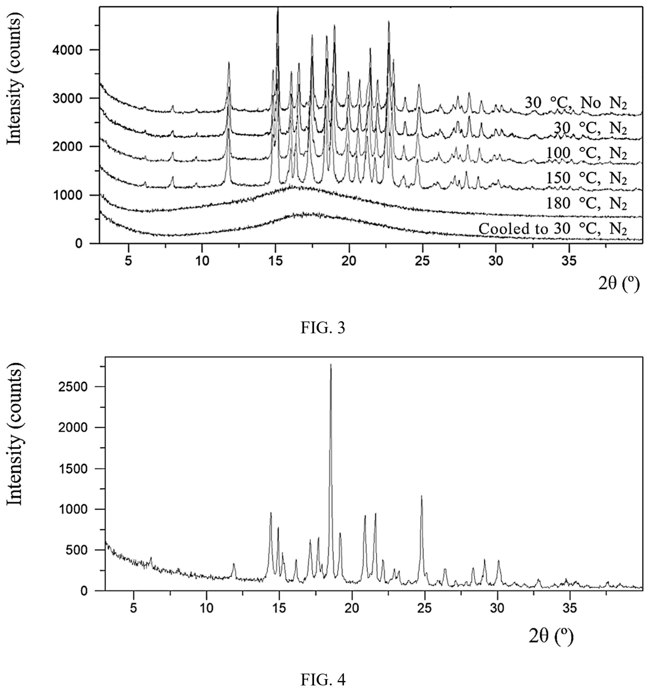

In a certain embodiment, the X-ray powder diffraction (XRPD) pattern for the crystal form B of the compound represented by formula I-3B using Cu-Kα radiation is substantially shown in FIG. 4.

In a certain embodiment, the crystal form B of the compound represented by formula I-3B has a differential scanning calorimetry (DSC) curve comprising an endothermic peak with an onset temperature at 164.2 ± 3°C, and/or the crystal form B of the compound represented by formula I-3B has a differential scanning calorimetry (DSC) curve comprising an endothermic peak with a peak temperature at 171.7 ± 3°C.

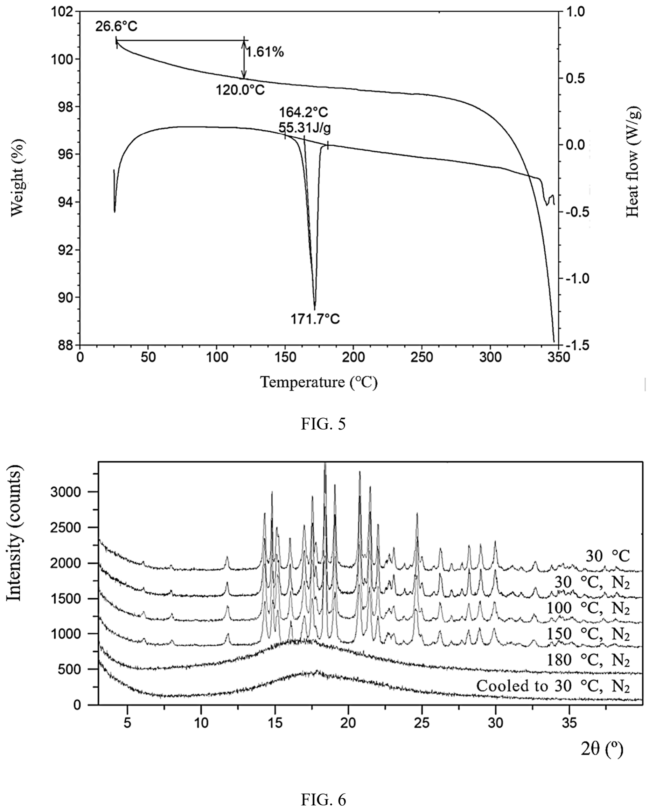

In a certain embodiment, the differential scanning calorimetry (DSC) curve for the crystal form B of the compound represented by formula I-3B is substantially shown in FIG. 5.

In a certain embodiment, the crystal form B of the compound represented by formula I-3B has a thermogravimetric analysis (TGA) curve with a weight loss of approximately 1.61% in the temperature range of 26.6 ± 3°C to 120 ± 3°C.

In a certain embodiment, the thermogravimetric analysis (TGA) curve for the crystal form B of the compound represented by formula I-3B is substantially shown in FIG. 5.

The present disclosure provides a crystal form of the hydrochloride salt of the compound represented by formula I-3B, which has an X-ray powder diffraction pattern using Cu-Kα radiation and expressed by 2θ angles comprising diffraction peaks at 9.5 ± 0.2°, 24.8 ± 0.2°, 17.3 ± 0.2°, 19.3 ± 0.2°, and 11.7 ± 0.2°.

In a certain embodiment, in the crystal form of the hydrochloride salt of the compound represented by formula I-3B, the compound represented by formula I-3B and hydrochloric acid have a molar ratio of 1:0.9.

In a certain embodiment, the crystal form of the hydrochloride salt of the compound represented by formula I-3B is a crystal form of the acetone solvate of the hydrochloride salt of the compound represented by formula I-3B, wherein the compound represented by formula I-3B, hydrochloric acid, and acetone have a molar ratio of 1:0.9:0.9.

In a certain embodiment, the X-ray powder diffraction pattern for the crystal form of the hydrochloride salt of the compound represented by formula I-3B, using Cu-Kα radiation and expressed by 20 angles, further comprises diffraction peaks at one or more of 20.7 ± 0.2°, 18.9 ± 0.2°, 22.8 ± 0.2°, 6.6 ± 0.2°, and 22.7 ± 0.2°.

In a certain embodiment, the X-ray powder diffraction pattern for the crystal form of the hydrochloride salt of the compound represented by formula I-3B, using Cu-Kα radiation and expressed by 20 angles, comprises diffraction peaks at 9.5 ± 0.2°, 24.8 ± 0.2°, 17.3 ± 0.2°, 19.3 ± 0.2°, 11.7 ± 0.2°, and 20.7 ± 0.2°.

In a certain embodiment, the X-ray powder diffraction pattern for the crystal form of the hydrochloride salt of the compound represented by formula I-3B, using Cu-Kα radiation and expressed by 20 angles, comprises diffraction peaks at 9.5 ± 0.2°, 24.8 ± 0.2°, 17.3 ± 0.2°, 19.3 ± 0.2°, 11.7 ± 0.2°, 20.7 ± 0.2°, 18.9 ± 0.2°, 22.8 ± 0.2°, 6.6 ± 0.2°, and 22.7 ± 0.2°.

In a certain embodiment, the X-ray powder diffraction pattern for the crystal form of the hydrochloride salt of the compound represented by formula I-3B, using Cu-Kα radiation and expressed by 20 angles, comprises diffraction peaks shown in Table 3.

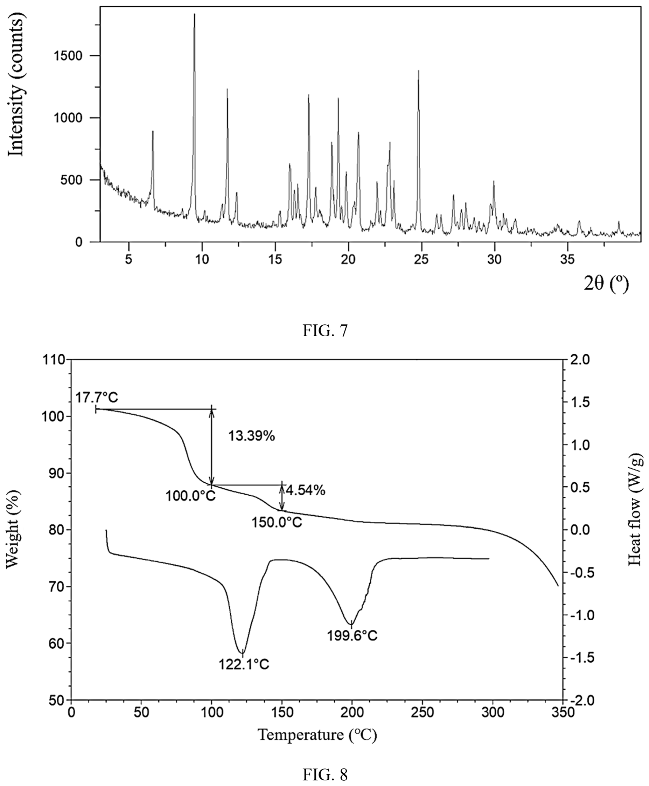

In a certain embodiment, the X-ray powder diffraction (XRPD) pattern for the crystal form of the hydrochloride salt of the compound represented by formula I-3B using Cu-Kα radiation is substantially shown in FIG. 7.

In a certain embodiment, the crystal form of the hydrochloride salt of the compound represented by formula I-3B has a differential scanning calorimetry (DSC) curve comprising endothermic peaks with peak temperatures at 122.1 ± 3°C and 199.6 ± 3°C.

In a certain embodiment, the differential scanning calorimetry (DSC) curve for the crystal form of the hydrochloride salt of the compound represented by formula I-3B is substantially shown in FIG. 8.

In a certain embodiment, the crystal form of the hydrochloride salt of the compound represented by formula I-3B has a thermogravimetric analysis (TGA) curve with a weight loss of approximately 13.39% in the temperature range of 17.7 ± 3°C to 100 ± 3°C and a weight loss of approximately 4.54% in the temperature range of 100 ± 3°C to 150 ± 3°C.

In a certain embodiment, the thermogravimetric analysis (TGA) curve for the crystal form of the hydrochloride salt of the compound represented by formula I-3B is substantially shown in FIG. 8.

The present disclosure provides a crystal form of the phosphate salt of the compound represented by formula I-3B, which has an X-ray powder diffraction pattern using Cu-Kα radiation and expressed by 20 angles comprising diffraction peaks at 16.7 ± 0.2°, 16.3 ± 0.2°, 8.3 ± 0.2°, 21.1 ± 0.2°, and 11.5 ± 0.2°.

In a certain embodiment, in the crystal form of the phosphate salt of the compound represented by formula I-3B, the compound represented by formula I-3B and phosphoric acid have a molar ratio of 1:1.0.

In a certain embodiment, the crystal form of the phosphate salt of the compound represented by formula I-3B is a crystal form of the acetone solvate of the phosphate salt of the compound represented by formula I-3B, wherein the compound represented by formula I-3B, phosphoric acid, and acetone have a molar ratio of 1:1.0:0.05.

In a certain embodiment, the X-ray powder diffraction pattern for the crystal form of the phosphate salt of the compound represented by formula I-3B, using Cu-Kα radiation and expressed by 20 angles, further comprises diffraction peaks at one or more of 23.2 ± 0.2°, 19.2 ± 0.2°, 20.1 ± 0.2°, 22.3 ± 0.2°, and 10.2 ± 0.2°.

In a certain embodiment, the X-ray powder diffraction pattern for the crystal form of the phosphate salt of the compound represented by formula I-3B, using Cu-Kα radiation and expressed by 20 angles, comprises diffraction peaks at 16.7 ± 0.2°, 16.3 ± 0.2°, 8.3 ± 0.2°, 21.1 ± 0.2°, 11.5 ± 0.2°, 23.2 ± 0.2°, and 19.2 ± 0.2°.

In a certain embodiment, the X-ray powder diffraction pattern for the crystal form of the phosphate salt of the compound represented by formula I-3B, using Cu-Kα radiation and expressed by 20 angles, comprises diffraction peaks at 16.7 ± 0.2°, 16.3 ± 0.2°, 8.3 ± 0.2°, 21.1 ± 0.2°, 11.5 ± 0.2°, 23.2 ± 0.2°, 19.2 ± 0.2°, 20.1 ± 0.2°, 22.3 ± 0.2°, and 10.2 ± 0.2°.

In a certain embodiment, the X-ray powder diffraction pattern for the crystal form of the phosphate salt of the compound represented by formula I-3B, using Cu-Kα radiation and expressed by 20 angles, comprises diffraction peaks shown in Table 4.

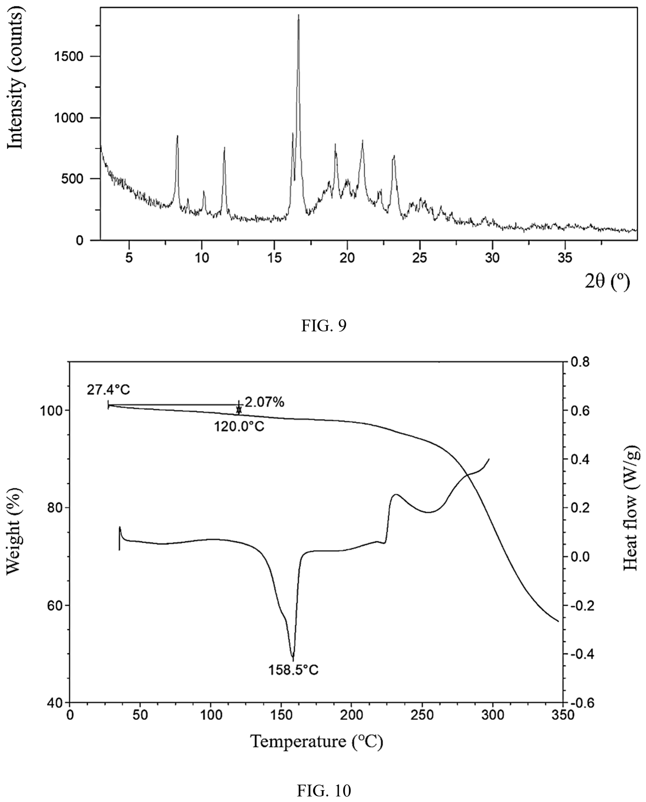

In a certain embodiment, the X-ray powder diffraction (XRPD) pattern for the crystal form of the phosphate salt of the compound represented by formula I-3B using Cu-Kα radiation is substantially shown in FIG. 9.

In a certain embodiment, the crystal form of the phosphate salt of the compound represented by formula I-3B has a differential scanning calorimetry (DSC) curve comprising an endothermic peak with a peak temperature at 158.5 ± 3°C.

In a certain embodiment, the differential scanning calorimetry (DSC) curve for the crystal form of the phosphate salt of the compound represented by formula I-3B is substantially shown in FIG. 10.

In a certain embodiment, the crystal form of the phosphate salt of the compound represented by formula I-3B has a thermogravimetric analysis (TGA) curve with a weight loss of approximately 2.07% in the temperature range of 27.4 ± 3°C to 120 ± 3°C.

In a certain embodiment, the thermogravimetric analysis (TGA) curve for the crystal form of the phosphate salt of the compound represented by formula I-3B is substantially shown in FIG. 10.

The present disclosure provides a crystal form of the fumarate complex of the compound represented by formula I-3B, which has an X-ray powder diffraction pattern using Cu-Kα radiation and expressed by 2θ angles comprising diffraction peaks at 20.5 ± 0.2°, 15.9 ± 0.2°, 26.6 ± 0.2°, 18.9 ± 0.2°, and 22.5 ± 0.2°.

In a certain embodiment, in the crystal form of the fumarate complex of the compound represented by formula I-3B, the compound represented by formula I-3B and fumaric acid have a molar ratio of 1:1.

In a certain embodiment, the fumarate complex of the compound represented by formula I-3B is a co-crystal of the compound represented by formula I-3B with fumaric acid.

In a certain embodiment, the X-ray powder diffraction pattern for the crystal form of the fumarate complex of the compound represented by formula I-3B, using Cu-Kα radiation and expressed by 20 angles, further comprises diffraction peaks at one or more of 22.3 ± 0.2°, 26.4 ± 0.2°, 10.8 ± 0.2°, 17.4 ± 0.2°, and 17.5 ± 0.2°.

In a certain embodiment, the X-ray powder diffraction pattern for the crystal form of the fumarate complex of the compound represented by formula I-3B, using Cu-Kα radiation and expressed by 20 angles, comprises diffraction peaks at 20.5 ± 0.2°, 15.9 ± 0.2°, 26.6 ± 0.2°, 18.9 ± 0.2°, 22.5 ± 0.2°, 22.3 ± 0.2°, and 26.4 ± 0.2°.

In a certain embodiment, the X-ray powder diffraction pattern for the crystal form of the fumarate complex of the compound represented by formula I-3B, using Cu-Kα radiation and expressed by 20 angles, comprises diffraction peaks at 20.5 ± 0.2°, 15.9 ± 0.2°, 26.6 ± 0.2°, 18.9 ± 0.2°, 22.5 ± 0.2°, 22.3 ± 0.2°, 26.4 ± 0.2°, 10.8 ± 0.2°, 17.4 ± 0.2°, and 17.5 ± 0.2°.

In a certain embodiment, the X-ray powder diffraction pattern for the crystal form of the fumarate complex of the compound represented by formula I-3B, using Cu-Kα radiation and expressed by 20 angles, comprises diffraction peaks shown in Table 5.

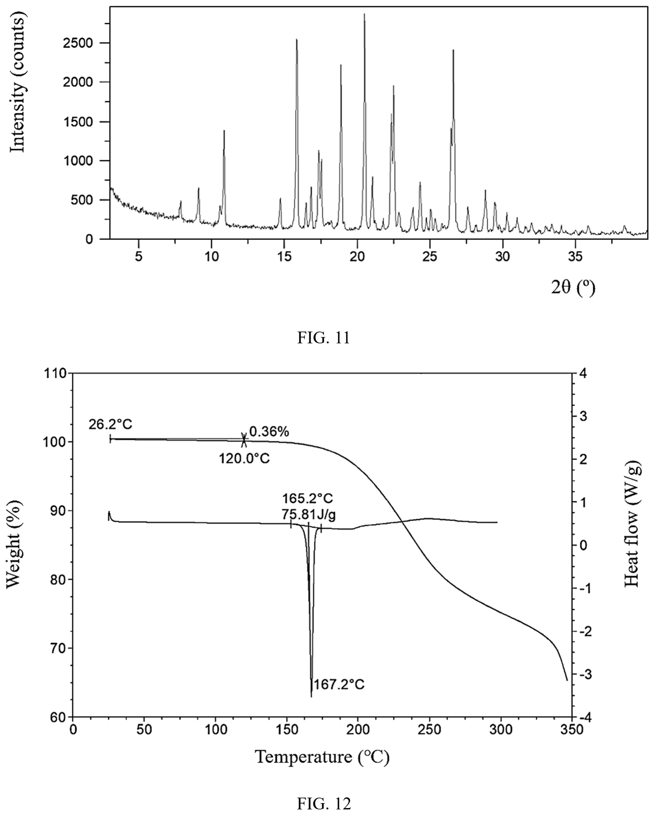

In a certain embodiment, the X-ray powder diffraction (XRPD) pattern for the crystal form of the fumarate complex of the compound represented by formula I-3B using Cu-Kα radiation is substantially shown in FIG. 11.

In a certain embodiment, the crystal form of the fumarate complex of the compound represented by formula I-3B has a differential scanning calorimetry (DSC) curve comprising an endothermic peak with an onset temperature at 165.2 ± 3°C, and/or the crystal form of the fumarate complex of the compound represented by formula I-3B has a differential scanning calorimetry (DSC) curve comprising an endothermic peak with a peak temperature at 167.2 ± 3°C.

In a certain embodiment, the differential scanning calorimetry (DSC) curve for the crystal form of the fumarate complex of the compound represented by formula I-3B is substantially shown in FIG. 12.

In a certain embodiment, the crystal form of the fumarate complex of the compound represented by formula I-3B has a thermogravimetric analysis (TGA) curve with a weight loss of approximately 0.36% in the temperature range of 26.2 ± 3°C to 120 ± 3°C.

In a certain embodiment, the thermogravimetric analysis (TGA) curve for the crystal form of the fumarate complex of the compound represented by formula I-3B is substantially shown in FIG. 12.

In a certain embodiment, the crystal form of the fumarate complex of the compound represented by formula I-3B is a single crystal of the fumarate complex of the compound represented by formula I-3B, having the following unit cell parameters: orthorhombic, space group of P2₁2₁2₁; a = 6.4400(4) Å, α = 90°, b = 11.9376(8) Å, β = 90°, c = 33.139(2) Å, γ = 90°, unit cell volume = 2547.7(3) Å³, number of asymmetric units in the unit cell Z = 4, crystal density of 1.451 mg/m³.

The present disclosure provides a crystal form of the L-tartrate salt of the compound represented by formula I-3B, which has an X-ray powder diffraction pattern using Cu-Kα radiation and expressed by 20 angles comprising diffraction peaks at 8.2 ± 0.2°, 20.7 ± 0.2°, 20.1 ± 0.2°, 15.7 ± 0.2°, and 23.3 ± 0.2°.

In a certain embodiment, in the crystal form of the L-tartrate salt of the compound represented by formula I-3B, the compound represented by formula I-3B and L-tartaric acid have a molar ratio of 1:1.

In a certain embodiment, the X-ray powder diffraction pattern for the crystal form of the L-tartrate salt of the compound represented by formula I-3B, using Cu-Kα radiation and expressed by 20 angles, further comprises diffraction peaks at one or more of 18.4 ± 0.2°, 9.6 ± 0.2°, 17.6 ± 0.2°, 21.3 ± 0.2°, and 23.0 ± 0.2°.

In a certain embodiment, the X-ray powder diffraction pattern for the crystal form of the L-tartrate salt of the compound represented by formula I-3B, using Cu-Kα radiation and expressed by 20 angles, comprises diffraction peaks at 8.2 ± 0.2°, 20.7 ± 0.2°, 20.1 ± 0.2°, 15.7 ± 0.2°, 23.3 ± 0.2°, and 18.4 ± 0.2°.

In a certain embodiment, the X-ray powder diffraction pattern for the crystal form of the L-tartrate salt of the compound represented by formula I-3B, using Cu-Kα radiation and expressed by 20 angles, comprises diffraction peaks at 8.2 ± 0.2°, 20.7 ± 0.2°, 20.1 ± 0.2°, 15.7 ± 0.2°, 23.3 ± 0.2°, 18.4 ± 0.2°, 9.6 ± 0.2°, 17.6 ± 0.2°, 21.3 ± 0.2°, and 23.0 ± 0.2°.

In a certain embodiment, the X-ray powder diffraction pattern for the crystal form of the L-tartrate salt of the compound represented by formula I-3B, using Cu-Kα radiation and expressed by 20 angles, comprises diffraction peaks shown in Table 6.

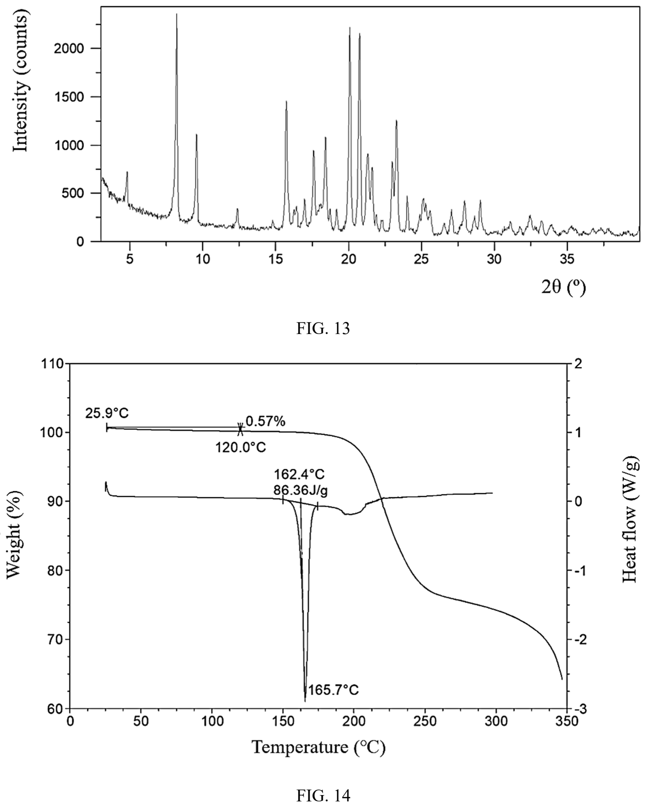

In a certain embodiment, the X-ray powder diffraction (XRPD) pattern for the crystal form of the L-tartrate salt of the compound represented by formula I-3B using Cu-Kα radiation is substantially shown in FIG. 13.

In a certain embodiment, the crystal form of the L-tartrate salt of the compound represented by formula I-3B has a differential scanning calorimetry (DSC) curve comprising an endothermic peak with an onset temperature at 162.4 ± 3°C, and/or the crystal form of the L-tartrate salt of the compound represented by formula I-3B has a differential scanning calorimetry (DSC) curve comprising an endothermic peak with a peak temperature at 165.7 ± 3°C.

In a certain embodiment, the differential scanning calorimetry (DSC) curve for the crystal form of the L-tartrate salt of the compound represented by formula I-3B is substantially shown in FIG. 14.

In a certain embodiment, the crystal form of the L-tartrate salt of the compound represented by formula I-3B has a thermogravimetric analysis (TGA) curve with a weight loss of approximately 0.57% in the temperature range of 25.9 ± 3°C to 120 ± 3°C.

In a certain embodiment, the thermogravimetric analysis (TGA) curve for the crystal form of the L-tartrate salt of the compound represented by formula I-3B is substantially shown in FIG. 14.

The present disclosure provides a crystal form of the p-toluenesulfonate salt of the compound represented by formula I-3B, which has an X-ray powder diffraction pattern using Cu-Kα radiation and expressed by 20 angles comprising a diffraction peak at 6.6 ± 0.2°.

In a certain embodiment, in the crystal form of the p-toluenesulfonate salt of the compound represented by formula I-3B, the compound represented by formula I-3B and p-toluenesulfonic acid have a molar ratio of 1:1.

In a certain embodiment, the X-ray powder diffraction pattern for the crystal form of the p-toluenesulfonate salt of the compound represented by formula I-3B, using Cu-Kα radiation and expressed by 20 angles, further comprises diffraction peaks at one or more of 19.8 ± 0.2°, 18.5 ± 0.2°, 19.7 ± 0.2°, 20.4 ± 0.2°, 9.8 ± 0.2°, 11.4 ± 0.2°, 13.2 ± 0.2°, 22.3 ± 0.2°, and 8.1 ± 0.2°.

In a certain embodiment, the X-ray powder diffraction pattern for the crystal form of the p-toluenesulfonate salt of the compound represented by formula I-3B, using Cu-Kα radiation and expressed by 20 angles, comprises diffraction peaks at 6.6 ± 0.2°, 19.8 ± 0.2°, 18.5 ± 0.2°, 19.7 ± 0.2°, and 20.4 ± 0.2°.

In a certain embodiment, the X-ray powder diffraction pattern for the crystal form of the p-toluenesulfonate salt of the compound represented by formula I-3B, using Cu-Kα radiation and expressed by 20 angles, comprises diffraction peaks at 6.6 ± 0.2°, 19.8 ± 0.2°, 18.5 ± 0.2°, 19.7 ± 0.2°, 20.4 ± 0.2°, 9.8 ± 0.2°, 11.4 ± 0.2°, 13.2 ± 0.2°, 22.3 ± 0.2°, and 8.1 ± 0.2°.

In a certain embodiment, the X-ray powder diffraction pattern for the crystal form of the p-toluenesulfonate salt of the compound represented by formula I-3B, using Cu-Kα radiation and expressed by 20 angles, comprises diffraction peaks shown in Table 7.

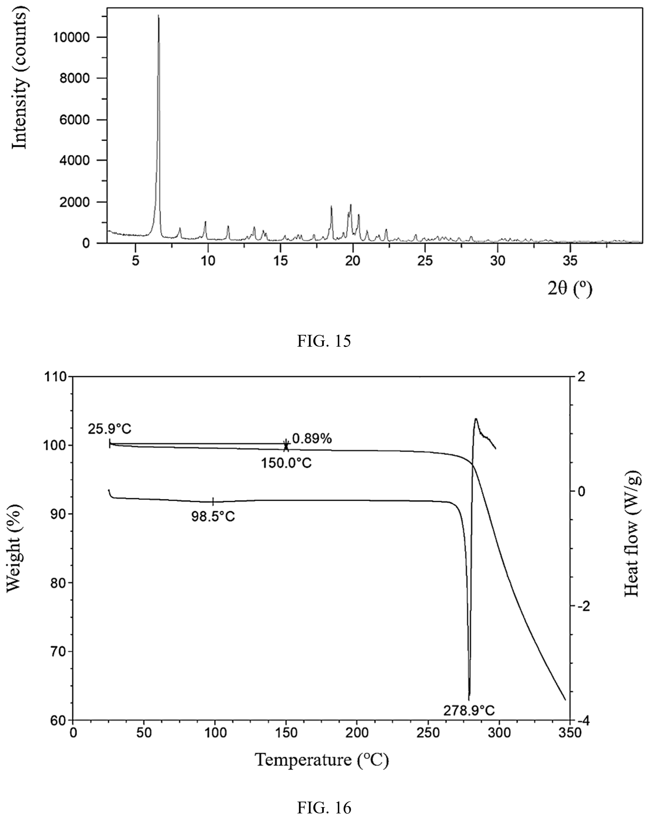

In a certain embodiment, the X-ray powder diffraction (XRPD) pattern for the crystal form of the p-toluenesulfonate salt of the compound represented by formula I-3B using Cu-Kα radiation is substantially shown in FIG. 15.

In a certain embodiment, the crystal form of the p-toluenesulfonate salt of the compound represented by formula I-3B has a differential scanning calorimetry (DSC) curve comprising an endothermic peak with a peak temperature at 278.9 ± 3°C.

In a certain embodiment, the differential scanning calorimetry (DSC) curve for the crystal form of the p-toluenesulfonate salt of the compound represented by formula I-3B is shown in FIG. 16.

In a certain embodiment, the crystal form of the p-toluenesulfonate salt of the compound represented by formula I-3B has a thermogravimetric analysis (TGA) curve with a weight loss of approximately 0.89% in the temperature range of 25.9 ± 3°C to 150 ± 3°C.

In a certain embodiment, the thermogravimetric analysis (TGA) curve for the crystal form of the p-toluenesulfonate salt of the compound represented by formula I-3B is shown in FIG. 16.

The present disclosure provides a crystal form of the L-malate salt of the compound represented by formula I-3B, which has an X-ray powder diffraction pattern using Cu-Kα radiation and expressed by 20 angles comprising diffraction peaks at 15.8 ± 0.2°, 20.3 ± 0.2°, 18.8 ± 0.2°, 21.8 ± 0.2°, and 22.0 ± 0.2°.

In a certain embodiment, in the crystal form of the L-malate salt of the compound represented by formula I-3B, the compound represented by formula I-3B and L-malic acid have a molar ratio of 1:1.

In a certain embodiment, the X-ray powder diffraction pattern for the crystal form of the L-malate salt of the compound represented by formula I-3B, using Cu-Kα radiation and expressed by 20 angles, further comprises diffraction peaks at one or more of 20.6 ± 0.2°, 25.5 ± 0.2°, 17.2 ± 0.2°, 16.1 ± 0.2°, and 17.6 ± 0.2°.

In a certain embodiment, the X-ray powder diffraction pattern for the crystal form of the L-malate salt of the compound represented by formula I-3B, using Cu-Kα radiation and expressed by 20 angles, comprises diffraction peaks at 15.8 ± 0.2°, 20.3 ± 0.2°, 18.8 ± 0.2°, 21.8 ± 0.2°, 22.0 ± 0.2°, 20.6 ± 0.2°, 25.5 ± 0.2°, and 17.2 ± 0.2°.

In a certain embodiment, the X-ray powder diffraction pattern for the crystal form of the L-malate salt of the compound represented by formula I-3B, using Cu-Kα radiation and expressed by 20 angles, comprises diffraction peaks at 15.8 ± 0.2°, 20.3 ± 0.2°, 18.8 ± 0.2°, 21.8 ± 0.2°, 22.0 ± 0.2°, 20.6 ± 0.2°, 25.5 ± 0.2°, 17.2 ± 0.2°, 16.1 ± 0.2°, and 17.6 ± 0.2°.

In a certain embodiment, the X-ray powder diffraction pattern for the crystal form of the L-malate salt of the compound represented by formula I-3B, using Cu-Kα radiation and expressed by 20 angles, comprises diffraction peaks shown in Table 8.

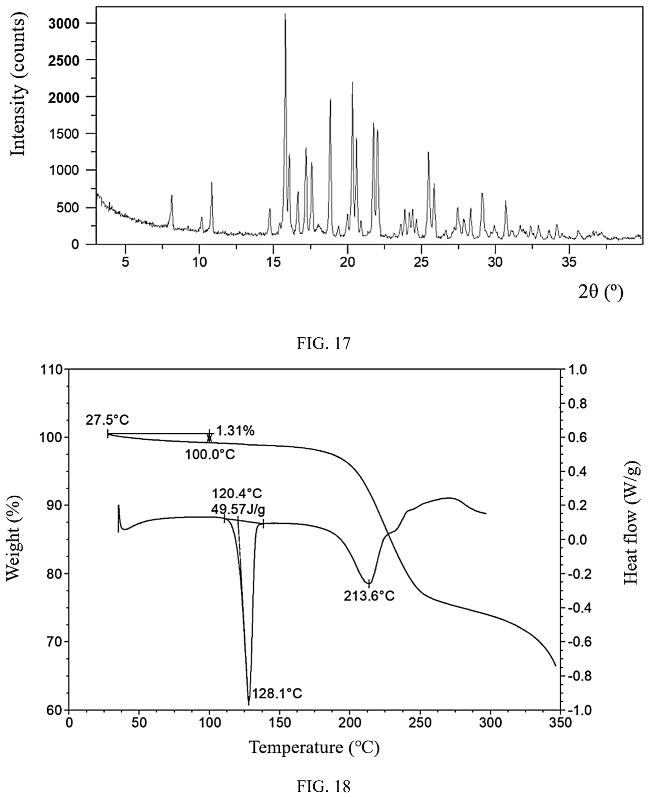

In a certain embodiment, the X-ray powder diffraction (XRPD) pattern for the crystal form of the L-malate salt of the compound represented by formula I-3B using Cu-Kα radiation is substantially shown in FIG. 17.

In a certain embodiment, the crystal form of the L-malate salt of the compound represented by formula I-3B has a differential scanning calorimetry (DSC) curve comprising an endothermic peak with an onset temperature at 120.4 ± 3°C, and/or the crystal form of the L-malate salt of the compound represented by formula I-3B has a differential scanning calorimetry (DSC) curve comprising endothermic peaks with peak temperatures at 128.1 ± 3°C and 213.6 ± 3°C.

In a certain embodiment, the differential scanning calorimetry (DSC) curve for the crystal form of the L-malate salt of the compound represented by formula I-3B is substantially shown in FIG. 18.

In a certain embodiment, the crystal form of the L-malate salt of the compound represented by formula I-3B has a thermogravimetric analysis (TGA) curve with a weight loss of approximately 1.31% in the temperature range of 27.5 ± 3°C to 100 ± 3°C.

In a certain embodiment, the thermogravimetric analysis (TGA) curve for the crystal form of the L-malate salt of the compound represented by formula I-3B is substantially shown in FIG. 18.

The present disclosure provides a crystal form A of the ethanedisulfonate salt of the compound represented by formula I-3B, which has an X-ray powder diffraction pattern using Cu-Kα radiation and expressed by 20 angles comprising diffraction peaks at 5.3 ± 0.2° and 18.9 ± 0.2°.

In a certain embodiment, in the crystal form A of the ethanedisulfonate salt of the compound represented by formula I-3B, the compound represented by formula I-3B and ethanedisulfonic acid have a molar ratio of 1:0.5.

In a certain embodiment, the crystal form A of the ethanedisulfonate salt of the compound represented by formula I-3B is a crystal form of the isopropanol solvate of the ethanedisulfonate salt of the compound represented by formula I-3B, wherein the compound represented by formula I-3B, ethanedisulfonic acid, and isopropanol have a molar ratio of 1:0.5:0.5.

In a certain embodiment, the X-ray powder diffraction pattern for the crystal form A of the ethanedisulfonate salt of the compound represented by formula I-3B, using Cu-Kα radiation and expressed by 20 angles, further comprises diffraction peaks at one or more of 20.1 ± 0.2°, 21.0 ± 0.2°, 18.2 ± 0.2°, 21.6 ± 0.2°, 17.4 ± 0.2°, 22.3 ± 0.2°, and 11.5 ± 0.2°.

In a certain embodiment, the X-ray powder diffraction pattern for the crystal form A of the ethanedisulfonate salt of the compound represented by formula I-3B, using Cu-Kα radiation and expressed by 20 angles, comprises diffraction peaks at 5.3 ± 0.2°, 18.9 ± 0.2°, 20.1 ± 0.2°, 21.0 ± 0.2°, 18.2 ± 0.2°, and 21.6 ± 0.2°.

In a certain embodiment, the X-ray powder diffraction pattern for the crystal form A of the ethanedisulfonate salt of the compound represented by formula I-3B, using Cu-Kα radiation and expressed by 20 angles, comprises diffraction peaks at 5.3 ± 0.2°, 18.9 ± 0.2°, 20.1 ± 0.2°, 21.0 ± 0.2°, 18.2 ± 0.2°, 21.6 ± 0.2°, 17.4 ± 0.2°, 22.3 ± 0.2°, and 11.5 ± 0.2°.

In a certain embodiment, the X-ray powder diffraction pattern for the crystal form A of the ethanedisulfonate salt of the compound represented by formula I-3B, using Cu-Kα radiation and expressed by 20 angles, comprises diffraction peaks shown in Table 9.

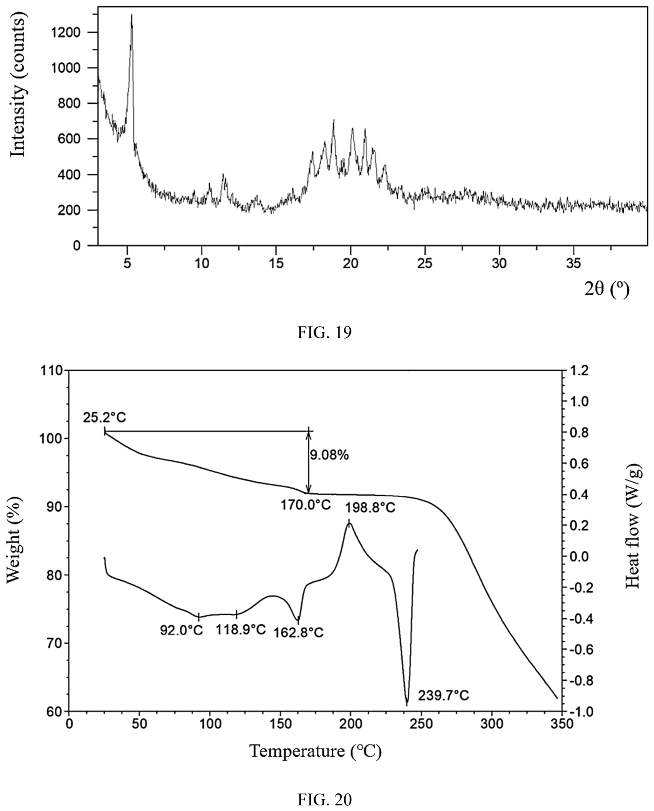

In a certain embodiment, the X-ray powder diffraction (XRPD) pattern for the crystal form A of the ethanedisulfonate salt of the compound represented by formula I-3B using Cu-Kα radiation is substantially shown in FIG. 19.

In a certain embodiment, the crystal form A of the ethanedisulfonate salt of the compound represented by formula I-3B has a differential scanning calorimetry (DSC) curve comprising endothermic peaks with peak temperatures at 162.8 ± 3°C and 239.7 ± 3°C and an exothermic peak with a peak temperature at 198.8 ± 3°C.

In a certain embodiment, the differential scanning calorimetry (DSC) curve for the crystal form A of the ethanedisulfonate salt of the compound represented by formula I-3B is substantially shown in FIG. 20.

In a certain embodiment, the crystal form A of the ethanedisulfonate salt of the compound represented by formula I-3B has a thermogravimetric analysis (TGA) curve with a weight loss of approximately 9.08% in the temperature range of 25.2 ± 3°C to 170 ± 3°C.

In a certain embodiment, the thermogravimetric analysis (TGA) curve for the crystal form A of the ethanedisulfonate salt of the compound represented by formula I-3B is substantially shown in FIG. 20.

The present disclosure provides a crystal form B of the ethanedisulfonate salt of the compound represented by formula I-3B, which has an X-ray powder diffraction pattern using Cu-Kα radiation and expressed by 2θ angles comprising diffraction peaks at 18.6 ± 0.2°, 19.4 ± 0.2°, and 21.6 ± 0.2°.

In a certain embodiment, in the crystal form B of the ethanedisulfonate salt of the compound represented by formula I-3B, the compound represented by formula I-3B and ethanedisulfonic acid have a molar ratio of 1:0.5.

In a certain embodiment, the X-ray powder diffraction pattern for the crystal form B of the ethanedisulfonate salt of the compound represented by formula I-3B, using Cu-Kα radiation and expressed by 20 angles, further comprises diffraction peaks at one or more of 17.7 ± 0.2°, 22.1 ± 0.2°, 15.1 ± 0.2°, 6.3 ± 0.2°, 20.8 ± 0.2°, 20.4 ± 0.2°, and 11.0 ± 0.2°.

In a certain embodiment, the X-ray powder diffraction pattern for the crystal form B of the ethanedisulfonate salt of the compound represented by formula I-3B, using Cu-Kα radiation and expressed by 20 angles, comprises diffraction peaks at 18.6 ± 0.2°, 19.4 ± 0.2°, 21.6 ± 0.2°, 17.7 ± 0.2°, 22.1 ± 0.2°, and 15.1 ± 0.2°.

In a certain embodiment, the X-ray powder diffraction pattern for the crystal form B of the ethanedisulfonate salt of the compound represented by formula I-3B, using Cu-Kα radiation and expressed by 20 angles, comprises diffraction peaks at 18.6 ± 0.2°, 19.4 ± 0.2°, 21.6 ± 0.2°, 17.7 ± 0.2°, 22.1 ± 0.2°, 15.1 ± 0.2°, 6.3 ± 0.2°, 20.8 ± 0.2°, 20.4 ± 0.2°, and 11.0 ± 0.2°.

In a certain embodiment, the X-ray powder diffraction pattern for the crystal form B of the ethanedisulfonate salt of the compound represented by formula I-3B, using Cu-Kα radiation and expressed by 20 angles, comprises diffraction peaks shown in Table 10.

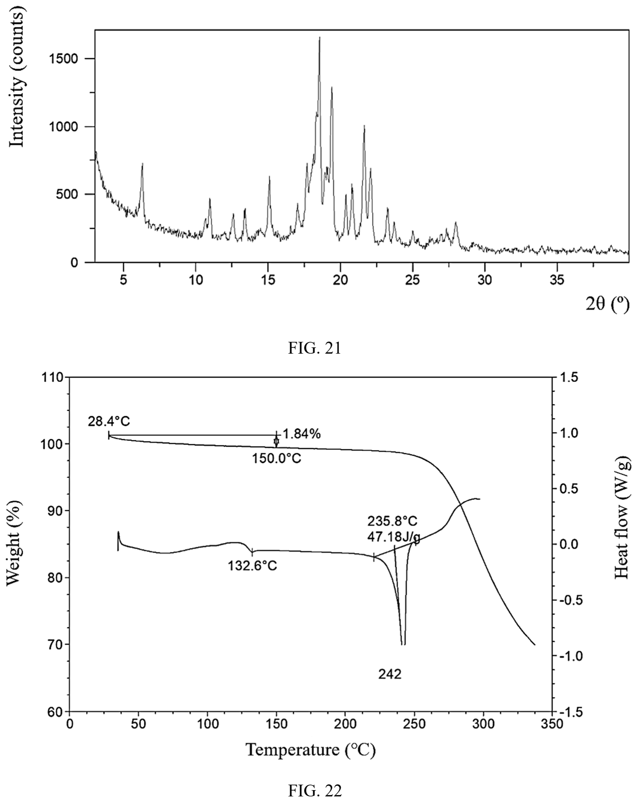

In a certain embodiment, the X-ray powder diffraction (XRPD) pattern for the crystal form B of the ethanedisulfonate salt of the compound represented by formula I-3B using Cu-Kα radiation is substantially shown in FIG. 21.

In a certain embodiment, the crystal form B of the ethanedisulfonate salt of the compound represented by formula I-3B has a differential scanning calorimetry (DSC) curve comprising an endothermic peak with an onset temperature at 235.8 ± 3°C, and/or the crystal form B of the ethanedisulfonate salt of the compound represented by formula I-3B has a differential scanning calorimetry (DSC) curve comprising an endothermic peak with a peak temperature at 242.4 ± 3°C.

In a certain embodiment, the differential scanning calorimetry (DSC) curve for the crystal form B of the ethanedisulfonate salt of the compound represented by formula I-3B is substantially shown in FIG. 22.

In a certain embodiment, the crystal form B of the ethanedisulfonate salt of the compound represented by formula I-3B has a thermogravimetric analysis (TGA) curve with a weight loss of approximately 1.84% in the temperature range of 28.4 ± 3°C to 150 ± 3°C.

In a certain embodiment, the thermogravimetric analysis (TGA) curve for the crystal form B of the ethanedisulfonate salt of the compound represented by formula I-3B is substantially shown in FIG. 22.

The present disclosure provides a crystal form C of the ethanedisulfonate salt of the compound represented by formula I-3B, which has an X-ray powder diffraction pattern using Cu-Kα radiation and expressed by 20 angles comprising diffraction peaks at 20.3 ± 0.2°, 19.6 ± 0.2°, 11.6 ± 0.2°, 24.0 ± 0.2°, and 23.0 ± 0.2°.

In a certain embodiment, in the crystal form C of the ethanedisulfonate salt of the compound represented by formula I-3B, the compound represented by formula I-3B and ethanedisulfonic acid have a molar ratio of 1:1.

In a certain embodiment, the crystal form C of the ethanedisulfonate salt of the compound represented by formula I-3B is a crystal form of the acetone/n-heptane solvate of the ethanedisulfonate salt of the compound represented by formula I-3B, wherein the compound represented by formula I-3B, ethanedisulfonic acid, acetone, and n-heptane have a molar ratio of 1:1:0.3:0.03.

In a certain embodiment, the X-ray powder diffraction pattern for the crystal form C of the ethanedisulfonate salt of the compound represented by formula I-3B, using Cu-Kα radiation and expressed by 20 angles, further comprises diffraction peaks at one or more of 7.3 ± 0.2°, 23.2 ± 0.2°, 13.0 ± 0.2°, 16.4 ± 0.2°, and 15.4 ± 0.2°.

In a certain embodiment, the X-ray powder diffraction pattern for the crystal form C of the ethanedisulfonate salt of the compound represented by formula I-3B, using Cu-Kα radiation and expressed by 20 angles, comprises diffraction peaks at 20.3 ± 0.2°, 19.6 ± 0.2°, 11.6 ± 0.2°, 24.0 ± 0.2°, 23.0 ± 0.2°, and 17.3 ± 0.2°.

In a certain embodiment, the X-ray powder diffraction pattern for the crystal form C of the ethanedisulfonate salt of the compound represented by formula I-3B, using Cu-Kα radiation and expressed by 20 angles, comprises diffraction peaks at 20.3 ± 0.2°, 19.6 ± 0.2°, 11.6 ± 0.2°, 24.0 ± 0.2°, 23.0 ± 0.2°, 17.3 ± 0.2°, 23.2 ± 0.2°, 13.0 ± 0.2°, 16.4 ± 0.2°, and 15.4 ± 0.2°.

In a certain embodiment, the X-ray powder diffraction pattern for the crystal form C of the ethanedisulfonate salt of the compound represented by formula I-3B, using Cu-Kα radiation and expressed by 20 angles, comprises diffraction peaks shown in Table 11.

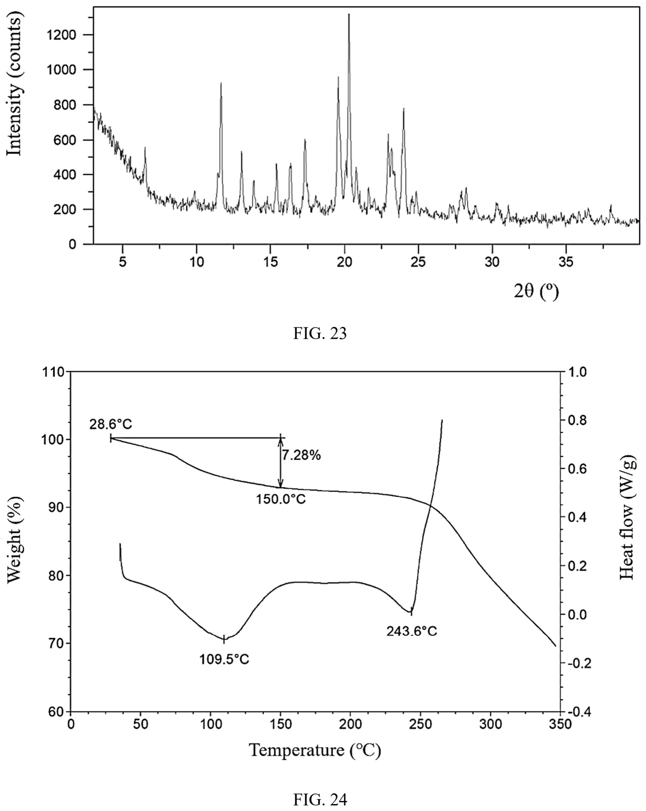

In a certain embodiment, the X-ray powder diffraction (XRPD) pattern for the crystal form C of the ethanedisulfonate salt of the compound represented by formula I-3B using Cu-Kα radiation is substantially shown in FIG. 23.

In a certain embodiment, the crystal form C of the ethanedisulfonate salt of the compound represented by formula I-3B has a differential scanning calorimetry (DSC) curve comprising endothermic peaks with peak temperatures at 109.5 ± 3°C and 243.6 ± 3°C.

In a certain embodiment, the differential scanning calorimetry (DSC) curve for the crystal form C of the ethanedisulfonate salt of the compound represented by formula I-3B is substantially shown in FIG. 24.

In a certain embodiment, the crystal form C of the ethanedisulfonate salt of the compound represented by formula I-3B has a thermogravimetric analysis (TGA) curve with a weight loss of approximately 7.28% in the temperature range of 28.6 ± 3°C to 150 ± 3°C.

In a certain embodiment, the thermogravimetric analysis (TGA) curve for the crystal form C of the ethanedisulfonate salt of the compound represented by formula I-3B is substantially shown in FIG. 24.

The present disclosure provides a crystal form of the methanesulfonate salt of the compound represented by formula I-3B, which has an X-ray powder diffraction pattern using Cu-Kα radiation and expressed by 20 angles comprising diffraction peaks at 9.4 ± 0.2°, 6.5 ± 0.2°, 19.9 ± 0.2°, 18.2 ± 0.2°, and 21.8 ± 0.2°.

In a certain embodiment, in the crystal form of the methanesulfonate salt of the compound represented by formula I-3B, the compound represented by formula I-3B and methanesulfonic acid have a molar ratio of 1:1.

In a certain embodiment, the crystal form of the methanesulfonate salt of the compound represented by formula I-3B is a crystal form of the n-heptane solvate of the methanesulfonate salt of the compound represented by formula I-3B, wherein the compound represented by formula I-3B, methanesulfonic acid, and n-heptane have a molar ratio of 1:1:0.008.

In a certain embodiment, the X-ray powder diffraction pattern for the crystal form of the methanesulfonate salt of the compound represented by formula I-3B, using Cu-Kα radiation and expressed by 20 angles, further comprises diffraction peaks at one or more of 18.6 ± 0.2°, 15.6 ± 0.2°, 7.6 ± 0.2°, 22.2 ± 0.2°, and 16.9 ± 0.2°.

In a certain embodiment, the X-ray powder diffraction pattern for the crystal form of the methanesulfonate salt of the compound represented by formula I-3B, using Cu-Kα radiation and expressed by 20 angles, comprises diffraction peaks at 9.4 ± 0.2°, 6.5 ± 0.2°, 19.9 ± 0.2°, 18.2 ± 0.2°, 21.8 ± 0.2°, 18.6 ± 0.2°, and 15.6 ± 0.2°.

In a certain embodiment, the X-ray powder diffraction pattern for the crystal form of the methanesulfonate salt of the compound represented by formula I-3B, using Cu-Kα radiation and expressed by 20 angles, comprises diffraction peaks at 9.4 ± 0.2°, 6.5 ± 0.2°, 19.9 ± 0.2°, 18.2 ± 0.2°, 21.8 ± 0.2°, 18.6 ± 0.2°, 15.6 ± 0.2°, 7.6 ± 0.2°, 22.2 ± 0.2°, and 16.9 ± 0.2°.

In a certain embodiment, the X-ray powder diffraction pattern for the crystal form of the methanesulfonate salt of the compound represented by formula I-3B, using Cu-Kα radiation and expressed by 20 angles, comprises diffraction peaks shown in Table 12.

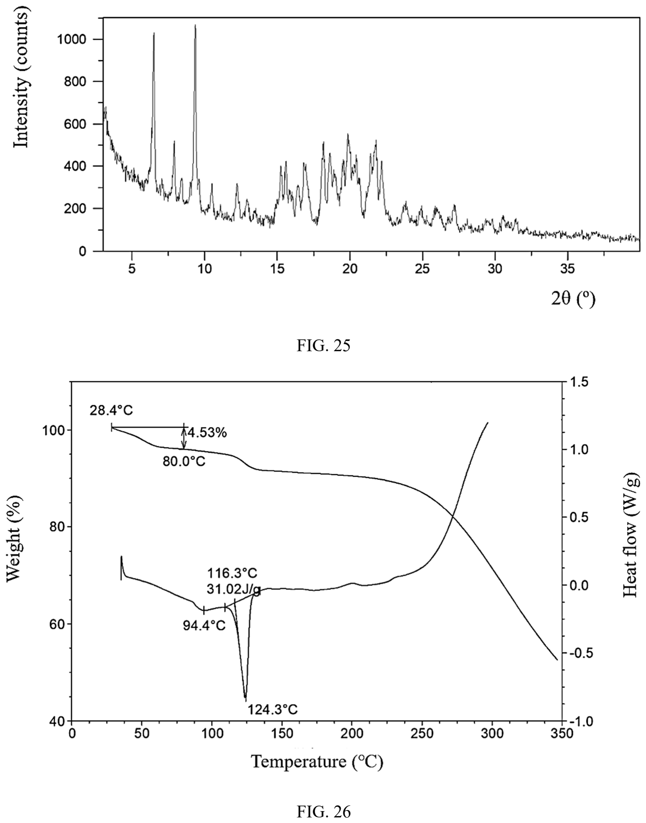

In a certain embodiment, the X-ray powder diffraction (XRPD) pattern for the crystal form of the methanesulfonate salt of the compound represented by formula I-3B using Cu-Kα radiation is substantially shown in FIG. 25.

In a certain embodiment, the crystal form of the methanesulfonate salt of the compound represented by formula I-3B has a differential scanning calorimetry (DSC) curve comprising an endothermic peak with an onset temperature at 116.3 ± 3°C, and/or the crystal form of the methanesulfonate salt of the compound represented by formula I-3B has a differential scanning calorimetry (DSC) curve comprising an endothermic peak with a peak temperature at 124.3 ± 3°C.

In a certain embodiment, the differential scanning calorimetry (DSC) curve for the crystal form of the methanesulfonate salt of the compound represented by formula I-3B is substantially shown in FIG. 26.

In a certain embodiment, the crystal form of the methanesulfonate salt of the compound represented by formula I-3B has a thermogravimetric analysis (TGA) curve with a weight loss of approximately 4.53% in the temperature range of 28.4 ± 3°C to 80 ± 3°C.

In a certain embodiment, the thermogravimetric analysis (TGA) curve for the crystal form of the methanesulfonate salt of the compound represented by formula I-3B is substantially shown in FIG. 26.

The present disclosure provides a crystal form of the benzenesulfonate salt of the compound represented by formula I-3B, which has an X-ray powder diffraction pattern using Cu-Kα radiation and expressed by 2θ angles comprising diffraction peaks at 21.4 ± 0.2°, 20.0 ± 0.2°, 19.6 ± 0.2°, 15.1 ± 0.2°, and 21.0 ± 0.2°.

In a certain embodiment, in the crystal form of the benzenesulfonate salt of the compound represented by formula I-3B, the compound represented by formula I-3B and benzenesulfonic acid have a molar ratio of 1:1.

In a certain embodiment, the crystal form of the benzenesulfonate salt of the compound represented by formula I-3B is a crystal form of the isopropanol solvate of the benzenesulfonate salt of the compound represented by formula I-3B, wherein the compound represented by formula I-3B, benzenesulfonic acid, and isopropanol have a molar ratio of 1:1:0.01.

In a certain embodiment, the X-ray powder diffraction pattern for the crystal form of the benzenesulfonate salt of the compound represented by formula I-3B, using Cu-Kα radiation and expressed by 20 angles, further comprises diffraction peaks at one or more of 20.5 ± 0.2°, 19.1 ± 0.2°, 21.2 ± 0.2°, 6.9 ± 0.2°, and 10.6 ± 0.2°.

In a certain embodiment, the X-ray powder diffraction pattern for the crystal form of the benzenesulfonate salt of the compound represented by formula I-3B, using Cu-Kα radiation and expressed by 20 angles, comprises diffraction peaks at 21.4 ± 0.2°, 20.0 ± 0.2°, 19.6 ± 0.2°, 15.1 ± 0.2°, 21.0 ± 0.2°, 20.5 ± 0.2°, and 19.1 ± 0.2°.

In a certain embodiment, the X-ray powder diffraction pattern for the crystal form of the benzenesulfonate salt of the compound represented by formula I-3B, using Cu-Kα radiation and expressed by 20 angles, comprises diffraction peaks at 21.4 ± 0.2°, 20.0 ± 0.2°, 19.6 ± 0.2°, 15.1 ± 0.2°, 21.0 ± 0.2°, 20.5 ± 0.2°, 19.1 ± 0.2°, 21.2 ± 0.2°, 6.9 ± 0.2°, and 10.6 ± 0.2°.

In a certain embodiment, the X-ray powder diffraction pattern for the crystal form of the benzenesulfonate salt of the compound represented by formula I-3B, using Cu-Kα radiation and expressed by 20 angles, comprises diffraction peaks shown in Table 13.

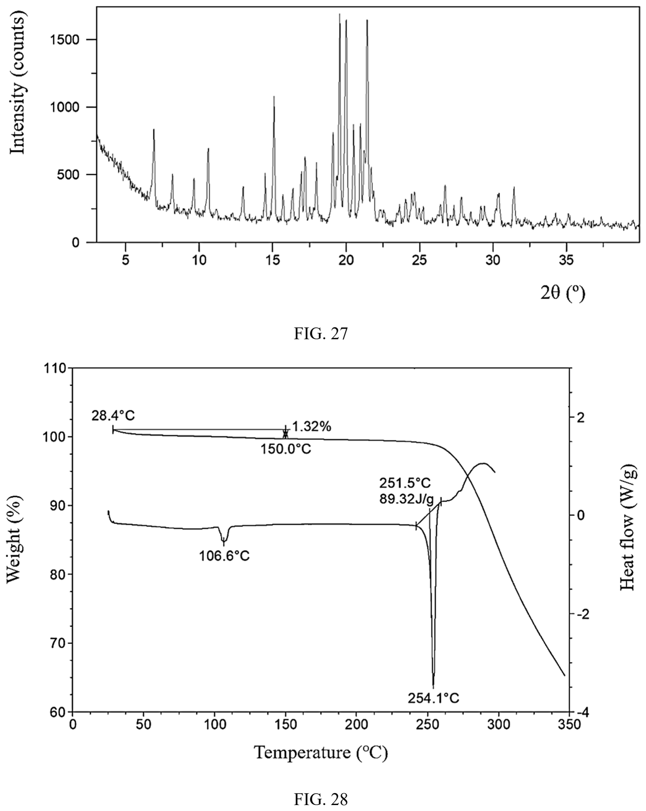

In a certain embodiment, the X-ray powder diffraction (XRPD) pattern for the crystal form of the benzenesulfonate salt of the compound represented by formula I-3B using Cu-Kα radiation is substantially shown in FIG. 27.

In a certain embodiment, the crystal form of the benzenesulfonate salt of the compound represented by formula I-3B has a differential scanning calorimetry (DSC) curve comprising an endothermic peak with a peak temperature at 254.1 ± 3°C.

In a certain embodiment, the differential scanning calorimetry (DSC) curve for the crystal form of the benzenesulfonate salt of the compound represented by formula I-3B is substantially shown in FIG. 28.

In a certain embodiment, the crystal form of the benzenesulfonate salt of the compound represented by formula I-3B has a thermogravimetric analysis (TGA) curve with a weight loss of approximately 1.32% in the temperature range of 28.4 ± 3°C to 150 ± 3°C.

In a certain embodiment, the thermogravimetric analysis (TGA) curve for the crystal form of the benzenesulfonate salt of the compound represented by formula I-3B is substantially shown in FIG. 28.

The present disclosure provides a crystal form of the oxalate salt of the compound represented by formula I-3B, which has an X-ray powder diffraction pattern using Cu-Kα radiation and expressed by 20 angles comprising diffraction peaks at 27.7 ± 0.2°, 7.8 ± 0.2°, 15.7 ± 0.2°, 17.1 ± 0.2°, and 16.5 ± 0.2°.

In a certain embodiment, in the crystal form of the oxalate salt of the compound represented by formula I-3B, the compound represented by formula I-3B and oxalic acid have a molar ratio of 1:1.

In a certain embodiment, the X-ray powder diffraction pattern for the crystal form of the oxalate salt of the compound represented by formula I-3B, using Cu-Kα radiation and expressed by 20 angles, further comprises diffraction peaks at one or more of 19.2 ± 0.2°, 15.4 ± 0.2°, 23.6 ± 0.2°, and 22.4 ± 0.2°.

In a certain embodiment, the X-ray powder diffraction pattern for the crystal form of the oxalate salt of the compound represented by formula I-3B, using Cu-Kα radiation and expressed by 20 angles, comprises diffraction peaks at 27.7 ± 0.2°, 7.8 ± 0.2°, 15.7 ± 0.2°, 17.1 ± 0.2°, 16.5 ± 0.2°, 19.2 ± 0.2°, and 15.4 ± 0.2°.

In a certain embodiment, the X-ray powder diffraction pattern for the crystal form of the oxalate salt of the compound represented by formula I-3B, using Cu-Kα radiation and expressed by 20 angles, comprises diffraction peaks at 27.7 ± 0.2°, 7.8 ± 0.2°, 15.7 ± 0.2°, 17.1 ± 0.2°, 16.5 ± 0.2°, 19.2 ± 0.2°, 15.4 ± 0.2°, 23.6 ± 0.2°, 22.4 ± 0.2°, and 28.5 ± 0.2°.

In a certain embodiment, the X-ray powder diffraction pattern for the crystal form of the oxalate salt of the compound represented by formula I-3B, using Cu-Kα radiation and expressed by 20 angles, comprises diffraction peaks shown in Table 14.

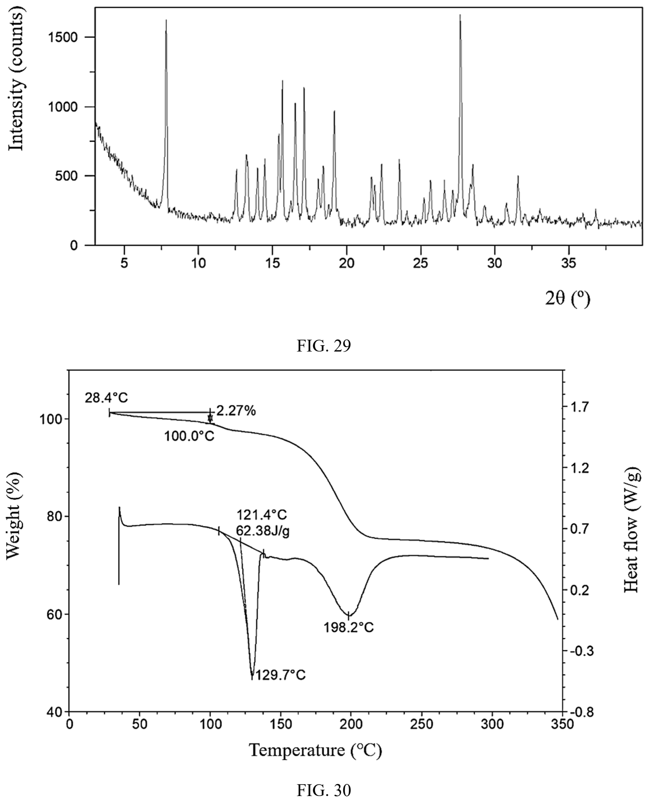

In a certain embodiment, the X-ray powder diffraction (XRPD) pattern for the crystal form of the oxalate salt of the compound represented by formula I-3B using Cu-Kα radiation is substantially shown in FIG. 29.

In a certain embodiment, the crystal form of the oxalate salt of the compound represented by formula I-3B has a differential scanning calorimetry (DSC) curve comprising endothermic peaks with peak temperatures at 129.7 ± 3°C and 198.2 ± 3°C.

In a certain embodiment, the differential scanning calorimetry (DSC) curve for the crystal form of the oxalate salt of the compound represented by formula I-3B is substantially shown in FIG. 30.

In a certain embodiment, the crystal form of the oxalate salt of the compound represented by formula I-3B has a thermogravimetric analysis (TGA) curve with a weight loss of approximately 2.27% in the temperature range of 28.4 ± 3°C to 100 ± 3°C.

In a certain embodiment, the thermogravimetric analysis (TGA) curve for the crystal form of the oxalate salt of the compound represented by formula I-3B is substantially shown in FIG. 30.

The present disclosure provides a preparation method for the crystal form of the compound represented by formula I-3B, comprising the following steps:
crystallizing a mixture of the compound represented by formula I-3B and a solvent to obtain the crystal form of the compound represented by formula I-3B or the solvate thereof; wherein
when the crystal form A of the compound represented by formula I-3B is obtained, the solvent is isopropyl acetate;
when the crystal form B of the compound represented by formula I-3B is obtained, the solvent is one or more of methanol, water, acetonitrile, and methyl *tert*-butyl ether.

In a certain embodiment, in the preparation method for the crystal form of the compound represented by formula I-3B, when the crystal form A of the compound represented by formula I-3B is obtained, the crystallizing involves cooling a mixture of isopropyl acetate and the compound represented by formula I-3B to room temperature, followed by drying (*e.g.,* evaporation) to obtain the crystal form A of the compound represented by formula I-3B.

In a certain embodiment, in the preparation method for the crystal form of the compound represented by formula I-3B, when the crystal form B of the compound represented by formula I-3B is obtained, the solvent is a mixture of methanol and water or a mixture of acetonitrile and methyl *tert*-butyl ether, preferably a mixture of methanol and water with a volume ratio of 1:5.

In a certain embodiment, in the preparation method for the crystal form of the compound represented by formula I-3B, when the crystal form B of the compound represented by formula I-3B is obtained, the crystallizing involves crystallizing a mixture of the compound represented by formula I-3B with methanol and water (*e.g*., a mixture of methanol and water with a volume ratio of 1:5) at 50°C (*e.g.,* stirring at 50°C for 3 days), followed by drying (*e.g.,* air-drying at room temperature) to obtain the crystal form B of the compound represented by formula I-3B.

The present disclosure provides a preparation method for the crystal form described above, comprising the following steps:
crystallizing a mixture of the compound represented by formula I-3B, an acid, and a solvent; wherein
when the crystal form of the hydrochloride salt of the compound represented by formula I-3B is obtained, the acid is hydrochloric acid, and the solvent is acetone;
when the crystal form of the phosphate salt of the compound represented by formula I-3B is obtained, the acid is phosphoric acid, and the solvent is a mixture of acetone and *n-*heptane;
when the crystal form of the fumarate complex of the compound represented by formula I-3B is obtained, the acid is fumaric acid, and the solvent is a mixture of acetone and *n*-heptane;
when the crystal form of the L-tartrate salt of the compound represented by formula I-3B is obtained, the acid is tartaric acid, and the solvent is a mixture of acetone and *n*-heptane;
when the crystal form of the *p*-toluenesulfonate salt of the compound represented by formula I-3B is obtained, the acid is *p*-toluenesulfonic acid, and the solvent is a mixture of acetone and *n*-heptane;
when the crystal form of the L-malate salt of the compound represented by formula I-3B is obtained, the acid is malic acid, and the solvent is a mixture of acetone and *n*-heptane;
when the crystal form A of the ethanedisulfonate salt of the compound represented by formula I-3B is obtained, the acid is ethanedisulfonic acid, and the solvent is isopropanol;
when the crystal form B of the ethanedisulfonate salt of the compound represented by formula I-3B is obtained, the acid is ethanedisulfonic acid, and the solvent is isopropyl acetate;
when the crystal form C of the ethanedisulfonate salt of the compound represented by formula I-3B is obtained, the acid is ethanedisulfonic acid, and the solvent is a mixture of acetone and *n*-heptane;
when the crystal form of the methanesulfonate salt of the compound represented by formula I-3B is obtained, the acid is methanesulfonic acid, and the solvent is a mixture of acetone and *n*-heptane;
when the crystal form of the benzenesulfonate salt of the compound represented by formula I-3B is obtained, the acid is benzenesulfonic acid, and the solvent is isopropanol;
when the crystal form of the oxalate salt of the compound represented by formula I-3B is obtained, the acid is oxalic acid, and the solvent is a mixture of acetone and *n*-heptane.

In a certain embodiment, in the preparation method, when the solvent is a mixture of acetone and *n*-heptane, the acetone and n-heptane have a volume ratio of 1:1.

In a certain embodiment, in the preparation method, the compound represented by formula I-3B and the acid have a molar ratio conventional in the art; for example, the heterocyclic compound and the acid have a molar ratio of 1:1.

In a certain embodiment, the preparation method comprises the following steps: suspending (*e.g*., stirring in suspension) the compound represented by formula I-3B, an acid, and a solvent to obtain a mixture, followed by crystallizing to obtain a crystal form.

In a certain embodiment, in the preparation method, when the crystal form of the hydrochloride salt of the compound represented by formula I-3B, the crystal form of the L-malate salt of the compound represented by formula I-3B, the crystal form B of the ethanedisulfonate salt of the compound represented by formula I-3B, the crystal form C of the ethanedisulfonate salt of the compound represented by formula I-3B, the crystal form of the methanesulfonate salt of the compound represented by formula I-3B, or the crystal form of the benzenesulfonate salt of the compound represented by formula I-3B is obtained, the crystallizing comprises the following steps: subjecting the mixture of the compound represented by formula I-3B, the acid, and the solvent to temperature cycling (*e.g.,* in the range of 50°C to 5°C, with a heating rate of 4.5°C/min and a cooling rate of 0.1°C/min, and maintaining the temperature at 50°C or 5°C for 2 hours for heating and cooling cycles), followed by drying (*e.g*., vacuum drying at room temperature).

In a certain embodiment, in the preparation method, when the crystal form of the phosphate salt of the compound represented by formula I-3B, the crystal form of the fumarate complex of the compound represented by formula I-3B, the crystal form of the L-tartrate salt of the compound represented by formula I-3B, the crystal form of the *p*-toluenesulfonate salt of the compound represented by formula I-3B, the crystal form A of the ethanedisulfonate salt of the compound represented by formula I-3B, or the crystal form of the oxalate salt of the compound represented by formula I-3B is obtained, the crystallizing comprises the following steps: subjecting the mixture of the compound represented by formula I-3B, the acid, and the solvent to drying (*e.g*., vacuum drying at room temperature).

The present disclosure provides a pharmaceutical composition, comprising the pharmaceutically acceptable salt of the heterocyclic compound or the solvate thereof (which refers to the solvate of the pharmaceutically acceptable salt of the heterocyclic compound) described above, and a pharmaceutically acceptable carrier.

The present disclosure provides a pharmaceutical composition, comprising a substance X and a pharmaceutically acceptable carrier, wherein the substance X is the crystal form A of the compound represented by formula I-3B, the crystal form B of the compound represented by formula I-3B, the crystal form of the hydrochloride salt of the compound represented by formula I-3B, the crystal form of the phosphate salt of the compound represented by formula I-3B, the crystal form of the fumarate complex of the compound represented by formula I-3B, the crystal form of the L-tartrate salt of the compound represented by formula I-3B, the crystal form of the *p*-toluenesulfonate salt of the compound represented by formula I-3B, the crystal form of the L-malate salt of the compound represented by formula I-3B, the crystal form A of the ethanedisulfonate salt of the compound represented by formula I-3B, the crystal form B of the ethanedisulfonate salt of the compound represented by formula I-3B, the crystal form C of the ethanedisulfonate salt of the compound represented by formula I-3B, the crystal form of the methanesulfonate salt of the compound represented by formula I-3B, the crystal form of the benzenesulfonate salt of the compound represented by formula I-3B, or the crystal form of the oxalate salt of the compound represented by formula I-3B described above.

In the pharmaceutical composition, the crystal form of the fumarate complex of the compound represented by formula I-3B may be the single crystal of the fumarate complex of the compound represented by formula I-3B described above.

The present disclosure provides a use of the pharmaceutically acceptable salt of the heterocyclic compound or the solvate thereof (which refers to the solvate of the pharmaceutically acceptable salt of the heterocyclic compound), the pharmaceutical composition, or the substance X;
the substance X is the crystal form A of the compound represented by formula I-3B, the crystal form B of the compound represented by formula I-3B, the crystal form of the hydrochloride salt of the compound represented by formula I-3B, the crystal form of the phosphate salt of the compound represented by formula I-3B, the crystal form of the fumarate complex of the compound represented by formula I-3B, the crystal form of the L-tartrate salt of the compound represented by formula I-3B, the crystal form of the p-toluenesulfonate salt of the compound represented by formula I-3B, the crystal form of the L-malate salt of the compound represented by formula I-3B, the crystal form A of the ethanedisulfonate salt of the compound represented by formula I-3B, the crystal form B of the ethanedisulfonate salt of the compound represented by formula I-3B, the crystal form C of the ethanedisulfonate salt of the compound represented by formula I-3B, the crystal form of the methanesulfonate salt of the compound represented by formula I-3B, the crystal form of the benzenesulfonate salt of the compound represented by formula I-3B, or the crystal form of the oxalate salt of the compound represented by formula I-3B described above;
the use comprises: inhibiting 15-PGDH; and/or preventing and/or treating a disease related to 15-PGDH; and/or preparing a 15-PGDH inhibitor; and/or preparing a medicament, pharmaceutical composition, or formulation for preventing and/or treating the disease related to 15-PGDH.

Preferably, the disease related to 15-PGDH includes, but is not limited to, one, two, or more of fibrotic disease, inflammatory disease, cardiovascular disease, trauma, autoimmune disease, graft-versus-host disease, hair growth, osteoporosis, ear disease, eye disease, neutropenia, diabetes, underactive bladder, implant promotion in stem cell or bone marrow transplantation or organ transplantation, neurogenesis and neuronal cell death, hematopoietic reconstruction, tissue injury, cervical disease, and kidney disease.

Preferably, the disease related to 15-PGDH includes, but is not limited to, one, two, or more of fibrotic disease, inflammatory disease, cardiovascular disease, trauma, autoimmune disease, graft-versus-host disease, hair growth, osteoporosis, ear disease, eye disease, neutropenia, diabetes, underactive bladder, implant promotion in stem cell or bone marrow transplantation or organ transplantation, neurogenesis and neuronal cell death, hematopoietic reconstruction, tissue injury, cervical disease, and kidney disease. Preferably, the disease related to 15-PGDH includes, but is not limited to, one, two, or more of fibrotic disease (*e.g.,* pulmonary fibrosis, including idiopathic pulmonary fibrosis, liver fibrosis, renal fibrosis, myocardial fibrosis, scleroderma, and myelofibrosis), inflammatory disease (*e.g*., chronic obstructive pulmonary disease (COPD), acute lung injury, sepsis, exacerbation of asthma and pulmonary disease, inflammatory bowel disease (IBD) (e.g., ulcerative colitis and Crohn's disease), peptic ulcer (*e.g.,* NSAID-induced ulcer), auto inflammatory disease (*e.g.,* Behcet's disease), vasculitis syndrome, acute liver injury, acute kidney injury, non-alcoholic fatty liver disease (NASH), atopic dermatitis, psoriasis, interstitial cystitis, prostatitis syndrome (*e.g*., chronic prostatitis/chronic pelvic pain syndrome)), cardiovascular disease (*e.g.,* pulmonary hypertension, angina, myocardial infarction, heart failure, ischemic heart disease, stroke, and peripheral circulatory disorder), kidney disease (*e.g.,* chronic kidney disease and renal failure), trauma (*e.g.,* diabetic ulcer, burn, pressure ulcer, acute mucosal injury, including Stevens-Johnson syndrome, mucosal injury (*e.g.,* mucositis or stomatitis), injury related to anticancer chemotherapeutic agents (particularly alkylating agents, DNA synthesis inhibitors, or DNA gyrase inhibitors) or injury related to antimetabolites, cellular or humoral immunotherapy or radiation), autoimmune disease (*e.g.,* multiple sclerosis or rheumatoid arthritis), graft-versus-host disease (GVHD), hair growth, osteoporosis, ear disease (*e.g.,* hearing loss, tinnitus, vertigo, and balance disorder), eye disease (*e.g.,* glaucoma and dry eye), neutropenia, diabetes, underactive bladder, implant promotion in stem cell or bone marrow transplantation or organ transplantation, neurogenesis and neuronal cell death (*e.g.,* neuropsychiatric disorder, neuropathy, neurotoxic disease, neuropathic pain, and neurodegenerative disease), liver regeneration, muscle regeneration (*e.g.,* muscle atrophy, muscular dystrophy, and muscle injury), and cervical disease.

Preferably, the tissue injury is liver injury and/or muscle injury (*e.g.,* muscle atrophy and muscular dystrophy).

Preferably, the disease related to 15-PGDH includes, but is not limited to, idiopathic pulmonary fibrosis (IPF).

Preferably, the prevention and/or treatment of the disease related to 15-PGDH includes, but is not limited to, liver regeneration.

Preferably, the disease related to 15-PGDH includes, but is not limited to, liver injury.

Preferably, the disease related to 15-PGDH includes, but is not limited to, inflammatory bowel disease (IBD).

The present disclosure provides a use of the pharmaceutically acceptable salt of the heterocyclic compound or the solvate thereof (which refers to the solvate of the pharmaceutically acceptable salt of the heterocyclic compound), the pharmaceutical composition, or the substance X, wherein the use is for preventing and/or treating a disease as follows, and/or the use is for preparing a medicament for preventing or treating a disease as follows; the disease is one or more of fibrotic disease, inflammatory disease, or tissue injury;

the substance X is the crystal form A of the compound represented by formula I-3B, the crystal form B of the compound represented by formula I-3B, the crystal form of the hydrochloride salt of the compound represented by formula I-3B, the crystal form of the phosphate salt of the compound represented by formula I-3B, the crystal form of the fumarate complex of the compound represented by formula I-3B, the crystal form of the L-tartrate salt of the compound represented by formula I-3B, the crystal form of the *p*-toluenesulfonate salt of the compound represented by formula I-3B, the crystal form of the L-malate salt of the compound represented by formula I-3B, the crystal form A of the ethanedisulfonate salt of the compound represented by formula I-3B, the crystal form B of the ethanedisulfonate salt of the compound represented by formula I-3B, the crystal form C of the ethanedisulfonate salt of the compound represented by formula I-3B, the crystal form of the methanesulfonate salt of the compound represented by formula I-3B, the crystal form of the benzenesulfonate salt of the compound represented by formula I-3B, or the crystal form of the oxalate salt of the compound represented by formula I-3B described above;

The fibrotic disease, the inflammatory disease, and the tissue injury may be as described above.

In the use, the crystal form of the fumarate complex of the compound represented by formula I-3B may be the single crystal of the fumarate complex of the compound represented by formula I-3B described above.

### Explanation of terms:

Unless otherwise specified, the definitions of groups and terms described in the description and claims include definitions thereof as examples, exemplary definitions, preferred definitions, definitions recorded in tables, and definitions of specific compounds in the examples, *etc.,* which can be arbitrarily combined and integrated with each other. Such combined and integrated definitions and compound structures shall fall within the scope of the description of the present disclosure. Unless otherwise specified, the terms used in the present disclosure have the following meanings:

The term "pharmaceutically acceptable carrier" includes, but is not limited to, any adjuvant, carrier, excipient, glidant, sweetener, diluent, preservative, dye/colorant, flavoring agent, surfactant, wetting agent, dispersing agent, suspending agent, stabilizer, isotonic agent, solvent, or emulsifier that is approved by relevant governmental authorities for use in humans or domestic animals.

The term "treatment" refers to therapeutic therapy. When referring to a specific disorder, treatment refers to: (1) ameliorating one or more biological manifestations of the disease or disorder, (2) interfering with (a) one or more points in the biological cascade leading to or causing the disorder or (b) one or more biological manifestations of the disorder, (3) ameliorating one or more symptoms, effects, or side effects associated with the disorder, or one or more symptoms, effects or side effects associated with the disorder or its treatment, or (4) slowing the progression of the disorder or one or more biological manifestations of the disorder.

The term "prevention" refers to the reduction of the risk of acquiring or developing a disease or disorder.

The term "inflammatory bowel disease" refers to IBD and is used to describe diseases involving chronic inflammation of the digestive tract. It mainly includes: ulcerative colitis and Crohn's disease. Ulcerative colitis causes inflammation and ulcers in the superficial lining of the large intestine (colon) and rectum. Crohn's disease is characterized by inflammation of the lining of the digestive tract, and the inflammation usually affects the deeper layers of the digestive tract.

On the basis of common knowledge in the art, the above preferred conditions can be arbitrarily combined to obtain preferred examples of the present disclosure.

The reagents and starting materials used in the present disclosure are all commercially available.

The positive and progressive effects of the present disclosure are that the crystal form of the heterocyclic compound provided by the present disclosure and the pharmaceutically acceptable salt of the heterocyclic compound or the solvate thereof have good efficacy and druggability.

### BRIEF DESCRIPTION OF THE DRAWINGS

FIG. 1 shows an X-ray powder diffraction pattern for the crystal form A of the compound represented by formula I-3B.
FIG. 2 shows a differential scanning calorimetry curve and a thermogravimetric analysis curve for the crystal form A of the compound represented by formula I-3B.
FIG. 3 shows a temperature-variable XRPD pattern for the crystal form A of the compound represented by formula I-3B.
FIG. 4 shows an X-ray powder diffraction pattern for the crystal form B of the compound represented by formula I-3B.
FIG. 5 shows a differential scanning calorimetry curve and a thermogravimetric analysis curve for the crystal form B of the compound represented by formula I-3B.
FIG. 6 shows a temperature-variable XRPD pattern for the crystal form B of the compound represented by formula I-3B.
FIG. 7 shows an X-ray powder diffraction pattern for the crystal form of the hydrochloride salt of the compound represented by formula I-3B.
FIG. 8 shows a differential scanning calorimetry curve and a thermogravimetric analysis curve for the crystal form of the hydrochloride salt of the compound represented by formula I-3B.
FIG. 9 shows an X-ray powder diffraction pattern for the crystal form of the phosphate salt of the compound represented by formula I-3B.
FIG. 10 shows a differential scanning calorimetry curve and a thermogravimetric analysis curve for the crystal form of the phosphate salt of the compound represented by formula I-3B.
FIG. 11 shows an X-ray powder diffraction pattern for the crystal form of the fumarate complex of the compound represented by formula I-3B.
FIG. 12 shows a differential scanning calorimetry curve and a thermogravimetric analysis curve for the crystal form of the fumarate complex of the compound represented by formula I-3B.
FIG. 13 shows an X-ray powder diffraction pattern for the crystal form of the L-tartrate salt of the compound represented by formula I-3B.
FIG. 14 shows a differential scanning calorimetry curve and a thermogravimetric analysis curve for the crystal form of the L-tartrate salt of the compound represented by formula I-3B.
FIG. 15 shows an X-ray powder diffraction pattern for the crystal form of the *p-*toluenesulfonate salt of the compound represented by formula I-3B.
FIG. 16 shows a differential scanning calorimetry curve and a thermogravimetric analysis curve for the crystal form of the *p*-toluenesulfonate salt of the compound represented by formula I-3B.
FIG. 17 shows an X-ray powder diffraction pattern for the crystal form of the L-malate salt of the compound represented by formula I-3B.
FIG. 18 shows a differential scanning calorimetry curve and a thermogravimetric analysis curve for the crystal form of the L-malate salt of the compound represented by formula I-3B.
FIG. 19 shows an X-ray powder diffraction pattern for the crystal form A of the ethanedisulfonate salt of the compound represented by formula I-3B.
FIG. 20 shows a differential scanning calorimetry curve and a thermogravimetric analysis curve for the crystal form A of the ethanedisulfonate salt of the compound represented by formula I-3B.
FIG. 21 shows an X-ray powder diffraction pattern for the crystal form B of the ethanedisulfonate salt of the compound represented by formula I-3B.
FIG. 22 shows a differential scanning calorimetry curve and a thermogravimetric analysis curve for the crystal form B of the ethanedisulfonate salt of the compound represented by formula I-3B.
FIG. 23 shows an X-ray powder diffraction pattern for the crystal form C of the ethanedisulfonate salt of the compound represented by formula I-3B.
FIG. 24 shows a differential scanning calorimetry curve and a thermogravimetric analysis curve for the crystal form C of the ethanedisulfonate salt of the compound represented by formula I-3B.
FIG. 25 shows an X-ray powder diffraction pattern for the crystal form of the methanesulfonate salt of the compound represented by formula I-3B.
FIG. 26 shows a differential scanning calorimetry curve and a thermogravimetric analysis curve for the crystal form of the methanesulfonate salt of the compound represented by formula I-3B.
FIG. 27 shows an X-ray powder diffraction pattern for the crystal form of the benzenesulfonate salt of the compound represented by formula I-3B.
FIG. 28 shows a differential scanning calorimetry curve and a thermogravimetric analysis curve for the crystal form of the benzenesulfonate salt of the compound represented by formula I-3B.
FIG. 29 shows an X-ray powder diffraction pattern for the crystal form of the oxalate salt of the compound represented by formula I-3B.
FIG. 30 shows a differential scanning calorimetry curve and a thermogravimetric analysis curve for the crystal form of the oxalate salt of the compound represented by formula I-3B.
FIG. 31 shows a diagram of the single crystal structure of compound R1.
FIG. 32 shows an XRPD pattern of the dynamic solubility of the compound represented by formula I-3B in H₂O.
FIG. 33 shows an XRPD pattern of the dynamic solubility of the compound represented by formula I-3B in SGF.
FIG. 34 shows an XRPD pattern of the dynamic solubility of the compound represented by formula I-3B in FaSSIF.
FIG. 35 shows an XRPD pattern of the dynamic solubility of the compound represented by formula I-3B in FeSSIF.
FIG. 36 shows an XRPD pattern of the dynamic solubility of the crystal form of the fumarate complex of the compound represented by formula I-3B in H₂O.
FIG. 37 shows an XRPD pattern of the dynamic solubility of the crystal form of the fumarate complex of the compound represented by formula I-3B in SGF.
FIG. 38 shows an XRPD pattern of the dynamic solubility of the crystal form of the fumarate complex of the compound represented by formula I-3B in FaSSIF.
FIG. 39 shows an XRPD pattern of the dynamic solubility of the crystal form of the fumarate complex of the compound represented by formula I-3B in FeSSIF.
FIG. 40 shows an XRPD pattern of the stability evaluation for the crystal form A of the compound represented by formula I-3B.
FIG. 41 shows an XRPD pattern of the stability evaluation for the crystal form B of the compound represented by formula I-3B.
FIG. 42 shows an XRPD pattern of the stability evaluation for the crystal form of the fumarate complex of the compound represented by formula I-3B.
FIG. 43 shows an XRPD pattern of the stability evaluation for the crystal form of the L-tartrate salt of the compound represented by formula I-3B.
FIG. 44 shows an XRPD pattern for the crystal form A of the compound represented by formula I-3B before and after hygroscopicity testing.
FIG. 45 shows an XRPD pattern for the crystal form B of the compound represented by formula I-3B before and after hygroscopicity testing.
FIG. 46 shows an XRPD pattern for the crystal form of the fumarate complex of the compound represented by formula I-3B before and after hygroscopicity testing.
FIG. 47 shows an XRPD pattern for the crystal form of the L-tartrate salt of the compound represented by formula I-3B before and after hygroscopicity testing.
FIG. 48 shows a comparison between the fitted XRPD pattern and the measured XRPD pattern for the crystal form of the fumarate complex of the compound represented by formula I-3B.
FIG. 49 shows an ellipsoid diagram of the molecular structure of the crystal form of the fumarate complex of the compound represented by formula I-3B.

### DETAILED DESCRIPTION OF THE PREFERRED EMBODIMENT

The present disclosure will be described in detail below by way of examples, but the scope of the present disclosure is not limited thereto. Experimental methods that do not indicate specific conditions in the following examples are selected according to conventional methods and conditions, or according to product instructions.

### Symbols or units:

IC₅₀: Half maximal inhibitory concentration, which refers to the concentration at which half of the maximum inhibitory effect is achieved.

M: mol/L, for example, *n*-butyllithium (14.56 mL, 29.1 mmol, 2.5 M in *n*-hexane) indicates a solution of *n*-butyllithium in *n*-hexane with a molar concentration of 2.5 mol/L.

N: normality, for example, 2 N hydrochloric acid indicates a hydrochloric acid solution with a concentration of 2 mol/L.

### Reagents:

Comparison table of Chinese and English names of solvents used in the examples

| **English** | **Chinese** | **English** | **Chinese** |
|---|---|---|---|
| MeOH | Methanol | 1,4-Dioxane | 1,4-Dioxane |
| EtOH | Ethanol | ACN | Acetonitrile |
| IPA | Isopropanol | DCM | Dichloromethane |
| Acetone | Acetone | CHCl₃ | Chloroform |
| MIBK | Methyl isobutyl ketone | Toluene | Toluene |
| EtOAc | Ethyl acetate | *n*-Heptane | *n*-Heptane |
| IPAc | Isopropyl acetate | DMSO | Dimethyl sulfoxide |
| MTBE | Methyl *tert*-butyl ether | DMAc | *N,N*-Dimethylacetamide |
| THF | Tetrahydrofuran | NMP | *N*-Methylpyrrolidone |
| 2-MeTHF | 2-Methyltetrahydrofuran | H₂O | Water |
| *n*-BuOH | *n*-Butanol | Anisole | Anisole |
| CPME | Cyclopentyl methyl ether | Butyl acetate | Butyl acetate |
| Cumene | Cumene | Butyl butyrate | Butyl butyrate |
| Cyclohexane | Cyclohexane | m-Xylene | m-Xylene |
| 2-Hexanone | 2-Hexanone | Isopropyl benzene | Isopropyl benzene |
| Cyclohexanol | Cyclohexanol | Methylcyclohexane | Methylcyclohexane |
| Nitromethane | Nitromethane | *n*-Hexane | *n*-Hexane |
| MEK | Methyl ethyl ketone | 1-PrOH | *n*-Propanol |
| Benzonitrile | Benzonitrile | Isoamyl acetate | Isoamyl acetate |
| PAc | Propyl acetate | i-BuOH | Isobutanol |
| BuOAc | Butyl acetate | -- | -- |

### Instruments and methods for crystal forms:

### 1. X-ray powder diffraction (XRPD)

XRPD patterns were collected on an X-ray powder diffractometer produced by PANalytacal. The scanning parameters are shown in the following table:

| **Parameter** | **VT-XRPD** | **XRPD** |
|---|---|---|
| Model | Empyrean | Empyrean/X' Pert3 |
| X-ray | Cu, Kα, | Cu, Kα, |
| | Kα1 (Å): 1.540598, | Kα1 (Å): 1.540598, |
| | Kα2 (Å): 1.544426 | Kα2 (Å): 1.544426 |
| | Kα2/Kα1 intensity ratio: 0.50 | Kα2/Kα1 intensity ratio: 0.50 |
| X-ray tube setting | 45 kV, 40 mA | 45 kV, 40 mA |
| Divergence slit | 1/8° | 1/8° |
| Scan mode | Continuous | Continuous |
| Scan range (°2θ) | 3-40 | 3-40 |
| Scan time per step (s) | 33.0 | 46.7 |
| Scan step size (°2θ) | 0.0167 | 0.0263 |
| Test time | Approximately 10 minutes and 13 seconds | Approximately 5 minutes |

### 2. X-ray single crystal diffraction

Test conditions: test temperature: 200(2) K, room temperature: 24°C, relative humidity: 34%. The X-ray single crystal diffraction method is as follows:

| **Parameter** | |
|---|---|
| Model | D8 Venture |
| X-ray tube | Ga-target |
| Wavelength | 1.34139 Å |
| Tube voltage | 70 kV |
| Tube current | 3.57 mA |
| Scan mode | ω-2θ scan |
| Scan rate | 0.5° per frame |
| Scan range | 2.32 to 72.28° |
| Test temperature | 200(2) K |

### 3. Thermogravimetric analysis (TGA) and differential scanning calorimetry (DSC)

TGA and DSC patterns were collected on a TA 5500 thermogravimetric analyzer and a TA 2500 differential scanning calorimeter, respectively. The test parameters are listed in the following table:

| **Parameter** | **TGA** | **DSC** |
|---|---|---|
| Method | Linear heating | Linear heating |
| Sample tray | Aluminum pan, open | Aluminum pan, with lid/without lid |
| Temperature range | Room temperature-set endpoint temperature | 25°C-set endpoint temperature |
| Scan rate (°C/min) | 10 | 10 |
| Protective gas | Nitrogen | Nitrogen |

### 4. Solution NMR:

Liquid NMR spectra were collected on a Bruker 400M NMR spectrometer, using DMSO-*d*₆ as the solvent.

### 5. High-performance liquid chromatography (HPLC):

The purity test, dynamic solubility test, and stability test in the assay were performed using an ultra-performance liquid chromatograph (UPLC). The analytical conditions are shown in the following table:

| | | |
|---|---|---|
| **Liquid chromatograph** | **Waters H-Class** | |
| Chromatographic column | Acquity UPLC BEH C18, 50 × 2.1 mm, 1.7 µm | |
| Mobile phase | A: 0.1% TFA in H₂O | |
| | B: 0.1% TFA in ACN | |
| | **Purity/solubility test** | |
| | **Time (min)** | **%B** |
| | 0.0 | 5 |
| | 13.0 | 60 |
| | 15.0 | 95 |
| | 17.0 | 95 |
| | 17.1 | 5 |
| | 20.0 | 5 |
| Run time | 20.0 min | |
| Flow rate for mobile phase | 0.5 mL/min | |
| Injection volume | 1 µL | |
| Detection wavelength | UV at 280 nm | |
| Column temperature | 40°C | |
| Injector temperature | RT | |
| Diluent | ACN/H₂O = 1:1 (v:v) | |

### 6. Dynamic vapor sorption (DVS):

Dynamic vapor sorption (DVS) curves were collected on DVS Intrinsic Plus from SMS (Surface Measurement Systems). The relative humidity at 25°C was corrected using the deliquescence points of LiCl, Mg(NO₃)₂, and KCl. The DVS test parameters are listed in the following table:

| **Parameter** | **Set value** |
|---|---|
| Temperature | 25°C |
| Sample size | 10-20 mg |
| Protective gas and flow rate | N₂, 200 mL/min |
| dm/dt | 0.002%/min |
| Minimum dm/dt equilibration time | 10 min |
| Maximum equilibrium time | 180 min |
| RH range | 0% RH-95% RH-0% RH |
| | 30% RH-95% RH-0% RH-95% RH |
| RH gradient | 10% (0% RH-90 %RH, 90% RH-0% RH) |
| | 5% (90% RH-95% RH, 95% RH-90% RH) |

### 7. Ion chromatography (IC):

The instrumental parameters for determining the molar ratio of counterions by ion chromatography (IC) are listed in the analytical conditions as shown in the following table:

| **Parameter** | **Ion chromatography (Thermo ICS1100)** |
|---|---|
| Mode | Anion |
| Chromatographic column | Dionex IonPac^{™} AS18 RFIC^{™} 4 × 250 mm Analytical |
| Mobile phase | 25 mM NaOH |
| Injection volume | 25 µL |
| Flow rate | 1.0 mL/min |
| Sample chamber temperature | 35°C |
| Column temperature | 35°C |
| Current | 80 mA |
| Run time | 20 min (Cl⁻, PO₄³⁻, C₂O₄²⁻) |

### Example 1: Preparation of 7-(6-(4,4-difluoropiperidine-1-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one

The synthetic route of the target compound was as follows:

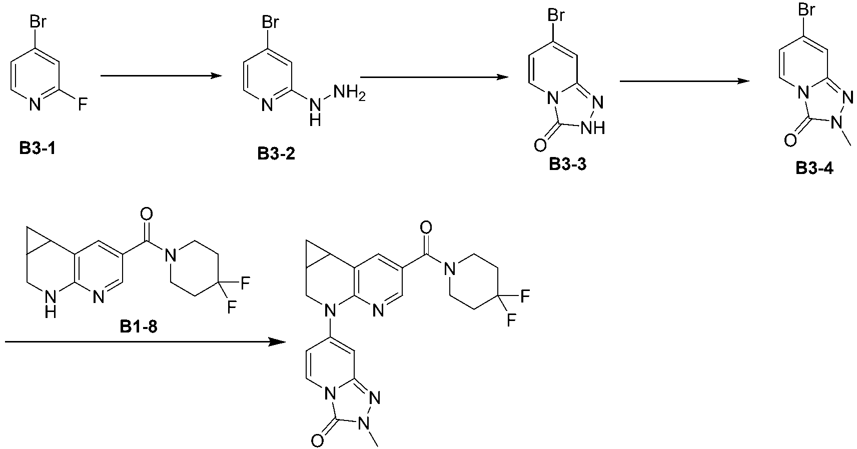

### Step 1: 4-Bromo-2-hydrazinopyridine (B3-2)

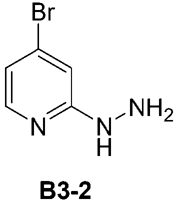

To a solution of 4-bromo-2-fluoropyridine (5 g, 28.4 mmol) in ethanol (50 mL) was added 80% hydrazine hydrate solution (17 mL). The reaction mixture was stirred at room temperature for 12 hours and concentrated. The residue was diluted with water (50 mL) and extracted with ethyl acetate (50 mL * 3). The organic phases were combined, dried over anhydrous sodium sulfate, filtered, and subjected to rotary evaporation until dryness to obtain 4-bromo-2-hydrazinopyridine (3.7 g, yield: 69.3%).

LC-MS, M/Z (ESI): 187.9 [M+H]⁺.

### Step 2: 7-Bromo-[1,2,4]triazolo[4,3-a]pyridin-3(2H)-one (B3-3)

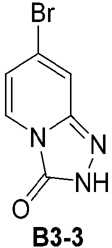

4-Bromo-2-hydrazinopyridine (3.2 g, 17.02 mmol) was dissolved in tetrahydrofuran (30 mL), and carbonyldiimidazole (5.52 g, 34.0 mmol) was added thereto. The reaction mixture was stirred at room temperature for 12 hours, diluted with water (50 mL), and extracted with ethyl acetate (50 mL * 3). The organic phases were combined, dried over anhydrous sodium sulfate, filtered, subjected to rotary evaporation until dryness, and slurried with ethyl acetate (10 mL) to obtain 7-bromo-[1,2,4]triazolo[4,3-*a*]pyridin-3(2*H*)-one (2.2 g, yield: 60.4%).

LC-MS, M/Z (ESI): 213.9 [M+H]⁺.

### Step 3: 7-Bromo-2-methyl-[1,2,4]triazolo[4,3-a]pyridin-3(2H)-one (B3-4)

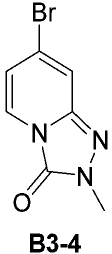

To a mixture of 7-bromo-[1,2,4]triazolo[4,3-*a*]pyridin-3(2*H*)-one (500 mg, 2.33 mmol) and cesium carbonate (1.14 g, 3.50 mmol) in DMF (5 mL) was added dropwise iodomethane (995 mg, 7.01 mmol) at room temperature. The reaction mixture was stirred at room temperature for 3 hours, diluted with water (20 mL), and extracted with ethyl acetate (20 mL * 3). The organic phases were combined, dried over anhydrous sodium sulfate, filtered, subjected to rotary evaporation until dryness, and the crude product was purified by silica gel column chromatography (petroleum ether: ethyl acetate (V/V) = 5:1 to 1:1) to obtain 7-bromo-2-methyl-[1,2,4]triazolo[4,3-*a*]pyridin-3(2*H*)-one (400 mg, yield: 75%).

LC-MS, M/Z (ESI): 227.9 [M+H]⁺.

### Step 4: 7-(6-(4,4-Difluoropiperidine-1-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one

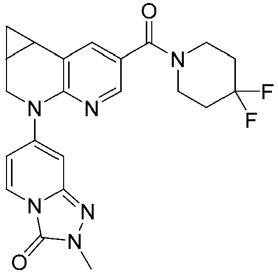

To dioxane (1 mL) were added (4,4-difluoropiperidin-1-yl)(la,2,3,7b-tetrahydro-1*H-*cyclopropa[c][1,8]naphthyridin-6-yl)methanone (35.0 mg, 119 µmol), cesium carbonate (155 mg, 477 µmol), 7-bromo-2-methyl-[1,2,4]triazolo[4,3-*a*]pyridin-3(2*H*)-one (40.8 mg, 179 µmol), bis(dibenzylideneacetone)palladium (6.86 mg, 11.9 µmol), and 4,5-bis(diphenylphosphino)-9,9-dimethylxanthene (69.0 mg, 119 µmol) under nitrogen atmosphere. The reaction mixture was stirred at 100°C for 12 hours and concentrated to obtain a crude product. The crude product was then purified by silica gel column chromatography (petroleum ether: ethyl acetate (V/V) = 5:1 to 1:1) to obtain the target compound 7-(6-(4,4-difluoropiperidine-1-carbonyl)-1,1a,2,7b-tetrahydro-3*H-*cyclopropa[*c*][1,8]naphthyridin-3-yl)-2-methyl-[1,2,4]triazolo[4,3-*a*]pyridin-3(2*H*)-one (**compound I-3**) (20.0 mg, yield: 38.1%).

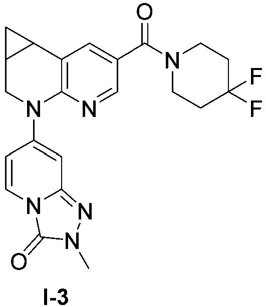

The resulting product was separated by SFC (chromatographic column: Chiralpak AD-3 50 × 4.6 mm I.D., 3 µm; mobile phase: mobile phase A: CO₂, mobile phase B: IPA + ACN (0.05 vol% DEA); isocratic elution: 50 vol% IPA + ACN (0.05 vol% DEA) in CO₂; flow rate: 3 mL/min; detector: PDA, column temperature: 35°C; column pressure: 100 Bar).

An isomer (compound I-3A (*i.e.,* the compound represented by formula I-3A in the present disclosure)) (RT = 0.743 min) and an isomer (compound I-3B (*i.e.,* the compound represented by formula I-3B in the present disclosure), whose structure was confirmed as follows) (RT = 1.670 min) were obtained.

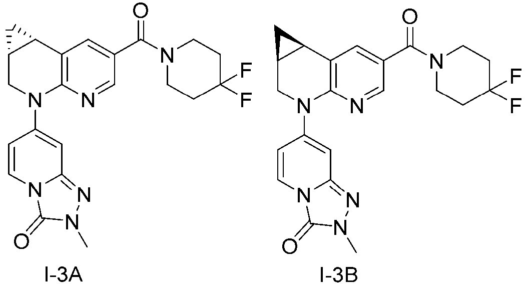

¹H NMR (400 MHz, DMSO) δ 8.04 (d, 1H), 7.81 (d, 1H), 7.61 (d, 1H), 6.68 (d, 1H), 6.58 (dd, 2.0 Hz, 1H), 3.90 (d, 1H), 3.70 - 3.52 (m, 5H), 3.48 (s, 3H), 2.18 (dt, 1H), 2.13 - 1.93 (m, 5H), 1.06 (dd, 2H).

LC-MS, M/Z (ESI): 441.1 [M+H]⁺.

### Example 1-1: Preparation of compound represented by formula I-3B

The synthetic route of the target compound was as follows:

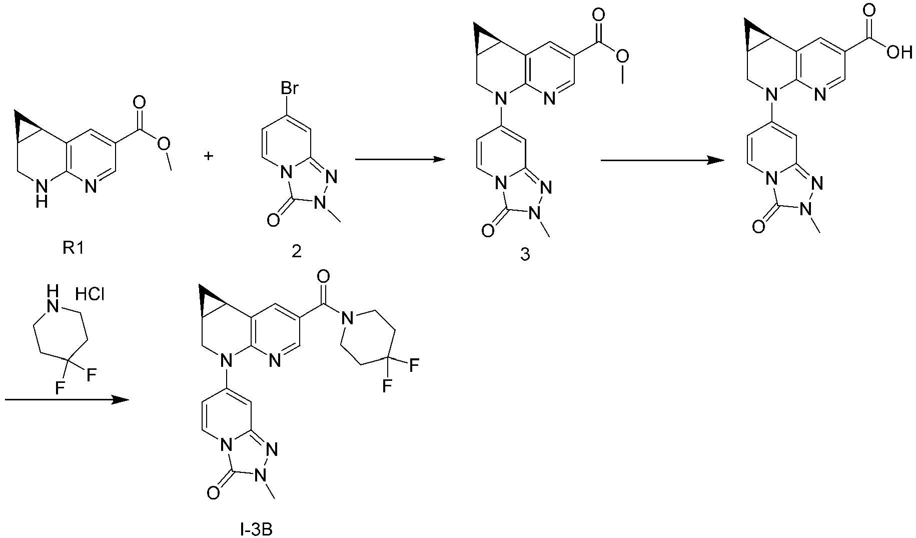

### Step 1: Synthesis of methyl (1aS,7bR)-3-(2-methyl-3-oxo-2,3-dihydro-[1,2,4]triazolo[4,3-a]pyridin-7-yl)-1a,2,3,7b-tetrahydro-1H-cyclopropa[c][1,8]naphthyridine-6-carboxylate (3).

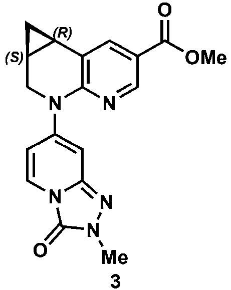

To a reaction flask were added methyl (1a*S*,7b*R*)-1a,2,3,7b-tetrahydro-1*H-*cyclopropa[*c*][1,8]naphthyridine-6-carboxylate (**R1**) (159.6 mg, 0.7 mmol), 7-bromo-2-methyl-[1,2,4]triazolo[4,3-*a*]pyridin-3(2*H*)-one (**2**) (130 mg, 0.6 mmol), Pd₂(dba)₃ (91.6 mg, 0.1 mmol), ligand L (ligand L is Xantphos) (57.9 mg, 0.1 mmol), and cesium carbonate (488.7 mg, 1.5 mmol). The reaction system was replaced with nitrogen three times, then added with anhydrous 1,4-dioxane (4 mL), and stirred at 85°C for 12 hours. The reaction mixture was cooled to room temperature, then quenched with water, and extracted with ethyl acetate. The organic phase was concentrated to dryness, and the residue was loaded onto silica gel and purified by column chromatography (dichloromethane: methanol (V/V) = 20:1) to obtain methyl (1a*S*,7b*R*)-3-(2-methyl-3-oxo-2,3-dihydro-[1,2,4]triazolo[4,3-*a*]pyridin-7-yl)-1a,2,3,7b-tetrahydro-1*H*-cyclopropa[*c*][1,8]naphthyridine-6-carboxylate (**3**) (200 mg, yield: 85.7%).

LC-MS, M/Z (ESI): 352.1 [M+H]⁺.

Ligand L:

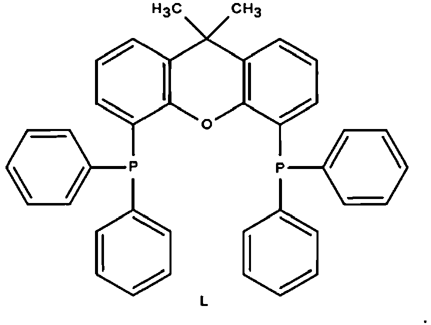

### Step 2: Synthesis of (1aS,7bR)-3-(2-methyl-3-oxo-2,3-dihydro-[1,2,4]triazolo[4,3-a]pyridin-7-yl)-1a,2,3,7b-tetrahydro-1H-cyclopropa[c][1,8]naphthyridine-6-carboxylic acid (4).

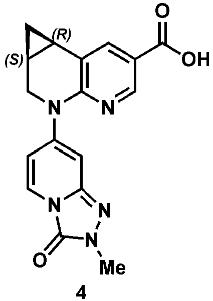

Methyl (1a*S*,7b*R*)-3-(2-methyl-3-oxo-2,3-dihydro-[1,2,4]triazolo[4,3-*a*]pyridin-7-yl)-1a,2,3,7b-tetrahydro-1*H*-cyclopropa[*c*][1,8]naphthyridine-6-carboxylate (**3**) (200 mg, 0.6 mmol) was dissolved in a mixture of tetrahydrofuran (4 mL), methanol (0.8 mL), and water (0.8 mL), and lithium hydroxide monohydrate (100 mg, 2.4 mmol) was added thereto. The resulting mixture was stirred at room temperature for 12 hours. After the reaction was completed, the reaction mixture was quenched with water, added with 1 M hydrochloric acid to adjust the pH to 1, and extracted with ethyl acetate. The organic phase was concentrated to dryness to obtain (1a*S*,7b*R*)-3-(2-methyl-3-oxo-2,3-dihydro-[1,2,4]triazolo[4,3-*a*]pyridin-7-yl)-1a,2,3,7b-tetrahydro-1*H*-cyclopropa[*c*][1,8]naphthyridine-6-carboxylic acid (**4**), which was directly used in the next reaction step.

LC-MS, M/Z (ESI): 338.1 [M+H]⁺.

### Step 3: Synthesis of 7-((1aS,7bR)-6-(4,4-difluoropiperidine-1-carbonyl)-1,1a,2,7b-tetrahydro-3H-cyclopropa[c][1,8]naphthyridin-3-yl)-2-methyl-[1,2,4]triazolo[4,3-a]pyridin-3(2H)-one (5).

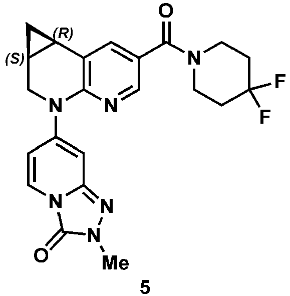

To a solution of (1a*S*,7b*R*)-3-(2-methyl-3-oxo-2,3-dihydro-[1,2,4]triazolo[4,3-*a*]pyridin-7-yl)-1a,2,3,7b-tetrahydro-1*H*-cyclopropa[*c*][1,8]naphthyridine-6-carboxylic acid (**4**) (120 mg, 0.4 mmol) in ethyl acetate (2 mL) were added 4,4-difluoropiperidine hydrochloride (72.7 mg, 0.6 mmol) and pyridine (0.2 mL) under nitrogen atmosphere, followed by the addition of a solution of T₃P in DMF (0.68 mL, 0.5 mmol), and the resulting mixture was stirred at room temperature for 3 hours. The reaction mixture was quenched with water and extracted with ethyl acetate. The organic phase was concentrated to dryness, and the residue was loaded onto silica gel and purified by column chromatography (dichloromethane: methanol (V/V) = 20:1) to obtain 7-((1a*S*,7b*R*)-6-(4,4-difluoropiperidine-1-carbonyl)-1,1a,2,7b-tetrahydro-3*H*-cyclopropa[*c*][1,8]naphthyridin-3-yl)-2-methyl-[1,2,4]triazolo[4,3-*a*]pyridin-3(2*H*)-one (**5**) (132 mg, yield: 75%).

LC-MS, M/Z (ESI): 441.1 [M+H]⁺.

¹H NMR (400 MHz, DMSO-*d*₆): δ 8.09 (d, 1H), 7.71 (d, 1H), 7.59 (d, 1H), 6.55 - 6.52 (m, 2H), 3.95 (d, 1H), 3.75 - 3.69 (m, 5H), 3.63 (s, 3H), 2.10 - 2.03 (m, 6H), 1.22 - 1.12 (m, 2H) ppm.

The resulting product was subjected to SFC (chromatographic column: ChiralpakAD-3 50 × 4.6 mm I.D., 3 µm; mobile phase: mobile phase A: CO₂, mobile phase B: IPA + ACN (0.05 vol% DEA); isocratic elution: 50 vol% IPA + ACN (0.05 vol% DEA) in CO₂; flow rate: 3 mL/min; detector: PDA, column temperature: 35°C; column pressure: 100 Bar).

Compound I-3B (*i.e.,* the compound represented by formula I-3B in the present disclosure) had a retention time RT = 1.674 min.

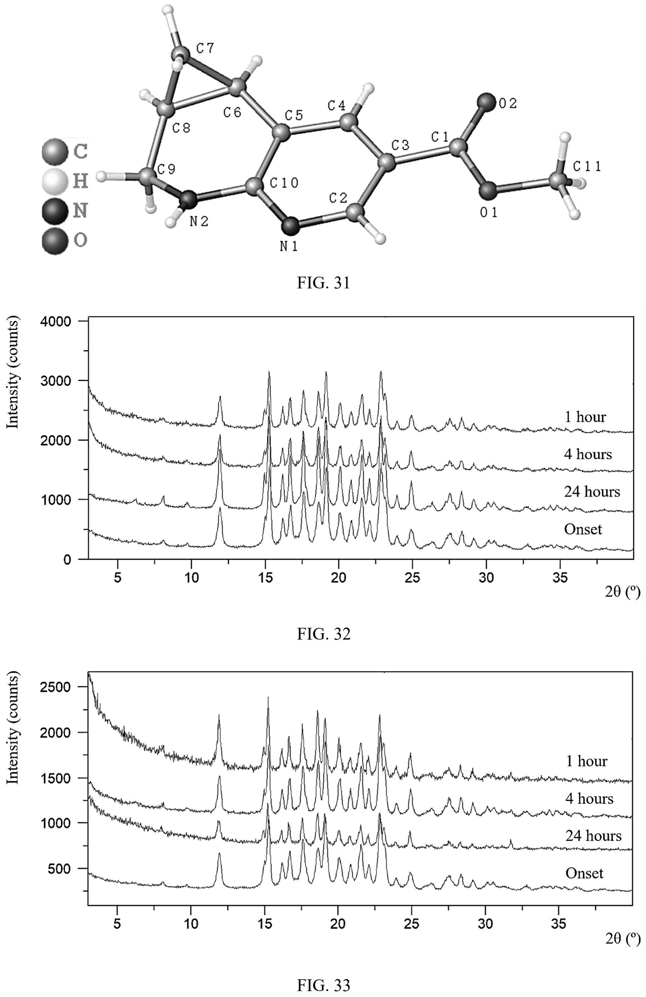

Single crystal preparation method: 5 mg of methyl (1a*S*,7b*R*)-1a,2,3,7b-tetrahydro-1*H*-cyclopropa[*c*][1,8]naphthyridine-6-carboxylate (R1) was weighed into a 2 mL LC-MS vial, dissolved by adding 0.4 mL of ethyl acetate, and mixed well with 0.6 mL of petroleum ether. The vial was allowed to stand at room temperature until the solvent was naturally evaporated to obtain a colorless transparent crystal (approximately 2 days). Single crystal structure analysis was performed. The single crystal structure of compound R1 is shown in FIG. 31, with its main crystal parameters as follows:

| | |
|---|---|
| Unit cell dimensions/mm³ | 0.38 × 0.21 × 0.13 mm³ |
| Wavelength | λ = 1.54178 |
| Crystal system | Triclinic |
| Space group | P1 |
| a/Å | 6.7536(6) |
| b/Å | 8.7867(9) |
| c/Å | 9.0173(10) |
| α/° | 71.444(2) |
| β/° | 80.225(3) |
| γ/° | 83.062(4) |
| Unit cell volume/Å³ | 498.64(9) |
| Z | 2 |
| Unit cell density mg/m³ | 1.360 |
| F(000) | 216 |
| Absorption coefficient mm⁻¹ | 0.782 |
| Diffraction index range (h/k/1) | -8≤h≤6,-10≤k≤10,-10≤1≤10 |
| Temperature/K | 298(2) |
| Theta range for data collection/° | 5.23 to 66.03 |
| Goodness-of-fit on F² | 1.068 |
| Final R indices (I ≥ 2σ(I)) | R₁ = 0.0447, wR₂ = 0.1217 |
| R indices (all data) | R₁ = 0.0483, wR₂ = 0.1292 |
| Largest diff. peak and hole e.Å⁻³ | 0.183/-0.225 |
| Reflections collected | 2767 |
| Independent reflections | 2011 [Rint = 0.0215] |
| Absolute structure parameter | -0.2(3) |

The free base was difficult to recrystallize and prone to gel formation. The free base of the compound represented by formula I-3B obtained in Example 1 was completely dissolved in a toluene/dimethyl ether solvent system, with no solid precipitation observed. Stirring for a prolonged time in a THF/MTBE system can lead to solid precipitation, but the required time is long, which is not conducive to scale-up production in factories. Gel solidification is prone to formation of large chunks of solids, with severe sample agglomeration, making it easy to jam the stirring paddle and difficult to stir.

### Example 2: Preparation of crystal form A of compound represented by formula I-3B

The compound represented by formula I-3B (RT = 1.670 min) obtained in Example 1 was subjected to slow cooling in IPAc, followed by evaporation at room temperature to obtain the crystal form A of the compound represented by formula I-3B. The characterization data were as follows:

The XRPD diffraction peak data for the crystal form A of the compound represented by formula I-3B are shown in the following table:

| **Position [°2Th.]** | **Peak height [cts]** | **Full width at half maximum (FWHM) [°2Th]** | **Interplanar spacing [Å]** | **Relative intensity [%]** |
|---|---|---|---|---|
| 6.1420 | 90.24 | 0.3070 | 14.39 | 3.71 |
| 8.0423 | 147.41 | 0.1535 | 10.99 | 6.06 |
| 11.9093 | 1191.15 | 0.1023 | 7.43 | 49.01 |
| 14.9167 | 899.53 | 0.1023 | 5.94 | 37.01 |
| 15.2411 | 2430.63 | 0.1023 | 5.81 | 100.00 |
| 16.1573 | 905.58 | 0.1279 | 5.49 | 37.26 |
| 16.6737 | 905.46 | 0.1279 | 5.32 | 37.25 |
| 17.5769 | 1464.49 | 0.1279 | 5.05 | 60.25 |
| 18.5881 | 2384.07 | 0.1279 | 4.77 | 98.08 |
| 19.1067 | 1748.64 | 0.1279 | 4.65 | 71.94 |
| 20.0614 | 934.23 | 0.1279 | 4.43 | 38.44 |
| 20.8133 | 577.70 | 0.1279 | 4.27 | 23.77 |
| 21.5509 | 1476.87 | 0.1023 | 4.12 | 60.76 |
| 22.0426 | 713.09 | 0.1279 | 4.03 | 29.34 |
| 22.8130 | 1948.51 | 0.1279 | 3.90 | 80.16 |
| 23.1182 | 1139.78 | 0.1279 | 3.85 | 46.89 |
| 23.9156 | 364.73 | 0.1023 | 3.72 | 15.01 |
| 24.8666 | 927.66 | 0.1535 | 3.58 | 38.17 |
| 26.3142 | 170.97 | 0.2047 | 3.39 | 7.03 |
| 27.5010 | 398.71 | 0.1023 | 3.24 | 16.40 |
| 28.2785 | 496.30 | 0.1279 | 3.16 | 20.42 |
| 29.1046 | 397.53 | 0.1535 | 3.07 | 16.35 |
| 30.0655 | 220.68 | 0.1023 | 2.97 | 9.08 |
| 30.4627 | 196.33 | 0.1535 | 2.93 | 8.08 |
| 31.2043 | 87.93 | 0.3070 | 2.87 | 3.62 |
| 32.7893 | 100.86 | 0.1535 | 2.73 | 4.15 |
| 34.2911 | 139.47 | 0.1535 | 2.62 | 5.74 |
| 34.7780 | 135.01 | 0.2047 | 2.58 | 5.55 |
| 35.3914 | 122.60 | 0.1535 | 2.54 | 5.04 |
| 36.0175 | 85.65 | 0.1535 | 2.49 | 3.52 |
| 37.7108 | 29.56 | 0.8187 | 2.39 | 1.22 |

The characterization data for the crystal form A of the compound represented by formula I-3B are shown in the following table:

| Parameter | Result |
|---|---|
| X-ray diffraction | see FIG. 1; |
| Differential scanning calorimetry | The DSC curve is shown in FIG. 2, comprising an endothermic peak with an onset temperature at 160.9°C, a peak temperature at 168.4°C, and an enthalpy of 51.75 J/g. |
| | Exothermic (upward). |
| Thermogravimetric analysis | The TGA curve is shown in FIG. 2, with a weight loss of 0.67% in the temperature range of 14.8°C to 120°C. |

The NMR data for the crystal form A of the compound represented by formula I-3B were as follows:

| # | ppm | Integral |
|---|---|---|
| 1 | 8.05 | (1H, d) |
| 2 | 7.83 | (1H, d) |
| 3 | 7.63 | (1H, d) |
| 4 | 6.70 | (1H, d) |
| 5 | 6.60 | (1H, dd) |
| 6 | 3.92 | (1H, d) |
| 7 | 3.68-3.61 | (5H, m) |
| 8 | 3.49 | **(3H,** s) |
| 9 | 2.22-2.17 | (1H, m) |
| 10 | 2.11-2.00 | (5H, m) |
| 11 | 1.11-1.05 | (2H, m) |

According to the NMR data, the molar ratio of the residual solvent IPAc to the compound represented by formula I-3B was approximately 0.04:1 (0.9 wt%). It is speculated that the crystal form A of the compound represented by formula I-3B is an anhydrous crystal form.

### Variable-temperature XRPD experiment:

To investigate the properties of the crystal form A of the compound represented by formula I-3B, the crystal form A of the compound represented by formula I-3B was heated from 100°C to 180°C under a nitrogen flow, and a variable-temperature XRPD (VT-XRPD) experiment was performed on the sample. The results are shown in FIG. 3. Comparing the samples at 30°C without nitrogen (30°C, no N₂) and with nitrogen (30°C, N₂), the crystal form remained unchanged after heating to 100°C and 150°C under N₂ atmosphere (100°C, N₂ and 150°C, N₂), and the crystal form transformed into an amorphous form after heating to 180°C (180°C, N₂) and cooling to room temperature (cooled to 30°C, N₂). It is speculated that the crystal form A of the compound represented by formula I-3B is an anhydrous crystal form.

### Example 3: Preparation of crystal form B of compound represented by formula I-3B

The compound represented by formula I-3B (RT = 1.670 min) obtained in Example 1 was suspended and stirred in MeOH/H₂O (1:5, v:v) at 50°C for 3 days to obtain the crystal form B of the compound represented by formula I-3B, which was then air-dried at room temperature overnight before characterization.

The XRPD diffraction peak data for the crystal form B of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 6.1517 | 105.82 | 0.1535 | 14.37 | **3.97** |
| 11.8771 | 192.76 | 0.1535 | 7.45 | 7.22 |
| 14.4103 | 805.02 | 0.1535 | 6.15 | 30.17 |
| 14.9210 | 650.57 | 0.1023 | 5.94 | 24.38 |
| 15.2246 | 330.85 | 0.1023 | 5.82 | 12.40 |
| 16.1472 | 265.67 | 0.0768 | 5.49 | 9.96 |
| 17.1304 | 486.15 | 0.1535 | 5.18 | 18.22 |
| 17.6764 | 532.87 | 0.1023 | 5.02 | 19.97 |
| 18.5415 | 2668.15 | 0.1023 | 4.79 | 100.00 |
| 19.1998 | 600.63 | 0.1279 | 4.62 | 22.51 |
| 20.8833 | 813.19 | 0.1535 | 4.25 | 30.48 |
| 21.6081 | 837.13 | 0.1791 | 4.11 | 31.37 |
| 22.1231 | 288.83 | 0.1279 | 4.02 | 10.83 |
| 22.9003 | 188.00 | 0.1023 | 3.88 | 7.05 |
| 23.2190 | 155.26 | 0.1023 | 3.83 | 5.82 |
| 24.7922 | 1054.94 | 0.1279 | 3.59 | 39.54 |
| 25.9202 | 59.25 | 0.2047 | 3.44 | 2.22 |
| 26.3847 | 206.31 | 0.2047 | 3.38 | 7.73 |
| 27.1228 | 58.79 | 0.1535 | 3.29 | 2.20 |
| 28.3312 | 223.21 | 0.1535 | 3.15 | 8.37 |
| 29.1001 | 306.82 | 0.1535 | 3.07 | 11.50 |
| 30.0651 | 313.20 | 0.1279 | 2.97 | 11.74 |
| 31.1891 | 50.06 | 0.3070 | 2.87 | 1.88 |
| 32.8352 | 90.17 | 0.2047 | 2.73 | 3.38 |
| 35.4419 | 58.26 | 0.3070 | 2.53 | 2.18 |
| 37.5535 | 60.61 | 0.1535 | 2.40 | 2.27 |
| 38.4198 | 35.26 | 0.3070 | 2.34 | 1.32 |

The characterization data for the crystal form B of the compound represented by formula I-3B are shown in the following table:

| Parameter | Result |
|---|---|
| X-ray diffraction | see FIG. 4; |
| Differential scanning calorimetry | The DSC curve is shown in FIG. 5, comprising an endothermic peak with an onset temperature at 164.2°C, a peak temperature at 171.7°C, and an enthalpy of 55.31 J/g. |
| | Exothermic (upward). |
| Thermogravimetric analysis | The TGA curve is shown in FIG. 5, with a weight loss of 1.61% in the temperature range of 26.6°C to 120°C. |

The NMR data for the crystal form B of the compound represented by formula I-3B were as follows:

| # | ppm | Integral |
|---|---|---|
| 1 | 8.06 | (1H, d) |
| 2 | 7.83 | (1H, d) |
| 3 | 7.65 | (1H, d) |
| 4 | 6.70 | (1H, d) |
| 5 | 6.61 | (1H, dd) |
| 6 | 3.93 | (1H, d) |
| 7 | 3.68-3.61 | (5H, m) |
| 8 | 3.50 | (3H, s) |
| 9 | 2.23-2.17 | (1H, m) |
| 10 | 2.12-2.01 | (5H, m) |
| 11 | 1.10-1.06 | (2H, m) |

No residual solvent was observed in the NMR data, indicating that the crystal form B is an anhydrous crystal form.

### Variable-temperature XRPD experiment:

To investigate the properties of the crystal form B of the compound represented by formula I-3B, the crystal form B of the compound represented by formula I-3B was heated from 100°C to 180°C under a nitrogen flow, and a variable-temperature XRPD (VT-XRPD) experiment was performed on the sample. The results are shown in FIG. 6. Comparing the samples at 30°C without nitrogen (30°C, no N₂) and with nitrogen (30°C, N₂), the crystal form remained unchanged after heating to 100°C and 150°C under N₂ atmosphere (100°C, N₂ and 150°C, N₂), and the crystal form transformed into an amorphous form after heating to 180°C (180°C, N₂) and cooling to room temperature (cooled to 30°C, N₂). It is speculated that the crystal form B of the compound represented by formula I-3B is an anhydrous crystal form.

### Example 4: Preparation of salt of compound represented by formula I-3B or solvate thereof

### 4.1 Crystal form of hydrochloride salt of compound represented by formula I-3B

The crystal form A of the compound represented by formula I-3B and an equimolar amount of hydrochloric acid (37% aqueous hydrochloric acid solution) were suspended and stirred in acetone/n-heptane (1:1, v:v) at room temperature overnight to form a gel. The gel was subjected to temperature cycling (50°C to 5°C, heating rate of 4.5°C/min, maintaining the temperature at 50°C for 2 hours, cooling rate of 0.1 °C/min, maintaining the temperature at 5°C for 2 hours, for a total of 6 cycles) for approximately 5 days to obtain the crystal form of the hydrochloride salt of the compound represented by formula I-3B, which was then vacuum dried at room temperature overnight before characterization.

The XRPD diffraction peak data for the crystal form of the hydrochloride salt of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 6.6300 | 596.64 | 0.1023 | 13.33 | 37.55 |
| 9.4658 | 1588.97 | 0.1023 | 9.34 | 100.00 |
| 11.7321 | 1015.21 | 0.1023 | 7.54 | 63.89 |
| 12.3605 | 257.39 | 0.1023 | 7.16 | 16.20 |
| 15.2996 | 128.04 | 0.1279 | 5.79 | 8.06 |
| 16.0098 | 481.50 | 0.1279 | 5.54 | 30.30 |
| 16.3075 | 301.38 | 0.1023 | 5.44 | 18.97 |
| 16.5517 | 312.56 | 0.0768 | 5.36 | 19.67 |
| 17.2876 | 1055.18 | 0.1023 | 5.13 | 66.41 |
| 17.7557 | 304.58 | 0.1023 | 5.00 | 19.17 |
| 18.8713 | 685.90 | 0.1023 | 4.70 | 43.17 |
| 19.3109 | 1052.87 | 0.1023 | 4.60 | 66.26 |
| 19.8470 | 434.72 | 0.1023 | 4.47 | 27.36 |
| 20.6874 | 768.76 | 0.1535 | 4.29 | 48.38 |
| 21.9642 | 375.14 | 0.0768 | 4.05 | 23.61 |
| 22.6628 | 517.95 | 0.0768 | 3.92 | 32.60 |
| 22.8204 | 651.67 | 0.1023 | 3.90 | 41.01 |
| 23.1091 | 382.09 | 0.1023 | 3.85 | 24.05 |
| 24.7906 | 1206.03 | 0.1279 | 3.59 | 75.90 |
| 26.0322 | 150.32 | 0.1023 | 3.42 | 9.46 |
| 26.3331 | 146.57 | 0.1023 | 3.38 | 9.22 |
| 27.1757 | 300.39 | 0.1023 | 3.28 | 18.90 |
| 27.7245 | 186.71 | 0.1279 | 3.22 | 11.75 |
| 28.0194 | 225.55 | 0.1535 | 3.18 | 14.19 |
| 28.5916 | 120.75 | 0.1791 | 3.12 | 7.60 |
| 29.2537 | 77.30 | 0.1535 | 3.05 | 4.86 |
| 29.7057 | 233.51 | 0.1023 | 3.01 | 14.70 |
| 29.9392 | 382.53 | 0.1023 | 2.98 | 24.07 |
| 31.3880 | 105.15 | 0.1791 | 2.85 | 6.62 |
| 34.3264 | 70.38 | 0.4093 | 2.61 | 4.43 |
| 35.7471 | 110.49 | 0.1279 | 2.51 | 6.95 |
| 36.5482 | 38.96 | 0.3070 | 2.46 | 2.45 |
| 38.5229 | 64.50 | 0.4093 | 2.34 | 4.06 |

The characterization data for the crystal form of the hydrochloride salt of the compound represented by formula I-3B are shown in the following table:

| Parameter | Result |
|---|---|
| X-ray diffraction | see FIG. 7; |
| Differential scanning calorimetry | The DSC curve is shown in FIG. 8, comprising endothermic peaks with peak temperatures at 122.1°C and 199.6°C. |
| | Exothermic (upward). |
| Thermogravimetric analysis | The TGA curve is shown in FIG. 8, with a weight loss of 13.39% in the temperature range of 17.7°C to 100°C and a weight loss of 4.54% in the temperature range of 100°C to 150°C. |

The NMR data for the crystal form of the hydrochloride salt of the compound represented by formula I-3B were as follows:

| # | ppm | Integral |
|---|---|---|
| 1 | 8.06 | (1H, d) |
| 2 | 7.84 | (1H, d) |
| 3 | 7.65 | (1H, d) |
| 4 | 6.72 | (1H, d) |
| 5 | 6.60 | (1H, dd) |
| 6 | 3.90 | (1H, d) |
| 7 | 3.69-3.61 | (5H, m) |
| 8 | 3.50 | (3H, s) |
| 9 | 2.22-2.18 | (1H, m) |
| 10 | 2.11-2.02 | (5H, m) |
| 11 | 1.11-1.07 | (2H, m) |

According to the NMR data, the molar ratio of the residual solvent acetone to the compound represented by formula I-3B was 0.9 (10.3 wt%). HPLC/IC results showed that the molar ratio of hydrochloric acid to the compound represented by formula I-3B was 0.9.

### 4.2 Crystal form of phosphate salt of compound represented by formula I-3B

The crystal form A of the compound represented by formula I-3B and an equimolar amount of phosphoric acid were suspended and stirred in acetone/n-heptane (1:1, v:v) at room temperature for 8 days to obtain the crystal form of the phosphate salt of the compound represented by formula I-3B, which was then vacuum dried at room temperature overnight before characterization.

The XRPD diffraction peak data for the crystal form of the phosphate salt of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 8.3077 | 610.81 | 0.1279 | 10.64 | 37.46 |
| 10.1638 | 161.89 | 0.1023 | 8.70 | 9.93 |
| 11.5462 | 547.43 | 0.1279 | 7.66 | 33.58 |
| 16.2546 | 705.04 | 0.1023 | 5.45 | 43.24 |
| 16.6545 | 1630.44 | 0.1279 | 5.32 | 100.00 |
| 19.2057 | 488.11 | 0.1279 | 4.62 | 29.94 |
| 20.0891 | 266.84 | 0.4093 | 4.42 | 16.37 |
| 21.0761 | 567.79 | 0.2558 | 4.22 | 34.82 |
| 22.2878 | 194.01 | 0.2558 | 3.99 | 11.90 |
| 23.1973 | 488.44 | 0.2558 | 3.83 | 29.96 |
| 24.4272 | 108.32 | 0.3070 | 3.64 | 6.64 |
| 25.2047 | 127.30 | 0.3070 | 3.53 | 7.81 |
| 26.4914 | 103.19 | 0.3070 | 3.36 | 6.33 |

The characterization data for the crystal form of the phosphate salt of the compound represented by formula I-3B are shown in the following table:

| Parameter | Result |
|---|---|
| X-ray diffraction | see FIG. 9; |
| Differential scanning calorimetry | The DSC curve is shown in FIG. 10, comprising an endothermic peak with a peak temperature at 158.5°C. Exothermic (upward). |
| Thermogravimetric analysis | The TGA curve is shown in FIG. 10, with a weight loss of 2.07% in the temperature range of 27.4°C to 120°C. |

The NMR data for the crystal form of the phosphate salt of the compound represented by formula I-3B were as follows:

| # | ppm | Integral |
|---|---|---|
| 1 | 8.06 | (1H, d) |
| 2 | 7.83 | (1H, d) |
| 3 | 7.64 | (1H, d) |
| 4 | 6.71 | (1H, d) |
| 5 | 6.60 | (1H, dd) |
| 6 | 3.92 | (1H, d) |
| 7 | 3.68-3.58 | (5H, m) |
| 8 | 3.50 | (3H, s) |
| 9 | 2.23-2.17 | (1H, m) |
| 10 | 2.12-1.97 | (5H, m) |
| 11 | 1.10-1.03 | (2H, m) |

According to the NMR data, the molar ratio of the residual solvent acetone to the compound represented by formula I-3B was 0.05 (0.5 wt%). HPLC/IC results showed that the molar ratio of phosphoric acid to the compound represented by formula I-3B was 1.0.

### 4.3 Crystal form of fumarate complex of compound represented by formula I-3B

Method 1: 300.6 mg of the crystal form A of the compound represented by formula I-3B and 80.0 mg of an equimolar amount of fumaric acid were weighed into a 5 mL vial, then suspended and stirred in 3 mL of acetone/n-heptane (1:1, v:v) at room temperature for 3 days, followed by centrifugation at room temperature to obtain the fumarate complex of the compound represented by formula I-3B.

The XRPD diffraction peak data for the crystal form of the fumarate complex of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 7.8737 | 244.76 | 0.0768 | 11.23 | 9.15 |
| 9.1031 | 428.71 | 0.0768 | 9.71 | 16.03 |
| 10.8610 | 1215.80 | 0.0768 | 8.15 | 45.45 |
| 14.7221 | 365.21 | 0.1023 | 6.02 | 13.65 |
| 15.8706 | 2401.00 | 0.1023 | 5.58 | 89.76 |
| 16.4943 | 340.49 | 0.1023 | 5.37 | 12.73 |
| 16.8448 | 504.55 | 0.1023 | 5.26 | 18.86 |
| 17.3568 | 956.36 | 0.0768 | 5.11 | 35.75 |
| 17.5394 | 807.46 | 0.0768 | 5.06 | 30.19 |
| 18.4000 | 99.93 | 0.8187 | 4.82 | 3.74 |
| 18.8933 | 2145.87 | 0.1023 | 4.70 | 80.22 |
| 20.5163 | 2674.98 | 0.1023 | 4.33 | 100.00 |
| 21.0547 | 674.16 | 0.0768 | 4.22 | 25.20 |
| 21.7893 | 154.34 | 0.1023 | 4.08 | 5.77 |
| 22.3331 | 1411.37 | 0.1023 | 3.98 | 52.76 |
| 22.5206 | 1739.95 | 0.0768 | 3.95 | 65.05 |
| 22.9013 | 219.91 | 0.1535 | 3.88 | 8.22 |
| 23.8410 | 306.58 | 0.1023 | 3.73 | 11.46 |
| 24.3254 | 634.13 | 0.1279 | 3.66 | 23.71 |
| 24.7494 | 165.87 | 0.1023 | 3.60 | 6.20 |
| 25.0729 | 288.68 | 0.1023 | 3.55 | 10.79 |
| 25.3664 | 161.54 | 0.1023 | 3.51 | 6.04 |
| 26.4422 | 1360.63 | 0.0768 | 3.37 | 50.87 |
| 26.6316 | 2212.64 | 0.1023 | 3.35 | 82.72 |
| 27.6087 | 294.31 | 0.1279 | 3.23 | 11.00 |
| 28.1521 | 85.55 | 0.1535 | 3.17 | 3.20 |
| 28.8093 | 549.02 | 0.1023 | 3.10 | 20.52 |
| 29.4739 | 353.72 | 0.1279 | 3.03 | 13.22 |
| 30.2870 | 235.18 | 0.1535 | 2.95 | 8.79 |
| 30.6610 | 33.74 | 0.4093 | 2.92 | 1.26 |
| 30.9895 | 197.60 | 0.1279 | 2.89 | 7.39 |
| 31.5798 | 72.32 | 0.1535 | 2.83 | 2.70 |
| 31.9729 | 135.34 | 0.1279 | 2.80 | 5.06 |
| 32.9655 | 83.85 | 0.2047 | 2.72 | 3.13 |
| 33.3910 | 105.21 | 0.1535 | 2.68 | 3.93 |
| 34.0246 | 109.55 | 0.1023 | 2.63 | 4.10 |
| 35.8898 | 101.27 | 0.1535 | 2.50 | 3.79 |
| 38.3771 | 107.88 | 0.2047 | 2.35 | 4.03 |

The characterization data for the crystal form of the fumarate complex of the compound represented by formula I-3B are shown in the following table:

| Parameter | Result |
|---|---|
| X-ray diffraction | see FIG. 11; |
| Differential scanning calorimetry | The DSC curve is shown in FIG. 12, comprising an endothermic peak with an onset temperature at 165.2°C, a peak temperature at 167.2°C, and an enthalpy of 75.81 J/g. |
| | Exothermic (upward). |
| Thermogravimetric analysis | The TGA curve is shown in FIG. 12, with a weight loss of 0.36% in the temperature range of 26.2°C to 120°C. |

The NMR data for the crystal form of the fumarate complex of the compound represented by formula I-3B were as follows:

| # | ppm | Integral |
|---|---|---|
| 1 | 8.06 | (1H, d) |
| 2 | 7.83 | (1H, d) |
| 3 | 7.64 | (1H, d) |
| 4 | 6.71 | (1H, d) |
| 5 | 6.63 | (2H, s) |
| 6 | 6.60 | (1H, dd) |
| 7 | 3.92 | (1H, d) |
| 8 | 3.61-3.68 | (5H, m) |
| 9 | 3.51 | (3H, s) |
| 10 | 2.23-2.17 | (1H, m) |
| 11 | 2.12-2.00 | (5H, m) |
| 12 | 1.10-1.06 | (2H, m) |

To further investigate the molecular structure of the aforementioned crystal, single crystals of the fumarate complex of the compound represented by formula I-3B were obtained by Method 2. Method 2: The compound represented by formula I-3B (approximately 15 mg) was weighed into an EP tube, followed by the addition of acetone (0.75 mL), and shaken until the sample was dissolved. The mixture was filtered through a microporous membrane into a 2 mL transparent glass vial, followed by the addition of n-heptane (0.75 mL) for phase separation. The vial was sealed with sealing film with two small holes pierced, followed by evaporation at room temperature for approximately 2 days to precipitate a transparent crystal, yielding the fumarate complex of the compound represented by formula I-3B (single crystal).

The single crystal of the fumarate complex of the compound represented by formula I-3B obtained by Method 2 was subjected to X-ray single crystal diffraction, and the crystallographic parameters were measured as follows:

| | |
|---|---|
| Empirical formula | C₂₆H₂₆F₂N₆O₆ |
| Molecular weight | 556.63 |
| Unit cell dimensions/mm³ | 0.15 × 0.12 × 0.12 mm³ |
| Wavelength | 1.34139 Å |
| Crystal system | Orthorhombic |
| Space group | P2₁2₁2₁ |
| a/Å | 6.4400(4) Å |
| b/Å | 11.9376(8) Å |
| c/Å | 33.139(2) Å |
| α/° | 90° |
| β/° | 90° |
| γ/° | 90° |
| Unit cell volume/Å³ | 2547.7(3) Å³ |
| Z | 4 |
| Unit cell density mg/m³ | 1.451 mg/m³ |
| F(000) | 1160 |
| Absorption coefficient mm⁻¹ | 0.621 mm⁻¹ |
| Diffraction index range (h/k/l) | -8 ≤ h ≤ 8, -16 ≤ k ≤ 13, -43 ≤ l ≤ 41 |
| Temperature/K | 200(2) K |
| Theta range for data collection/° | 2.32 to 72.278° |
| Data/restraints/parameters | 6335/0/363 |
| Absorption correction | Semi-empirical from equivalents |
| Max. and min. transmission | 0.726 and 0.562 |
| Refinement method | Full-matrix least-squares on F² |
| Goodness-of-fit on F² | 1.055 |
| Final R indices (I ≥ 2σ(I)) | R₁ = 0.0401, wR₂ = 0.0907 |
| R indices (all data) | R₁ = 0.0431, wR₂ = 0.0920 |
| Absolute structure parameter | 0.07(4) |
| Extinction coefficient | n/a |
| Largest diff. peak and hole e.Å⁻³ | 0.263 and -0.244 e.Å⁻³ |
| Reflections collected | 24503 |
| Independent reflections | 6335 [R(int) = 0.0659] |
| Completeness to theta = 53.594° | 97.5% |

According to the X-ray single crystal diffraction results, fumaric acid was connected to the compound represented by I-3B via a hydrogen bond, with a distance of 0.84 Å between the hydrogen atom and the oxygen atom, and a distance of 1.89 Å between the hydrogen atom and the nitrogen atom. The hydrogen atom was closer to the oxygen atom, indicating that the single crystal sample formed a co-crystal *(i.e.,* a co-crystal of (1a*S*,7b*R*)-7-(6-(4,4-difluoropiperidine-1-carbonyl)-1,1a,2,7b-tetrahydro-3*H*-cyclopropa[*c*][1,8]naphthyridin-3-yl)-2-methyl-[1,2,4]triazolo[4,3-*a*]pyridin-3(2*H*)-one with fumaric acid). The molar ratio of fumaric acid to the compound represented by formula I-3B was 1.0, and no residual solvent signal was observed in the NMR data.

The hydrogen bonding in crystals of the fumarate complex of the compound represented by formula I-3B is shown in the following table:

| D-H...A | d(D-H) | d(H...A) | d(D...A) | <(DHA) |
|---|---|---|---|---|
| C(19)-H(19)...N(5) | 0.95 | 2.56 | 3.155(2) | 121.1 |
| O(4)-H(4)...O(1) | 0.84 | 1.79 | 2.6175(16) | 169.1 |
| O(5)-H(5)...N(2) | 0.84 | 1.89 | 2.6755(16) | 155.1 |

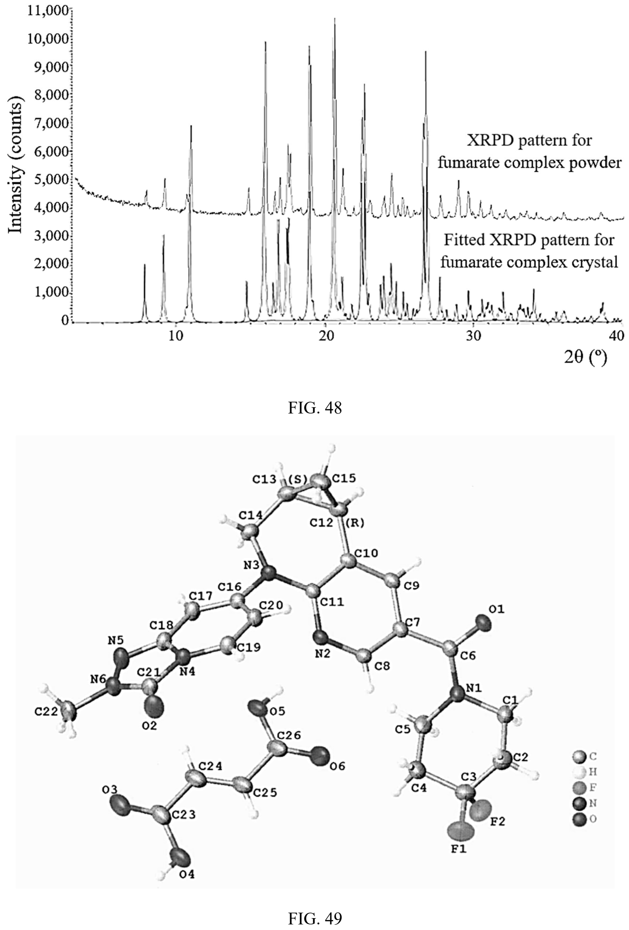

The molecular structure ellipsoid model of the single crystal of the fumarate complex of the compound represented by formula I-3B is shown in FIG. 49. The comparison between the fitted XRPD diffraction pattern and the measured XRPD diffraction pattern of the crystal obtained by Method 1 is shown in FIG. 48. The results indicate that the complex crystals obtained by Method 1 and Method 2 are of the same crystal form.

### 4.4 Crystal form of L-tartrate salt of compound represented by formula I-3B

Method 1: The crystal form A of the compound represented by formula I-3B and an equimolar amount of tartaric acid were suspended and stirred in acetone/n-heptane (1:1, v:v) at room temperature for 8 days to obtain the crystal form of the L-tartrate salt of the compound represented by formula I-3B, which was then vacuum dried at room temperature overnight before characterization.

Method 2: 299.7 mg of the crystal form A of the compound represented by formula I-3B and 102.5 mg of an equimolar amount of L-tartaric acid were weighed into a 5 mL vial, then suspended and stirred in 3 mL of acetone/n-heptane (1:1, v:v) at room temperature for 3 days, followed by centrifugation at room temperature to obtain the crystal form of the L-tartrate salt of the compound represented by formula I-3B.

The XRPD diffraction peak data for the crystal form of the L-tartrate salt of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 4.8049 | 346.02 | 0.0768 | 18.39 | 16.15 |
| 8.2103 | 2141.93 | 0.1023 | 10.77 | 100.00 |
| 9.5787 | 890.80 | 0.1023 | 9.23 | 41.59 |
| 12.3727 | 200.74 | 0.1023 | 7.15 | 9.37 |
| 14.8090 | 89.41 | 0.1535 | 5.98 | 4.17 |
| 15.7367 | 1341.79 | 0.1279 | 5.63 | 62.64 |
| 16.4412 | 216.00 | 0.1023 | 5.39 | 10.08 |
| 16.9943 | 312.22 | 0.1279 | 5.22 | 14.58 |
| 17.6141 | 800.62 | 0.1279 | 5.04 | 37.38 |
| 18.4213 | 967.22 | 0.1279 | 4.82 | 45.16 |
| 18.7259 | 224.00 | 0.1023 | 4.74 | 10.46 |
| 19.1806 | 222.24 | 0.1023 | 4.63 | 10.38 |
| 20.1000 | 2006.06 | 0.1535 | 4.42 | 93.66 |
| 20.7424 | 2045.80 | 0.1535 | 4.28 | 95.51 |
| 21.3245 | 798.43 | 0.2047 | 4.17 | 37.28 |
| 21.6058 | 666.70 | 0.1279 | 4.11 | 31.13 |
| 22.2508 | 118.55 | 0.1791 | 4.00 | 5.53 |
| 22.9897 | 710.89 | 0.1279 | 3.87 | 33.19 |
| 23.3042 | 1158.76 | 0.1279 | 3.82 | 54.10 |
| 24.0390 | 341.70 | 0.1279 | 3.70 | 15.95 |
| 25.0957 | 349.78 | 0.1279 | 3.55 | 16.33 |
| 25.6029 | 239.40 | 0.1279 | 3.48 | 11.18 |
| 26.5670 | 120.83 | 0.2047 | 3.36 | 5.64 |
| 27.0583 | 262.16 | 0.1791 | 3.30 | 12.24 |
| 27.9598 | 339.20 | 0.1535 | 3.19 | 15.84 |
| 28.6123 | 158.32 | 0.1535 | 3.12 | 7.39 |
| 29.0290 | 351.79 | 0.1279 | 3.08 | 16.42 |
| 31.1103 | 131.01 | 0.1535 | 2.87 | 6.12 |
| 31.7786 | 79.78 | 0.2047 | 2.82 | 3.72 |
| 32.4638 | 192.87 | 0.2047 | 2.76 | 9.00 |
| 33.2278 | 144.24 | 0.1791 | 2.70 | 6.73 |
| 33.9027 | 103.09 | 0.2558 | 2.64 | 4.81 |
| 35.3088 | 79.05 | 0.4093 | 2.54 | 3.69 |

The characterization data for the crystal form of the L-tartrate salt of the compound represented by formula I-3B are shown in the following table:

| Type | Result |
|---|---|
| X-ray diffraction | see FIG. 13; |
| Differential scanning calorimetry | The DSC curve is shown in FIG. 14, comprising an endothermic peak with an onset temperature at 162.4°C, a peak temperature at 165.7°C, and an enthalpy of 86.36 J/g. |
| | Exothermic (upward). |
| Thermogravimetric analysis | The TGA curve is shown in FIG. 14, with a weight loss of 0.57% in the temperature range of 25.9°C to 120°C. |

.

The NMR data for the crystal form of the L-tartrate salt of the compound represented by formula I-3B were as follows:

| # | ppm | Integral |
|---|---|---|
| 1 | 8.06 | (1H, d) |
| 2 | 7.83 | (1H, d) |
| 3 | 7.64 | (1H, d) |
| 4 | 6.71 | (1H, d) |
| 5 | 6.60 | (1H, dd) |
| 6 | 4.31 | (2H, s) |
| 7 | 3.92 | (1H, d) |
| 8 | 3.68-3.61 | (5H, m) |
| 9 | 3.50 | (3H, s) |
| 10 | 2.23-2.17 | (1H, m) |
| 11 | 2.12-2.00 | (5H, m) |
| 12 | 1.10-1.06 | (2H, m) |

The molar ratio of tartaric acid to the compound represented by formula I-3B was 1.0, and no residual solvent signal was observed in the NMR data.

### 4.5 Crystal form of p-toluenesulfonate salt of compound represented by formula I-3B

Method 1: The crystal form A of the compound represented by formula I-3B and an equimolar amount of p-toluenesulfonic acid were suspended and stirred in acetone/n-heptane (1:1, v:v) at room temperature for 8 days to obtain the crystal form of the p-toluenesulfonate salt of the compound represented by formula I-3B, which was then vacuum dried at room temperature overnight before characterization.

Method 2: 300.9 mg of the crystal form A of the compound represented by formula I-3B and 130.8 mg of an equimolar amount of p-toluenesulfonic acid monohydrate were weighed into a 5 mL vial, then suspended and stirred in 3 mL of acetone/n-heptane (1:1, v:v) at room temperature for 3 days, followed by centrifugation at room temperature to obtain the crystal form of the p-toluenesulfonate salt of the compound represented by formula I-3B.

The XRPD diffraction peak data for the crystal form of the p-toluenesulfonate salt of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 6.5908 | 10367.33 | 0.1023 | 13.41 | 100.00 |
| 8.0578 | 513.37 | 0.1023 | 10.97 | 4.95 |
| 9.8041 | 864.88 | 0.1023 | 9.02 | 8.34 |
| 11.3953 | 647.25 | 0.1023 | 7.77 | 6.24 |
| 12.6889 | 207.81 | 0.0768 | 6.98 | 2.00 |
| 13.1862 | 646.57 | 0.0768 | 6.71 | 6.24 |
| 13.8108 | 491.80 | 0.1023 | 6.41 | 4.74 |
| 13.9933 | 344.05 | 0.0768 | 6.33 | 3.32 |
| 15.2991 | 247.36 | 0.1023 | 5.79 | 2.39 |
| 16.2016 | 293.86 | 0.0768 | 5.47 | 2.83 |
| 16.4239 | 290.69 | 0.1023 | 5.40 | 2.80 |
| 17.3162 | 311.91 | 0.1023 | 5.12 | 3.01 |
| 17.9450 | 187.88 | 0.1023 | 4.94 | 1.81 |
| 18.5212 | 1631.25 | 0.0768 | 4.79 | 15.73 |
| 19.3358 | 416.86 | 0.1023 | 4.59 | 4.02 |
| 19.6691 | 1315.34 | 0.0768 | 4.51 | 12.69 |
| 19.8455 | 1736.65 | 0.0768 | 4.47 | 16.75 |
| 20.3870 | 1239.82 | 0.1023 | 4.36 | 11.96 |
| 20.9737 | 472.84 | 0.1279 | 4.24 | 4.56 |
| 21.6058 | 235.13 | 0.1023 | 4.11 | 2.27 |
| 21.8008 | 286.85 | 0.0768 | 4.08 | 2.77 |
| 22.2850 | 558.50 | 0.1023 | 3.99 | 5.39 |
| 23.1284 | 156.98 | 0.1023 | 3.85 | 1.51 |
| 23.8340 | 56.89 | 0.1535 | 3.73 | 0.55 |
| 24.3142 | 298.01 | 0.1535 | 3.66 | 2.87 |
| 24.8854 | 146.54 | 0.1535 | 3.58 | 1.41 |
| 25.8394 | 240.61 | 0.1023 | 3.45 | 2.32 |
| 26.1503 | 207.55 | 0.1279 | 3.41 | 2.00 |
| 26.3794 | 209.99 | 0.0768 | 3.38 | 2.03 |
| 26.7409 | 146.19 | 0.1279 | 3.33 | 1.41 |
| 27.3263 | 173.67 | 0.1279 | 3.26 | 1.68 |
| 28.1047 | 247.89 | 0.1791 | 3.18 | 2.39 |
| 29.3253 | 89.40 | 0.2047 | 3.05 | 0.86 |
| 30.3321 | 114.69 | 0.3070 | 2.95 | 1.11 |
| 30.8342 | 181.00 | 0.1023 | 2.90 | 1.75 |
| 31.8898 | 138.33 | 0.1023 | 2.81 | 1.33 |
| 32.2705 | 124.88 | 0.1535 | 2.77 | 1.20 |
| 33.2074 | 75.22 | 0.4093 | 2.70 | 0.73 |
| 33.6478 | 82.70 | 0.1535 | 2.66 | 0.80 |
| 37.2358 | 65.82 | 0.1535 | 2.41 | 0.63 |
| 38.0411 | 80.23 | 0.1535 | 2.37 | 0.77 |
| 38.3313 | 42.30 | 0.4093 | 2.35 | 0.41 |

The characterization data for the crystal form of the p-toluenesulfonate salt of the compound represented by formula I-3B are shown in the following table:

| Type | Result |
|---|---|
| X-ray diffraction | see FIG. 15; |
| Differential scanning calorimetry | The DSC curve is shown in FIG. 16, comprising endothermic peaks with peak temperatures at 278.9°C and 98.5°C. |
| | Exothermic (upward). |
| Thermogravimetric analysis | The TGA curve is shown in FIG. 16, with a weight loss of 0.89% in the temperature range of 25.9°C to 150°C. |

The NMR data for the crystal form of the p-toluenesulfonate salt of the compound represented by formula I-3B were as follows:

| # | ppm | Integral |
|---|---|---|
| 1 | 8.05 | (1H, d) |
| 2 | 7.84 | (1H, d) |
| 3 | 7.65 | (1H, d) |
| 4 | 7.48 | (2H, dt) |
| 5 | 7.12 | (2H, d) |
| 6 | 6.72 | (1H, d) |
| 7 | 6.60 | (1H, dd) |
| 8 | 3.92 | (1H, d) |
| 9 | 3.69-3.61 | (5H, m) |
| 10 | 3.50 | (3H, s) |
| 11 | 2.31 | (3H, s) |
| 12 | 2.23-2.18 | (1H, m) |
| 13 | 2.12-2.00 | (5H, m) |
| 14 | 1.11-1.06 | (2H, m) |

The molar ratio of *p*-toluenesulfonic acid to the compound represented by formula I-3B was 1.0, and no residual solvent signal was observed in the NMR data.

### 4.6 Crystal form of L-malate salt of compound represented by formula I-3B

The crystal form A of the compound represented by formula I-3B and an equimolar amount of malic acid were suspended and stirred in acetone/n-heptane (1:1, v:v) at room temperature overnight to form a gel. The gel was subjected to temperature cycling (50°C to 5°C, heating rate of 4.5°C/min, maintaining the temperature at 50°C for 2 hours, cooling rate of 0.1°C/min, maintaining the temperature at 5°C for 2 hours, for a total of 6 cycles) for approximately 5 days to obtain the crystal form of the L-malate salt of the compound represented by formula I-3B, which was then vacuum dried at room temperature overnight before characterization.

The XRPD diffraction peak data for the crystal form of the L-malate salt of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 8.1199 | 447.78 | 0.0768 | 10.89 | 15.08 |
| 10.1492 | 197.52 | 0.0768 | 8.72 | 6.65 |
| 10.8339 | 682.79 | 0.0768 | 8.17 | 23.00 |
| 14.7440 | 341.80 | 0.1023 | 6.01 | 11.51 |
| 15.8121 | 2968.78 | 0.1023 | 5.60 | 100.00 |
| 16.0616 | 1092.87 | 0.1023 | 5.52 | 36.81 |
| 16.6556 | 589.67 | 0.1279 | 5.32 | 19.86 |
| 17.1975 | 1135.16 | 0.1279 | 5.16 | 38.24 |
| 17.5998 | 951.91 | 0.1023 | 5.04 | 32.06 |
| 18.0327 | 137.69 | 0.2047 | 4.92 | 4.64 |
| 18.8478 | 1849.44 | 0.1023 | 4.71 | 62.30 |
| 19.3847 | 121.61 | 0.1023 | 4.58 | 4.10 |
| 19.9904 | 278.78 | 0.1023 | 4.44 | 9.39 |
| 20.3420 | 2016.39 | 0.1023 | 4.37 | 67.92 |
| 20.6221 | 1323.94 | 0.1023 | 4.31 | 44.60 |
| 20.9302 | 205.86 | 0.1023 | 4.24 | 6.93 |
| 21.7656 | 1504.11 | 0.1023 | 4.08 | 50.66 |
| 22.0359 | 1423.32 | 0.1279 | 4.03 | 47.94 |
| 23.6140 | 172.31 | 0.1023 | 3.77 | 5.80 |
| 23.8808 | 376.84 | 0.1023 | 3.73 | 12.69 |
| 24.1935 | 323.60 | 0.1023 | 3.68 | 10.90 |
| 24.4039 | 382.25 | 0.1023 | 3.65 | 12.88 |
| 24.6737 | 222.86 | 0.1023 | 3.61 | 7.51 |
| 25.4739 | 1154.75 | 0.1279 | 3.50 | 38.90 |
| 25.8627 | 712.87 | 0.1279 | 3.45 | 24.01 |
| 26.6521 | 94.62 | 0.1023 | 3.34 | 3.19 |
| 27.4457 | 404.52 | 0.1023 | 3.25 | 13.63 |
| 27.8574 | 237.91 | 0.1535 | 3.20 | 8.01 |
| 28.3386 | 391.95 | 0.1023 | 3.15 | 13.20 |
| 29.1111 | 605.91 | 0.1791 | 3.07 | 20.41 |
| 29.9537 | 153.98 | 0.2047 | 2.98 | 5.19 |
| 30.7066 | 485.62 | 0.1279 | 2.91 | 16.36 |
| 31.1229 | 95.84 | 0.2047 | 2.87 | 3.23 |
| 31.7678 | 130.58 | 0.3070 | 2.82 | 4.40 |
| 32.3952 | 169.29 | 0.1023 | 2.76 | 5.70 |
| 32.9045 | 173.90 | 0.1279 | 2.72 | 5.86 |
| 33.6382 | 94.21 | 0.1279 | 2.66 | 3.17 |
| 34.1439 | 195.12 | 0.1535 | 2.63 | 6.57 |
| 35.6074 | 107.51 | 0.2558 | 2.52 | 3.62 |
| 36.9261 | 73.16 | 0.8187 | 2.43 | 2.46 |

The characterization data for the crystal form of the L-malate salt of the compound represented by formula I-3B are shown in the following table:

| Type | Result |
|---|---|
| X-ray diffraction | see FIG. 17; |
| Differential scanning calorimetry | The DSC curve is shown in FIG. 18, comprising an endothermic peak with an onset temperature at 120.4°C and peak temperatures at 128.1°C (enthalpy: 49.57 J/g) and 213.6°C. |
| | Exothermic (upward). |
| Thermogravimetric analysis | The TGA curve is shown in FIG. 18, with a weight loss of 1.31% in the temperature range of 27.5°C to 100°C. |

.

The NMR data for the crystal form of the L-malate salt of the compound represented by formula I-3B were as follows:

| # | ppm | Integral |
|---|---|---|
| 1 | 8.06 | (1H, d) |
| 2 | 7.83 | (1H, d) |
| 3 | 7.64 | (1H, d) |
| 4 | 6.71 | (1H, d) |
| 5 | 6.60 | (1H, dd) |
| 6 | 4.27 | (1H, q) |
| 7 | 3.92 | (1H, d) |
| 8 | 3.68-3.61 | (5H, m) |
| 9 | 3.50 | (3H, s) |
| 10 | 2.23-2.17 | (1H, m) |
| 11 | 2.12-2.02 | (5H, m) |
| 12 | 1.10-1.06 | (2H, m) |

The malic acid signal was observed in the NMR data, and the molar ratio of malic acid to the compound represented by formula I-3B was 1.0, while no residual solvent signal was observed in the NMR data.

### 4.7 Crystal form A of ethanedisulfonate salt of compound represented by formula I-3B

The crystal form A of the compound represented by formula I-3B and an equimolar amount of ethanedisulfonic acid were suspended and stirred in IPA at room temperature for 8 days to obtain the crystal form of the L-malate salt of the compound represented by formula I-3B, which was then vacuum dried at room temperature overnight before characterization.

The XRPD data for the crystal form A of the ethanedisulfonate salt of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 5.3000 | 789.34 | 0.1535 | 16.67 | 100.00 |
| 11.4960 | 156.31 | 0.3070 | 7.70 | 19.80 |
| 17.4290 | 259.67 | 0.3582 | 5.09 | 32.90 |
| 18.1885 | 298.34 | 0.3070 | 4.88 | 37.80 |
| 18.8525 | 400.81 | 0.2558 | 4.71 | 50.78 |
| 20.1426 | 387.41 | 0.2047 | 4.41 | 49.08 |
| 20.9788 | 359.04 | 0.1535 | 4.23 | 45.49 |
| 21.6000 | 268.38 | 0.2558 | 4.11 | 34.00 |
| 22.2815 | 186.80 | 0.3070 | 3.99 | 23.67 |

The characterization data for the crystal form A of the ethanedisulfonate salt of the compound represented by formula I-3B are shown in the following table:

| Type | Result |
|---|---|
| X-ray diffraction | see FIG. 19; |
| Differential scanning calorimetry | The DSC curve is shown in FIG. 20, comprising endothermic peaks with peak temperatures at 92.0°C, 118.9°C, 162.8°C, and 239.7°C, and an exothermic peak with a peak temperature at 198.8 ± 3°C. |
| | Exothermic (upward). |
| Thermogravimetric analysis | The TGA curve is shown in FIG. 20, with a weight loss of 9.08% in the temperature range of 25.2°C to 170°C. |

The NMR data for the crystal form A of the ethanedisulfonate salt of the compound represented by formula I-3B were as follows:

| # | ppm | Integral |
|---|---|---|
| 1 | 8.05 | (1H, d) |
| 2 | 7.85 | (1H, d) |
| 3 | 7.65 | (1H, d) |
| 4 | 6.73 | (1H, d) |
| 5 | 6.60 | (1H, dd) |
| 6 | 3.92 | (1H, d) |
| 7 | 3.69-3.61 | (5H, m) |
| 8 | 3.50 | (3H, s) |
| 9 | 2.67 | (2H, s) |
| 10 | 2.23-2.18 | (1H, m) |
| 11 | 2.12-2.00 | (5H, m) |
| 13 | 1.13-1.07 | (2H, m) |
| 14 | 1.04 | (3H, d) Peak of solvent IPA |

According to the NMR data, the molar ratio of ethanedisulfonic acid to the compound represented by formula I-3B was 0.5, and the molar ratio of the solvent IPA to the compound represented by formula I-3B was 0.5 (5.5 wt%).

### 4.8 Crystal form B of ethanedisulfonate salt of compound represented by formula I-3B

The crystal form A of the compound represented by formula I-3B and an equimolar amount of ethanedisulfonic acid were suspended and stirred in IPAc at room temperature overnight to form a gel. The gel was subjected to temperature cycling (50°C to 5°C, heating rate of 4.5°C/min, maintaining the temperature at 50°C for 2 hours, cooling rate of 0.1°C/min, maintaining the temperature at 5°C for 2 hours, for a total of 6 cycles) for approximately 5 days to obtain the crystal form B of the ethanedisulfonate salt of the compound represented by formula I-3B, which was then vacuum dried at room temperature overnight before characterization.

The XRPD diffraction peak data for the crystal form B of the ethanedisulfonate salt of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 6.2890 | 417.57 | 0.1023 | 14.05 | 28.10 |
| 10.9547 | 290.91 | 0.1535 | 8.08 | 19.58 |
| 12.5900 | 187.01 | 0.1535 | 7.03 | 12.58 |
| 13.3889 | 165.20 | 0.1279 | 6.61 | 11.12 |
| 15.0766 | 442.38 | 0.1279 | 5.88 | 29.77 |
| 17.0251 | 229.73 | 0.1535 | 5.21 | 15.46 |
| 17.6827 | 539.03 | 0.1279 | 5.02 | 36.27 |
| 18.5564 | 1486.03 | 0.1023 | 4.78 | 100.00 |
| 19.4099 | 1103.05 | 0.1535 | 4.57 | 74.23 |
| 20.3746 | 325.20 | 0.1535 | 4.36 | 21.88 |
| 20.8070 | 399.16 | 0.1535 | 4.27 | 26.86 |
| 21.6387 | 836.10 | 0.1535 | 4.11 | 56.26 |
| 22.1118 | 516.84 | 0.1535 | 4.02 | 34.78 |
| 23.2957 | 245.51 | 0.1791 | 3.82 | 16.52 |
| 23.7324 | 146.58 | 0.1535 | 3.75 | 9.86 |
| 25.0126 | 98.98 | 0.1535 | 3.56 | 6.66 |
| 27.3515 | 129.54 | 0.2047 | 3.26 | 8.72 |
| 27.9516 | 178.02 | 0.2047 | 3.19 | 11.98 |
| 29.2118 | 47.24 | 0.6140 | 3.06 | 3.18 |
| 38.7310 | 50.88 | 0.3070 | 2.32 | 3.42 |

The characterization data for the crystal form B of the ethanedisulfonate salt of the compound represented by formula I-3B are shown in the following table:

| Type | Result |
|---|---|
| X-ray diffraction | see FIG. 21; |
| Differential scanning calorimetry | The DSC curve is shown in FIG. 22, comprising an endothermic peak with an onset temperature at 235.8°C and peak temperatures at 132.6°C and 242.4°C |
| | (enthalpy: 47.18 J/g). |
| | Exothermic (upward). |
| Thermogravimetric analysis | The TGA curve is shown in FIG. 22, with a weight loss of 1.84% in the temperature range of 28.4°C to 150°C. |

The NMR data for the crystal form B of the ethanedisulfonate salt of the compound represented by formula I-3B were as follows:

| # | ppm | Integral |
|---|---|---|
| 1 | 8.06 | (1H, d) |
| 2 | 7.83 | (1H, d) |
| 3 | 7.65 | (1H, d) |
| 4 | 6.71 | (1H, d) |
| 5 | 6.60 | (1H, dd) |
| 6 | 3.92 | (1H, d) |
| 7 | 3.69-3.61 | (5H, m) |
| 8 | 3.50 | (3H, d) |
| 9 | 2.65 | (2H, s) |
| 10 | 2.23-2.18 | (1H, m) |
| 11 | 2.10-2.00 | (5H, m) |
| 12 | 1.11-1.07 | (2H, m) |

The molar ratio of ethanedisulfonic acid to the compound represented by formula I-3B was 0.5, and no residual solvent was observed in the NMR data.

### 4.9 Crystal form C of ethanedisulfonate salt of compound represented by formula I-3B

The crystal form A of the compound represented by formula I-3B and an equimolar amount of ethanedisulfonic acid were suspended and stirred in acetone/n-heptane (1:1, v:v) at room temperature overnight to form a gel. The gel was subjected to temperature cycling (50°C to 5°C, heating rate of 4.5°C/min, maintaining the temperature at 50°C for 2 hours, cooling rate of 0.1°C/min, maintaining the temperature at 5°C for 2 hours, for a total of 6 cycles) for approximately 5 days and remained as a gel. After evaporation at room temperature, the gel was vacuum dried to obtain the crystal form C of the ethanedisulfonate salt of the compound represented by formula I-3B before characterization at room temperature.

The XRPD diffraction peak data for the crystal form C of the ethanedisulfonate salt of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 6.5191 | 188.05 | 0.1023 | 13.56 | 17.64 |
| 11.6432 | 724.17 | 0.1023 | 7.60 | 67.92 |
| 13.0307 | 343.09 | 0.0768 | 6.79 | 32.18 |
| 13.8506 | 176.50 | 0.1023 | 6.39 | 16.55 |
| 15.4047 | 273.36 | 0.0768 | 5.75 | 25.64 |
| 16.3654 | 274.81 | 0.1279 | 5.42 | 25.77 |
| 17.3178 | 407.97 | 0.1279 | 5.12 | 38.26 |
| 18.1167 | 70.75 | 0.3070 | 4.90 | 6.64 |
| 19.5724 | 748.26 | 0.1279 | 4.54 | 70.18 |
| 20.3101 | 1066.22 | 0.1279 | 4.37 | 100.00 |
| 20.7875 | 243.31 | 0.1535 | 4.27 | 22.82 |
| 21.6456 | 134.26 | 0.1535 | 4.11 | 12.59 |
| 22.9576 | 440.53 | 0.1023 | 3.87 | 41.32 |
| 23.1939 | 379.67 | 0.1023 | 3.84 | 35.61 |
| 24.0056 | 605.49 | 0.1279 | 3.71 | 56.79 |
| 24.8706 | 134.54 | 0.1535 | 3.58 | 12.62 |
| 27.8869 | 136.21 | 0.2047 | 3.20 | 12.78 |
| 28.2146 | 172.44 | 0.1279 | 3.16 | 16.17 |
| 30.3093 | 101.29 | 0.2047 | 2.95 | 9.50 |
| 36.4827 | 67.26 | 0.3070 | 2.46 | 6.31 |
| 38.0492 | 74.21 | 0.3070 | 2.37 | 6.96 |

The characterization data for the crystal form C of the ethanedisulfonate salt of the compound represented by formula I-3B are shown in the following table:

| Type | Result |
|---|---|
| X-ray diffraction | see FIG. 23; |
| Differential scanning calorimetry | The DSC curve is shown in FIG. 24, comprising endothermic peaks with peak temperatures at 109.5°C and 243.6°C. |
| | Exothermic (upward). |
| Thermogravimetric analysis | The TGA curve is shown in FIG. 24, with a weight loss of 7.28% in the temperature range of 28.6°C to 150°C. |

The NMR data for the crystal form C of the ethanedisulfonate salt of the compound represented by formula I-3B were as follows:

| # | ppm | Integral |
|---|---|---|
| 1 | 8.05 | (1H, d) |
| 2 | 7.85 | (1H, d) |
| 3 | 7.65 | (1H, d) |
| 4 | 6.73 | (1H, d) |
| 5 | 6.60 | (1H, dd) |
| 6 | 3.92 | (1H, d) |
| 7 | 3.69-3.61 | (5H, m) |
| 8 | 3.50 | (3H, s) |
| 9 | 3.23 | (1H, m) |
| 10 | 2.28-2.17 | (2H, m) |
| 11 | 2.12-2.02 | (5H, m) |
| 12 | 1.14-1.06 | (2H, m) |

The molar ratio of ethanedisulfonic acid to the compound represented by formula I-3B was 1.0, the molar ratio of the solvent acetone to the compound represented by formula I-3B was 0.3 (2.3 wt%), and the molar ratio of the solvent n-heptane to the compound represented by formula I-3B was 0.03 (0.5 wt%).

### 4.10 Crystal form of methanesulfonate salt of compound represented by formula I-3B

The crystal form A of the compound represented by formula I-3B and an equimolar amount of methanesulfonic acid were suspended and stirred in acetone/n-heptane (1:1, v:v) at room temperature overnight to form a gel. The gel was subjected to temperature cycling (50°C to 5°C, heating rate of 4.5°C/min, maintaining the temperature at 50°C for 2 hours, cooling rate of 0.1°C/min, maintaining the temperature at 5°C for 2 hours, for a total of 6 cycles) for approximately 5 days to obtain the crystal form of the methanesulfonate salt of the compound represented by formula I-3B, which was then vacuum dried at room temperature overnight before characterization. The XRPD diffraction peak data for the crystal form of the methanesulfonate salt of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 6.4920 | 725.88 | 0.1023 | 13.62 | 83.98 |
| 7.9002 | 269.32 | 0.1023 | 11.19 | 31.16 |
| 8.4009 | 115.91 | 0.1535 | 10.53 | 13.41 |
| 9.3501 | 864.35 | 0.1279 | 9.46 | 100.00 |
| 10.4943 | 128.79 | 0.2047 | 8.43 | 14.90 |
| 12.2112 | 159.96 | 0.2047 | 7.25 | 18.51 |
| 12.9398 | 98.21 | 0.2047 | 6.84 | 11.36 |
| 15.2670 | 244.78 | 0.1279 | 5.80 | 28.32 |
| 15.5976 | 283.21 | 0.1279 | 5.68 | 32.77 |
| 16.3993 | 172.15 | 0.1535 | 5.41 | 19.92 |
| 16.8865 | 256.41 | 0.3070 | 5.25 | 29.66 |
| 18.1977 | 358.00 | 0.2047 | 4.88 | 41.42 |
| 18.5979 | 301.51 | 0.2047 | 4.77 | 34.88 |
| 19.8986 | 388.60 | 0.1535 | 4.46 | 44.96 |
| 20.5213 | 229.85 | 0.3070 | 4.33 | 26.59 |
| 21.8338 | 317.20 | 0.2303 | 4.07 | 36.70 |
| 22.1987 | 262.59 | 0.2047 | 4.00 | 30.38 |
| 23.8218 | 92.91 | 0.3582 | 3.74 | 10.75 |
| 24.9016 | 76.20 | 0.3070 | 3.58 | 8.82 |
| 25.9461 | 81.53 | 0.4093 | 3.43 | 9.43 |
| 27.2069 | 100.09 | 0.2558 | 3.28 | 11.58 |
| 29.7013 | 45.28 | 0.5117 | 3.01 | 5.24 |
| 30.5169 | 63.17 | 0.3070 | 2.93 | 7.31 |

The characterization data for the crystal form of the methanesulfonate salt of the compound represented by formula I-3B are shown in the following table:

| Type | Result |
|---|---|
| X-ray diffraction | see FIG. 25; |
| Differential scanning calorimetry | The DSC curve is shown in FIG. 26, comprising an endothermic peak with an onset temperature at 116.3°C and peak temperatures at 94.4°C and 124.3°C (enthalpy: 31.02 J/g). |
| | Exothermic (upward). |
| Thermogravimetric analysis | The TGA curve is shown in FIG. 26, with a weight loss of 4.53% in the temperature range of 28.4°C to 80°C. |

The NMR data for the crystal form of the methanesulfonate salt of the compound represented by formula I-3B were as follows:

| # | ppm | Integral |
|---|---|---|
| 1 | 8.05 | (1H, d) |
| 2 | 7.84 | (1H, d) |
| 3 | 7.65 | (1H, d) |
| 4 | 6.72 | (1H, d) |
| 5 | 6.60 | (1H, dd) |
| 6 | 3.93 | (1H, d) |
| 7 | 3.69-3.57 | (5H, m) |
| 8 | 3.50 | (3H, s) |
| 9 | 2.35 | (3H, s) |
| 10 | 2.23-2.18 | (1H, m) |
| 11 | 2.12-2.09 | (5H, m) |
| 14 | 1.12-1.07 | (2H, m) |

The molar ratio of methanesulfonic acid to the compound represented by formula I-3B was 1.0, and the molar ratio of the residual solvent n-heptane to the compound represented by formula I-3B was 0.008 (0.2 wt%).

### 4.11 Crystal form of benzenesulfonate salt of compound represented by formula I-3B

The crystal form A of the compound represented by formula I-3B and an equimolar amount of benzenesulfonic acid were suspended and stirred in IPA at room temperature for 8 days to obtain the crystal form of the benzenesulfonate salt of the compound represented by formula I-3B, which was then vacuum dried at room temperature overnight before characterization.

The XRPD diffraction peak data for the crystal form of the benzenesulfonate salt of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 6.9158 | 552.08 | 0.1023 | 12.78 | 35.99 |
| 8.1784 | 252.57 | 0.1023 | 10.81 | 16.47 |
| 9.6463 | 266.90 | 0.1023 | 9.17 | 17.40 |
| 10.6171 | 509.82 | 0.1023 | 8.33 | 33.24 |
| 12.9975 | 245.87 | 0.1023 | 6.81 | 16.03 |
| 14.4950 | 336.51 | 0.0768 | 6.11 | 21.94 |
| 15.0932 | 900.93 | 0.1023 | 5.87 | 58.73 |
| 15.7135 | 191.65 | 0.1023 | 5.64 | 12.49 |
| 16.3580 | 238.28 | 0.0768 | 5.42 | 15.53 |
| 16.9456 | 354.79 | 0.1023 | 5.23 | 23.13 |
| 17.2095 | 458.71 | 0.1023 | 5.15 | 29.90 |
| 17.9799 | 419.87 | 0.1023 | 4.93 | 27.37 |
| 19.1080 | 649.63 | 0.1023 | 4.64 | 42.35 |
| 19.5628 | 1456.45 | 0.0768 | 4.54 | 94.95 |
| 20.0072 | 1457.71 | 0.1279 | 4.44 | 95.03 |
| 20.5109 | 688.31 | 0.1023 | 4.33 | 44.87 |
| 20.9850 | 709.40 | 0.1023 | 4.23 | 46.25 |
| 21.2246 | 552.75 | 0.0768 | 4.19 | 36.03 |
| 21.4383 | 1533.97 | 0.1023 | 4.14 | 100.00 |
| 21.7020 | 405.01 | 0.0768 | 4.10 | 26.40 |
| 23.6100 | 101.13 | 0.2047 | 3.77 | 6.59 |
| 24.0518 | 175.81 | 0.1023 | 3.70 | 11.46 |
| 24.6720 | 213.86 | 0.1023 | 3.61 | 13.94 |
| 25.2329 | 102.55 | 0.1535 | 3.53 | 6.69 |
| 26.7253 | 285.92 | 0.1279 | 3.34 | 18.64 |
| 27.8291 | 192.59 | 0.1279 | 3.21 | 12.55 |
| 29.1875 | 107.64 | 0.1023 | 3.06 | 7.02 |
| 30.3499 | 228.24 | 0.1535 | 2.95 | 14.88 |
| 31.4304 | 282.10 | 0.1279 | 2.85 | 18.39 |
| 35.1487 | 79.46 | 0.1535 | 2.55 | 5.18 |

The characterization data for the crystal form of the benzenesulfonate salt of the compound represented by formula I-3B are shown in the following table:

| Type | Result |
|---|---|
| X-ray diffraction | see FIG. 27; |
| Differential scanning calorimetry | The DSC curve is shown in FIG. 28, comprising an endothermic peak with an onset temperature at 251.5°C and peak temperatures at 106.6°C and 254.1°C (enthalpy: 89.32 J/g). |
| | Exothermic (upward). |
| Thermogravimetric analysis | The TGA curve is shown in FIG. 28, with a weight loss of 1.32% in the temperature range of 28.4°C to 150°C. |

The NMR data for the crystal form of the benzenesulfonate salt of the compound represented by formula I-3B were as follows:

| # | ppm | Integral |
|---|---|---|
| 1 | 8.05 | (1H, d) |
| 2 | 7.86 | (1H, d) |
| 3 | 7.65 | (1H, d) |
| 4 | 7.62-7.58 | (2H, m) |
| 5 | 7.35-7.29 | (3H, m) |
| 6 | 6.73 | (1H, d) |
| 7 | 6.60 | (1H, dd) |
| 8 | 3.92 | (1H, d) |
| 9 | 3.69-3.61 | (5H, m) |
| 10 | 3.50 | (3H, s) |
| 11 | 2.23-2.18 | (1H, m) |
| 12 | 2.12-2.00 | (5H, m) |
| 13 | 1.11-1.05 | (2H, m) |

The molar ratio of benzenesulfonic acid to the compound represented by formula I-3B was 1.0, and the molar ratio of the residual solvent IPA to the compound represented by formula I-3B was 0.01 (0.1 wt%).

### 4.12 Crystal form of oxalate salt of compound represented by formula I-3B

The crystal form A of the compound represented by formula I-3B and an equimolar amount of oxalic acid were suspended and stirred in acetone/n-heptane (1:1, v:v) at room temperature for 8 days to obtain the crystal form of the oxalate salt of the compound represented by formula I-3B, which was then vacuum dried at room temperature overnight before characterization.

The XRPD diffraction peak data for the crystal form of the oxalate salt of the compound represented by formula I-3B are shown in the following table:

| **Position [°2Th.]** | **Peak height [cts]** | **FWHM [°2Th]** | **Interplanar spacing [Å]** | **Relative intensity [%]** |
|---|---|---|---|---|
| 7.8295 | 1331.50 | 0.1023 | 11.29 | 89.35 |
| 12.5604 | 362.10 | 0.1023 | 7.05 | 24.30 |
| 13.3365 | 401.04 | 0.1791 | 6.64 | 26.91 |
| 13.9846 | 369.62 | 0.1023 | 6.33 | 24.80 |
| 14.4767 | 432.28 | 0.1279 | 6.12 | 29.01 |
| 15.4167 | 612.60 | 0.1023 | 5.75 | 41.11 |
| 15.6570 | 996.13 | 0.1023 | 5.66 | 66.85 |
| 16.5308 | 855.99 | 0.1023 | 5.36 | 57.44 |
| 17.1400 | 932.66 | 0.1023 | 5.17 | 62.59 |
| 18.0738 | 263.97 | 0.1279 | 4.91 | 17.71 |
| 18.4049 | 407.73 | 0.1279 | 4.82 | 27.36 |
| 19.1721 | 820.36 | 0.1279 | 4.63 | 55.05 |
| 20.7476 | 51.01 | 0.3070 | 4.28 | 3.42 |
| 21.6726 | 344.41 | 0.1279 | 4.10 | 23.11 |
| 21.8928 | 284.03 | 0.0768 | 4.06 | 19.06 |
| 22.3565 | 434.28 | 0.1023 | 3.98 | 29.14 |
| 23.5654 | 459.67 | 0.1279 | 3.78 | 30.85 |
| 25.2383 | 181.26 | 0.1023 | 3.53 | 12.16 |
| 25.6656 | 316.93 | 0.1023 | 3.47 | 21.27 |
| 26.5959 | 324.97 | 0.1023 | 3.35 | 21.81 |
| 27.1484 | 245.83 | 0.1023 | 3.28 | 16.50 |
| 27.6765 | 1490.17 | 0.1535 | 3.22 | 100.00 |
| 28.5248 | 433.74 | 0.0768 | 3.13 | 29.11 |
| 29.2798 | 137.10 | 0.1791 | 3.05 | 9.20 |
| 30.8091 | 136.06 | 0.1535 | 2.90 | 9.13 |
| 31.5734 | 365.42 | 0.1023 | 2.83 | 24.52 |
| 33.0538 | 66.16 | 0.6140 | 2.71 | 4.44 |
| 36.8368 | 73.70 | 0.2047 | 2.44 | 4.95 |

The characterization data for the crystal form of the oxalate salt of the compound represented by formula I-3B are shown in the following table:

| Type | Result |
|---|---|
| X-ray diffraction | see FIG. 29; |
| Differential scanning calorimetry | The DSC curve is shown in FIG. 30, comprising an endothermic peak with an onset temperature at 121.4°C and peak temperatures at 129.7°C (enthalpy: 62.38 J/g) and 198.2°C. |
| | Exothermic (upward). |
| Thermogravimetric analysis | The TGA curve is shown in FIG. 30, with a weight loss of 2.27% in the temperature range of 28.4°C to 100°C. |

The NMR data for the crystal form of the oxalate salt of the compound represented by formula I-3B were as follows:

| # | ppm | Integral |
|---|---|---|
| 1 | 8.06 | (1H, d) |
| 2 | 7.83 | (1H, d) |
| 3 | 7.64 | (1H, d) |
| 4 | 6.71 | (1H, d) |
| 5 | 6.60 | (1H, dd) |
| 6 | 3.93 | (1H, d) |
| 7 | 3.68-3.61 | (5H, m) |
| 8 | 3.50 | (3H, s) |
| 9 | 2.23-2.17 | (1H, m) |
| 10 | 2.12-2.00 | (5H, m) |
| 11 | 1.10-1.05 | (2H, m) |

No residual solvent signal was observed in the NMR data. HPLC/IC results showed that the molar ratio of oxalic acid to the compound represented by formula I-3B was 1.4.

### Effect Example 1: Effect test of compound represented by formula I-3B

15-PGDH (R&D Systems, Cat. No. 5660-DH-010) was prepared to twice the final concentration, *i.e.,* 30 nM, using Assay Buffer (50 mM Tris-HCl, pH 7.5, 0.01% Tween 20 by volume). 15-PGDH was then added to a 384-well white plate (Cisbio Bioassays, Cat. No. 66PL384025) at 8 µL/well. Negative control wells (with Assay Buffer only and without enzyme) were set. The compound was then prepared to 4 times the final concentration using Assay Buffer, *i.e.,* diluted 3-fold to 10 concentrations starting from 4000 nM. The compound was added to the above white plate at 4 µL/well, mixed well, centrifuged at 1000 rpm for 1 minute, and incubated at 25°C for 10 minutes. Both positive control wells (with 15-PGDH only) and negative control wells (without 15-PGDH) were set. A mixture of NAD⁺ (Select, Cat. No. S2518) and PGE₂ (R&D Systems, Cat. No.:2296110) was then prepared using Assay Buffer. NAD⁺ and PGE₂ were prepared to four times their final concentrations, *i.e.,* 2 mM and 0.12 mM, respectively, using Assay Buffer. The mixture was then added to the above white plate at 4 µL/well, mixed well, centrifuged at 1000 rpm for 1 minute, and incubated at 25°C for 30 minutes for the reaction. The fluorescence was measured at an excitation wavelength of 340 nm and an emission wavelength of 485 nm using a TECAN SPARK 20M microplate reader. The IC₅₀ value was calculated using four-parameter fitting with GraphPad Prism 8.0.

The results of 15-PGDH inhibition by the compounds obtained in Example 1 are shown in the following table:

| Compound No. | IC₅₀ (nM) |
|---|---|
| Compound I-3 | 4.2 |
| Compound I-3A | 12.16 |
| Compound I-3B | 3.58 |

The experimental results showed that the compounds of the present disclosure had a significant inhibitory effect on 15-PGDH.

### Effect Example 2: Effect of compounds on PGE₂ levels in A549 cell supernatant

A549 cells (Procell, Wuhan) were cultured in F12K + 10% FBS. The cells with good status in log phase were selected for the experiment, digested, counted, and inoculated into a 24-well plate at 8,000 cells per well. The cells were cultured in a 37°C, 5% CO₂ incubator overnight. After adherence, the cells were transferred to a medium containing 0.5% FBS and cultured for approximately 10 hours. IL-1β was added to each well (final concentration of 20 ng/mL, 1 mL per well), and a control group (without IL-1β) was set up. After stimulation with IL-1β for approximately 24 hours, the cell culture medium of each well was aspirated and discarded, and the wells were gently washed with fresh medium containing 0.5% FBS. 400 µL of medium containing the compound obtained in Example 1 at various concentrations (20 nM and 2,500 nM) was added to each well and cultured for approximately 12 hours. The supernatant was collected and PGE₂ was detected using an ELISA kit (R&D Systems, Cat. No. KGE004B).

The fold increase of PGE₂ in A549 cell supernatant by the compounds obtained in Example 1 is shown in the following table:

| Compound No./concentration | 20 nM |
|---|---|
| Compound I-3A | 0.9 |
| Compound I-3B | 4.8 |

The experimental results showed that the compounds of the present disclosure could significantly increase the production of PGE₂.

### Effect Example 3: Efficacy test on mouse IPF prevention model

Male mice were adaptively fed for 1 to 2 weeks and after reaching the standard weight (25 g), the IPF model (idiopathic pulmonary fibrosis model) was induced with a certain dose of Bleomycin. On the day of modeling, the animals were randomly divided into a model group and an administration group according to their weight, in which the administration groups were administered Nintedanib (60 mg/kg, once a day (qd)) and compound I-3B obtained in Example 1 (2.5 mg/kg, twice a day (bid)) by oral gavage for 21 consecutive days while the vehicle control group was administered blank vehicle. During the administration period, the animals were weighed every 3 days. At the end of the last day of administration, the animals were euthanized. The lungs were removed from the thyroid cartilage (without perfusion), slowly perfused with 10% formalin until both lungs were filled, and fixed in 10% formalin 5 to 10 times the volume of the tissues after the trachea was ligated. The left lung tissues were embedded in paraffin wax, sectioned, and stained with HE and Masson Trichrome. The sections were subjected to panoramic scanning using a Hamamatsu NanoZoomer Digital Pathology (S210) slide scanner for pathological analysis.

The indicators for pathological evaluation of pulmonary fibrosis are shown in the following table:

| Fibrosis grade | Ashcroft scoring criteria |
|---|---|
| 0 | Alveolar septum: no fibrotic lesions; |
| | Lung structure: normal. |
| 1 | Alveolar septum: isolated gentle fibrotic changes (thickened alveolar septum, but less than three times that of normal lung); |
| | Lung structure: partly enlarged alveolar cavity, small amount of exudate, no fibrotic material. |
| 2 | Alveolar septum: definite fibrotic changes (thickened alveolar septum, larger than three times that of normal lung), formation of small nodules, but not connected; |
| | Lung structure: partly enlarged alveolar cavity, small amount of exudate, no fibrotic material. |
| 3 | Alveolar septum: non-intermittent fibrosis (thickened alveolar septum, larger than three times that of normal lung) is visible in almost all alveolar walls in each high-power field; |
| | Lung structure: partly enlarged alveolar cavity, small amount of exudate, no fibrotic material. |
| 4 | Alveolar septum: still visible; |
| | Lung structure: isolated fibrotic nodules in alveolar cavity (≤ 10% of high-power field). |
| 5 | Alveolar septum: still visible; |
| | Lung structure: confluent fibrotic nodules in alveolar cavity (> 10% to ≤ 50% of high-power field), lung architecture severely damaged but still existent. |
| 6 | Alveolar septum: visible but almost non-existent. |
| | Lung structure: large contiguous fibrotic nodules (> 50% of high-power field), lung architecture almost non-existent. |
| 7 | Alveolar septum: non-existent; |
| | Lung structure: alveolar cavity almost filled with fibrotic material but still up to five vacuole-like structures. |
| 8 | Alveolar septum: non-existent; |
| | Lung structure: alveolar cavity filled with fibrotic tissue in high-power field. |

The summary of pulmonary fibrosis scores (mean) is shown in the following table:

| Group | Total pulmonary fibrosis score |
|---|---|
| G1 sham surgery group (sham) | 0 |
| G2 model group | 4.69 |
| G3 Nintedanib | 3.39 |
| Compound I-3B | 3.34 |

The experimental results showed that the compounds of the present disclosure could significantly reduce the degree of fibrosis in the mouse IPF prevention model.

### Effect Example 4: Efficacy test on mouse IPF treatment model

Male mice were adaptively fed for 1 to 2 weeks and after reaching the standard weight (25 g), the IPF model (idiopathic pulmonary fibrosis model) was induced with a certain dose of Bleomycin. On the day of modeling, the animals were randomly divided into a model group and an administration group according to their weight, in which the administration groups were administered Nintedanib (60 mg/kg, qd), low-dose compound I-3B obtained in Example 1 (1 mg/kg, bid), and high-dose compound I-3B obtained in Example 1 (2.5 mg/kg, bid) by oral gavage for 14 consecutive days starting from day 7 while the vehicle control group was administered blank vehicle. During the administration period, the animals were weighed twice a week. At the end of the last day of administration, the animals were euthanized. The lungs were removed from the thyroid cartilage (without perfusion), slowly perfused with 10% formalin until both lungs were filled, and fixed in 10% formalin 5 to 10 times the volume of the tissues after the trachea was ligated. The left lung tissues were embedded in paraffin wax, sectioned, and stained with HE and Masson Trichrome. The sections were subjected to panoramic scanning using a Hamamatsu NanoZoomer Digital Pathology (S210) slide scanner for pathological analysis.

The indicators for pathological evaluation of pulmonary fibrosis are shown in the following table:

| Fibrosis grade | Ashcroft scoring criteria |
|---|---|
| 0 | Alveolar septum: no fibrotic lesions; |
| | Lung structure: normal. |
| 1 | Alveolar septum: isolated gentle fibrotic changes (thickened alveolar septum, but less than three times that of normal lung); |
| | Lung structure: partly enlarged alveolar cavity, small amount of exudate, no fibrotic material. |
| 2 | Alveolar septum: definite fibrotic changes (thickened alveolar septum, larger than three times that of normal lung), formation of small nodules, but not connected; |
| | Lung structure: partly enlarged alveolar cavity, small amount of exudate, no fibrotic material. |
| 3 | Alveolar septum: non-intermittent fibrosis (thickened alveolar septum, larger than three times that of normal lung) is visible in almost all alveolar walls in each high-power field; |
| | Lung structure: partly enlarged alveolar cavity, small amount of exudate, no fibrotic material. |
| 4 | Alveolar septum: still visible; |
| | Lung structure: isolated fibrotic nodules in alveolar cavity (≤ 10% of high-power field). |
| 5 | Alveolar septum: still visible; |
| | Lung structure: confluent fibrotic nodules in alveolar cavity (> 10% to ≤ 50% of high-power field), lung architecture severely damaged but still existent. |
| 6 | Alveolar septum: visible but almost non-existent. |
| | Lung structure: large contiguous fibrotic nodules (> 50% of high-power field), lung architecture almost non-existent. |
| 7 | Alveolar septum: non-existent; |
| | Lung structure: alveolar cavity almost filled with fibrotic material but still up to five vacuole-like structures. |
| 8 | Alveolar septum: non-existent; |
| | Lung structure: alveolar cavity filled with fibrotic tissue in high-power field. |

The summary of pulmonary fibrosis scores (mean) is shown in the following table:

| Group | Total pulmonary fibrosis score |
|---|---|
| G1 sham | 0 |
| G2 model group | 3.68 |
| G3 Nintedanib | 2.84 |
| G4 low-dose compound I-3B group | 2.74 |
| G4 high-dose compound I-3B group | 2.6 |

The experimental results showed that the compounds of the present disclosure could significantly reduce the degree of fibrosis in the mouse IPF treatment model.

### Effect Example 5: Efficacy test on mouse liver regeneration after resection

Male C57BL/6J mice aged 8 weeks (20 to 24 g) were anesthetized and fixed with the abdomen facing upwards. The surgical site was shaved and disinfected with iodophor. A transverse incision of approximately 1.5 to 2 cm was made at the abdomen, and the epigastric arteries on both sides were clamped with a hemostat. After opening the abdominal cavity, each liver lobe was freed, and the hilum of the liver lobe to be resected was ligated with surgical suture. The left outer lobe and middle lobe of the liver were resected as the color became darker. After surgery, the residual blood in the abdominal cavity was cleaned up, and the muscle layer and fur layer were sutured layer by layer. Postoperative care should be taken into consideration. Administration was started on the day of modeling, and 8 animals were killed on day 1 and day 3, respectively. Intact liver tissues were collected and weighed for comparison with the model group to evaluate the effect of the drug on promoting liver regeneration.

The experimental results showed that the compounds of the present disclosure could significantly promote liver regeneration.

### Effect Example 6: Pharmacokinetics in mice

The pharmacokinetic properties of the compounds obtained in Example 1 in mice were determined with reference to the following experimental methods.

Three male CD-1 mice were administered by oral gavage at a dose of 10 mg/kg, and the vehicle was 5% DMSO + 10% Solutol + 85% Saline. The mice were fasted overnight, and the time points of blood collection were before administration and at 15 minutes, 30 minutes, 1 hour, 2 hours, 4 hours, 8 hours, and 24 hours after administration. Blood samples were centrifuged at 6,800 g for 6 minutes at 2 to 8°C, and plasma was collected and stored at - 80°C. 10 µL of plasma at each time point was taken and added with 200 µL of methanol containing 100 ng/mL internal standard. The mixture was vortexed, mixed well, and centrifuged at 18,000 g for 7 minutes at 2 to 8°C. 200 µL of the mixture was transferred to a 96-well injection plate for LC-MS/MS quantitative analysis. The main pharmacokinetic parameters were analyzed using WinNonlin 7.0 software with non-compartmental model.

The pharmacokinetic data in mice are shown in the following table:

| Compound No. | Cₘₐₓ **(ng/mL)** | AUC₍₀₋ₜ₎ (h·ng/mL) |
|---|---|---|
| Compound I-3 | 3710 | 35502 |
| Compound I-3B | 4480 | 33173 |

The experimental results showed that the compounds of the present disclosure exhibited excellent pharmacokinetic properties in mice.

### Effect Example 7: Efficacy on IBD in mice

Female C57BL/6 mice aged 6 to 8 weeks were divided into 5 groups of G1 to G5, namely normal control group, model control group, positive control group, low-dose group of compound I-3B obtained in Example 1, and high-dose group of compound I-3B obtained in Example 1, respectively. The mice in G2 to G6 were given 2% DSS aqueous solution on day 0 to day 6, normal water on day 0 to day 10, vehicle/positive/test substance on day 0 to day 9, and euthanized on day 10 for necropsy. Colon weight (CW) and colon length (CL) were measured and colon tissues (BW) were subjected to histopathological examination. The experimental results showed that compared with the animals in the G2 model group, there was a significant increase in body weight of the animals in the G3-positive control group (cyclosporine CsA 25 mg/kg-qd); there was a significant decrease in DAI, a significant increase in CL, and a significant decrease in CW, CL/CW/BW, and CL/CW of the model animals; the results of the histopathological examination of colon tissues of the model animals showed that there was a decrease in inflammatory cell infiltration and tissue injury scores, but there was no significant difference. There was an increase in body weight of the animals in both G4-compound I-3B (2.5 mg/kg-bid) and G5-compound I-3B (5 mg/kg-bid) groups, with a significant increase in body weight of the animals in G5; there was a significant decrease in DAI, a significant increase in CL, and a significant decrease in CW, CL/CW/BW, and CL/CW of the animals in both groups; the results of the histopathological examination of colon tissues showed that there was a significant decrease in inflammatory cell infiltration and tissue injury scores in both groups.

The summary of colon length (CL), colon weight (CW), CL/CW/BW, and CW/CL of animals in each group is shown in the following table:

| Group | Colon length CL (cm) | Colon weight CW (g) | CW/CL/BW * 1000 | CW/CL * 10 |
|---|---|---|---|---|
| G1-normal control group | 6.90 | 0.15 | 1.11 | 0.21 |
| G2-model control group | 5.52 | 0.21 | 2.31 | 0.38 |
| G3-CsA group 25 mg/kg | 6.70 | 0.18 | 1.42 | 0.27 |
| G4-low-dose compound I-3B group | 6.67 | 0.17 | 1.39 | 0.26 |
| G5-high-dose compound I-3B group | 6.50 | 0.16 | 1.36 | 0.24 |

The experimental results showed that compound I-3B (2.5 mg/kg and 5 mg/kg) could significantly improve IBD symptoms and tissue injury in mice with the efficacy better than that of the positive control.

### Effect Example 8: Dynamic solubility and stability test of crystal forms

Samples of the crystal form A of the compound represented by formula I-3B and the crystal form of the fumarate complex of the compound represented by formula I-3B were tested for their dynamic solubility in H₂O, SGF, FaSSIF, and FeSSIF at 37°C.

The above samples were mixed by rotation at 37°C at a loading concentration of 10 mg/mL (calculated based on the compound represented by formula I-3B), and the solubility of each sample was measured at different time points (1, 4, and 24 hours). After sampling at each time point, the samples were centrifuged (12,000 rpm, 2 minutes) and filtered (0.45 µm PTFE membrane), and the HPLC concentration and pH value of the filtrate were measured. The solid samples after centrifugation were tested for XRPD.

The results of the dynamic solubility test are shown in the following table:

| Sample | Vehicle | 1 hour | | | 4 hours | | | 24 hours | | |
|---|---|---|---|---|---|---|---|---|---|---|
| | | S | pH | FC | S | pH | FC | S | pH | FC |
| Crystal form A of compound represented by formula I-3B | H₂O | 3.3 | 8.57 | No | 2.6 | 8.59 | No | 0.90 | 8.95 | No |
| | SGF | 5.3 | 1.82 | No | 5.1 | 1.94 | No | 4.7 | 2.11 | No |
| | FaSSIF | 2.6 | 6.46 | No | 2.5 | 6.60 | No | 2.7 | 6.70 | No |
| | FeSSIF | 3.0 | 4.91 | No | 3.4 | 4.96 | No | 3.9 | 4.93 | No |
| Crystal form of fumarate complex of compound represented by formula I-3B | H₂O | 5.1 | 2.46 | No | 5.4 | 2.54 | No | 5.7 | 2.39 | No |
| | SGF | 6.7 | 1.80 | No | 7.2 | 1.93 | - | 7.6 | 2.40 | No |
| | FaSSIF | 5.1 | 3.15 | No | 4.5 | 2.90 | - | 4.7 | 2.91 | No |
| | FeSSIF | 6.0 | 4.54 | No | 6.0 | 4.37 | No | 4.8 | 4.29 | No |

| | | | | | | | | | | |
|---|---|---|---|---|---|---|---|---|---|---|
| S: concentration of the compound represented by formula I-3B (mg/mL); FC: crystal form conversion, --: not tested. | | | | | | | | | | |

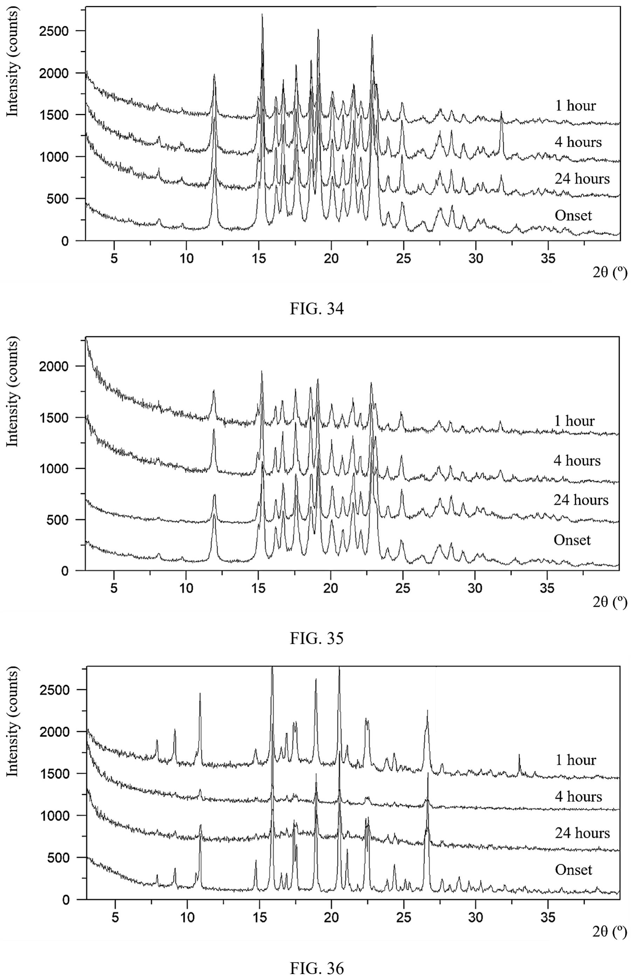

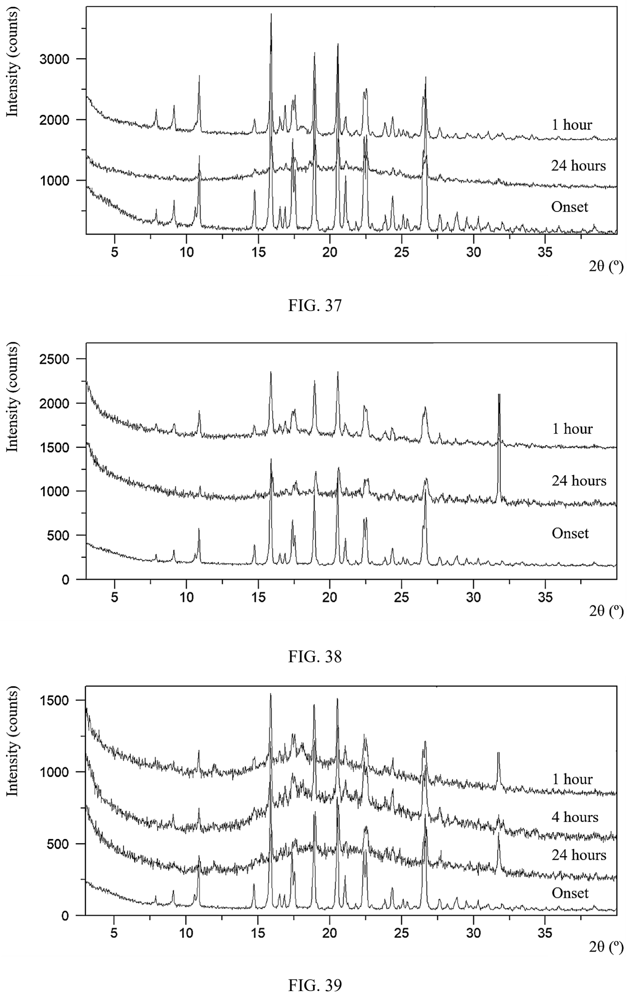

The results showed that the crystal form A of the compound represented by formula I-3B and the crystal form of the fumarate complex of the compound represented by formula I-3B exhibited good solubility in various vehicles. The XRPD results, as shown in FIGs. 32, 33, 34, 35, 36, 37, 38, and 39, indicated that when comparing the data of each sample at the onset, 1 hour, 4 hours, and 24 hours, no changes in crystal form were observed in the crystal form A of the compound represented by formula I-3B and the crystal form of the fumarate complex of the compound represented by formula I-3B in all tested systems, demonstrating excellent stability.

### Effect Example 9: Solid-state stability evaluation test

Samples of the crystal form A of the compound represented by formula I-3B, the crystal form B of the compound represented by formula I-3B, the crystal form of the fumarate complex of the compound represented by formula I-3B, and the crystal form of the tartrate salt of the compound represented by formula I-3B were placed under conditions of 60°C/closed/1 day, 25°C/60% RH/open/1 week, and 40°C/75% RH/open/1 week, respectively. Their physical and chemical stability were evaluated by XRPD and HPLC.

The purity and stability test data of the samples were as follows:

| Initial sample | Initial HPLC purity (area%) | Condition | HPLC purity (area%) | Change in crystal form |
|---|---|---|---|---|
| Crystal form A of compound represented by formula I-3B | 99.68 | 60°C/closed/1 day | 99.68 | No |
| | | 25°C/60% RH/open/1 week | 99.68 | No |
| | | 40°C/75% RH/open/1 week | 99.68 | No |
| Crystal form B of compound represented by formula I-3B | 99.72 | 60°C/closed/1 day | 99.72 | No |
| | | 25°C/60% RH/open/1 week | 99.72 | No |
| | | 40°C/75% RH/open/1 week | 99.72 | No |
| Crystal form of fumarate complex of compound represented by formula I-3B | 99.70 | 60°C/closed/1 day | 99.66 | No |
| | | 25°C/60% RH/open/1 week | 99.70 | No |
| | | 40°C/75% RH/open/1 week | 99.70 | No |
| Crystal form of tartrate salt of compound represented by formula I-3B | 99.71 | 60°C/closed/1 day | 99.70 | No |
| | | 25°C/60% RH/open/1 week | 99.71 | No |
| | | 40°C/75% RH/open/1 week | 99.71 | No |

The HPLC data for the crystal form A of the compound represented by formula I-3B were as follows:

| Peak | RRT (relative retention time) (min) | HPLC purity (area%) | | | |
|---|---|---|---|---|---|
| | | Onset | 60°C/closed/1 day | 25°C/60% RH/open/1 week | 40°C/75% RH/open/1 week |
| 1 | 0.52 | 0.22 | 0.22 | 0.22 | 0.22 |
| 2 | 0.97 | 0.10 | 0.10 | 0.10 | 0.09 |
| 3 | 1.00 | 99.68 | 99.68 | 99.68 | 99.68 |

| | | | | | |
|---|---|---|---|---|---|
| Note: The relative retention time of 1 min corresponds to the compound represented by formula I-3B in the sample. | | | | | |

The HPLC data for the crystal form B of the compound represented by formula I-3B were as follows:

| Peak | RRT (relative retention time) | HPLC purity (area%) | | | |
|---|---|---|---|---|---|
| | | Onset | 60°C/closed/1 day | 25°C/60% RH/open/1 week | 40°C/75% RH/open/1 week |
| 1 | 0.53 | 0.28 | 0.28 | 0.28 | 0.28 |
| 2 | 1.00 | 99.72 | 99.72 | 99.72 | 99.72 |

| | | | | | |
|---|---|---|---|---|---|
| Note: The relative retention time of 1 min corresponds to the compound represented by formula I-3B in the sample. | | | | | |

The HPLC data for the crystal form of the fumarate complex of the compound represented by formula I-3B were as follows:

| Peak | RRT (relative retention time) | HPLC purity (area%) | | | |
|---|---|---|---|---|---|
| | | Onset | 60°C/closed/1 day | 25°C/60% RH/open/1 week | 40°C/75% RH/open/1 week |
| 1 | 0.52 | 0.24 | 0.24 | 0.24 | 0.24 |
| 2 | 0.97 | 0.06 | 0.05 | 0.06 | 0.06 |
| 3 | 1.00 | 99.70 | 99.66 | 99.70 | 99.70 |

| | | | | | |
|---|---|---|---|---|---|
| Note: The relative retention time of 1 min corresponds to the compound represented by formula I-3B in the sample. | | | | | |

The HPLC data for the crystal form of the L-tartrate salt of the compound represented by formula I-3B were as follows:

| Peak | RRT (relative retention time) | HPLC purity (area%) | | | |
|---|---|---|---|---|---|
| | | Onset | 60°C/closed/1 day | 25°C/60% RH/open/1 week | 40°C/75% RH/open/1 week |
| 1 | 0.52 | 0.23 | 0.24 | 0.23 | 0.23 |
| 2 | 0.97 | 0.06 | 0.06 | 0.06 | 0.06 |
| 3 | 1.00 | 99.71 | 99.70 | 99.71 | 99.71 |

| | | | | | |
|---|---|---|---|---|---|
| Note: The relative retention time of 1 min corresponds to the compound represented by formula I-3B in the sample. | | | | | |

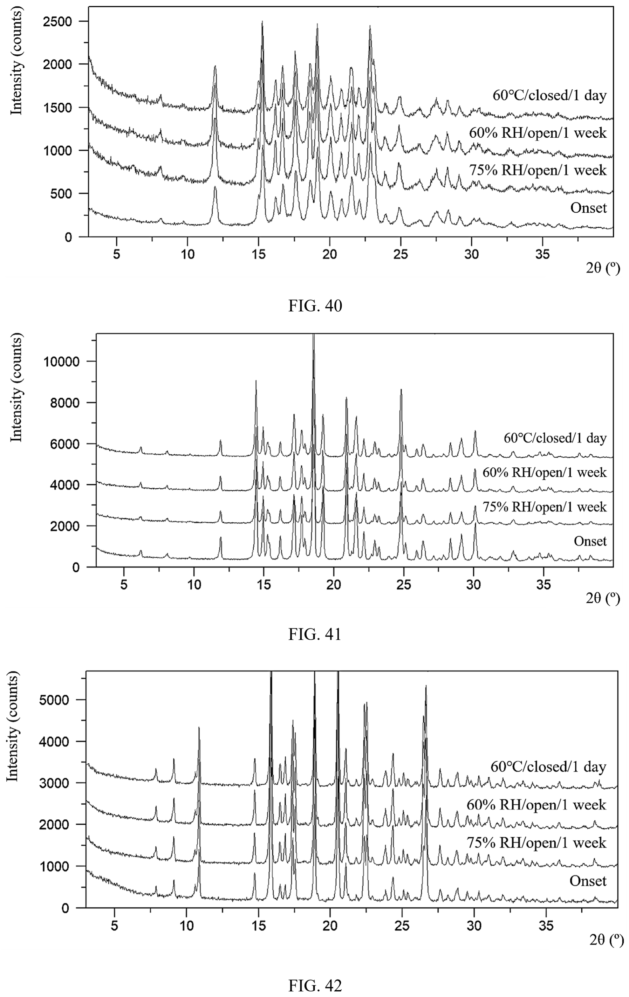

The XRPD results are shown in FIGs. 40, 41, 42, and 43. The results showed that each tested crystal form maintained good purity after a period of storage, with essentially no significant change in HPLC purity. No changes in crystal form were observed in any of the tested crystal forms, demonstrating high stability.

### Effect Example 10: Hygroscopicity test

The crystal form A of the compound represented by formula I-3B, the crystal form B of the compound represented by formula I-3B, the crystal form of the fumarate complex of the compound represented by formula I-3B, and the crystal form of the tartrate salt of the compound represented by formula I-3B were evaluated for their hygroscopicity using DVS.

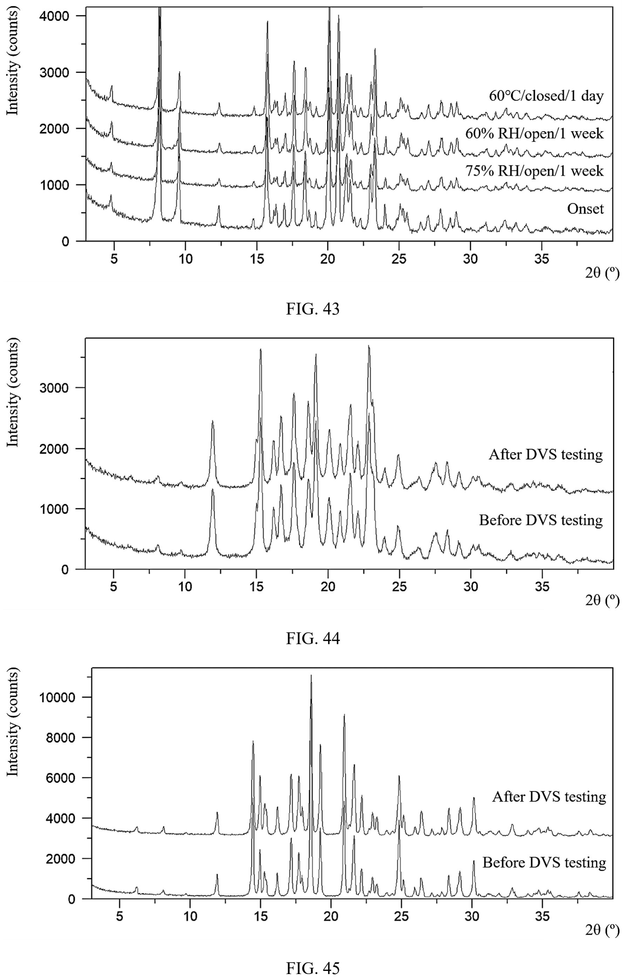

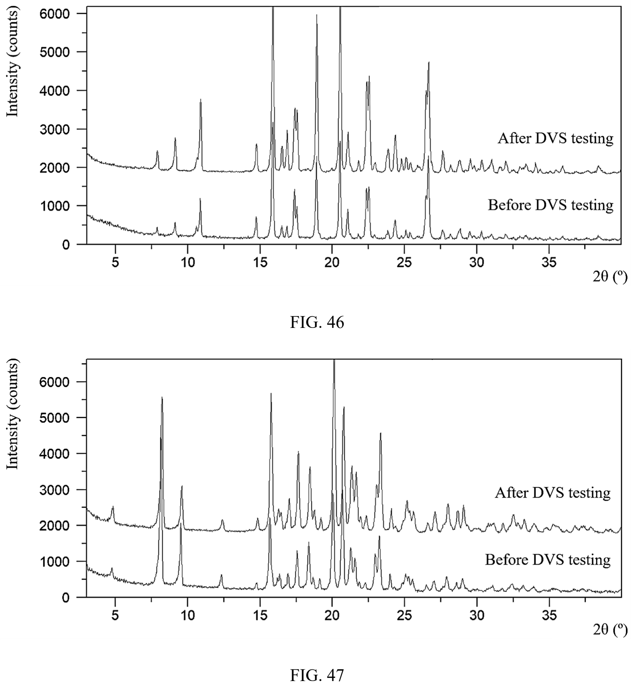

The results showed that the test samples exhibited good hygroscopicity. The XRPD results of the samples after DVS testing are shown in FIGs. 44, 45, 46, and 47. By comparing the data before and after DVS testing, the results of hygroscopicity evaluation indicated that the crystal form A of the compound represented by formula I-3B had a moisture absorption weight gain of 1.112% at 25°C/80% RH, with no change in crystal form after testing; the crystal form B of the compound represented by formula I-3B had a moisture absorption weight gain of 0.1309% at 25°C/80% RH, with no hygroscopicity and no change in crystal form after testing; the crystal form of the fumarate complex of the compound represented by formula I-3B had a moisture absorption weight gain of 0.0880% at 25°C/80% RH, with no hygroscopicity and no change in crystal form after testing; the crystal form of the tartrate salt of the compound represented by formula I-3B had a moisture absorption weight gain of 0.2291% at 25°C/80% RH, with slight hygroscopicity and no change in crystal form after testing.

Although specific embodiments of the present disclosure have been described above, those skilled in the art should understand that these are merely illustrative examples and that various changes or modifications can be made to these embodiments without departing from the principles and essence of the present disclosure. Therefore, the scope of protection of the present disclosure is defined by the appended claims.

## Claims

1. A pharmaceutically acceptable salt of a heterocyclic compound or a solvate thereof, wherein the heterocyclic compound is a compound represented by formula I-3A and/or a compound represented by formula I-3B; 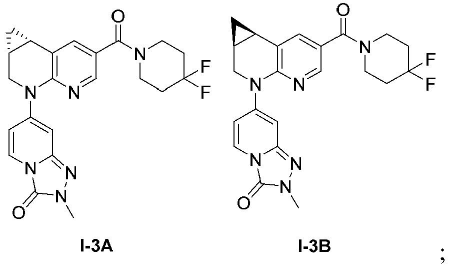 wherein the pharmaceutically acceptable salt is a salt formed by the heterocyclic compound with an acid; the acid is selected from the group consisting of hydrochloric acid, phosphoric acid, fumaric acid, tartaric acid, malic acid, ethanedisulfonic acid, *p-*toluenesulfonic acid, methanesulfonic acid, benzenesulfonic acid, and oxalic acid.

2. The pharmaceutically acceptable salt of the heterocyclic compound or the solvate thereof according to claim 1, wherein the pharmaceutically acceptable salt of the heterocyclic compound or the solvate thereof satisfies one or more of the following conditions:
(1) in the solvate of the pharmaceutically acceptable salt of the heterocyclic compound, the solvent is one or two selected from the group consisting of isopropyl acetate, acetone, isopropanol, and *n*-heptane;
(2) when the heterocyclic compound is a compound represented by formula I-3A and a compound represented by formula I-3B, the heterocyclic compound is a compound represented by formula I-3: 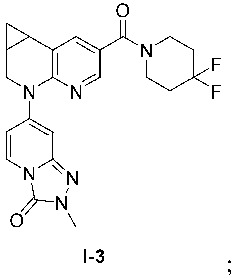
(3) in the pharmaceutically acceptable salt of the heterocyclic compound or the solvate thereof, the heterocyclic compound and the acid have a molar ratio of 1:(0.5 to 2); for example, 1:(0.5 to 1); for another example, 1:1, 1:0.9, or 1:0.5;
(4) the heterocyclic compound is a compound represented by formula I-3B: 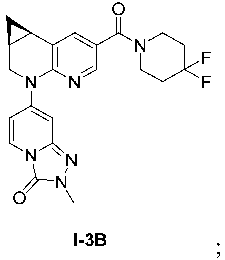
(5) the solvate of the pharmaceutically acceptable salt of the heterocyclic compound is not a monohydrochloride monohydrate of the heterocyclic compound; and
(6) in the solvate of the pharmaceutically acceptable salt of the heterocyclic compound, the heterocyclic compound and the solvent have a molar ratio of 1:(0.001 to 2); for example, 1:(0.001 to 1); for another example, 1:0.05, 1:0.9, 1:0.01, 1:0.04, 1:0.008, 1:0.03, 1:0.3, or 1:0.5.

3. The pharmaceutically acceptable salt of the heterocyclic compound or the solvate thereof according to claim 1, wherein the pharmaceutically acceptable salt of the heterocyclic compound or the solvate thereof is:
an acetone solvate of a hydrochloride salt of the compound represented by formula I-3B, wherein hydrochloric acid, the compound represented by formula I-3B, and acetone have a molar ratio of 0.9:1:0.9;
or, a phosphate salt of the compound represented by formula I-3B, wherein phosphoric acid and the compound represented by formula I-3B have a molar ratio of 1:1;
or, an acetone solvate of a phosphate salt of the compound represented by formula I-3B, wherein phosphoric acid, the compound represented by formula I-3B, and acetone have a molar ratio of 1:1:0.05;
or, a fumarate salt of the compound represented by formula I-3B, wherein fumaric acid and the compound represented by formula I-3B have a molar ratio of 1:1;
or, an L-tartrate salt of the compound represented by formula I-3B, wherein tartaric acid and the compound represented by formula I-3B have a molar ratio of 1:1;
or, an L-malate salt of the compound represented by formula I-3B, wherein malic acid and the compound represented by formula I-3B have a molar ratio of 1:1;
or, an isopropanol solvate of an ethanedisulfonate salt of the compound represented by formula I-3B, wherein ethanedisulfonic acid, the compound represented by formula I-3B, and isopropanol have a molar ratio of 0.5:1:0.5;
or, an ethanedisulfonate salt of the compound represented by formula I-3B, wherein ethanedisulfonic acid and the compound represented by formula I-3B have a molar ratio of 0.5:1;
or, an ethanedisulfonate salt of the compound represented by formula I-3B, wherein ethanedisulfonic acid and the compound represented by formula I-3B have a molar ratio of 1:1;
or, an acetone/*n*-heptane solvate of an ethanedisulfonate salt of the compound represented by formula I-3B, wherein ethanedisulfonic acid, the compound represented by formula I-3B, acetone, and *n*-heptane have a molar ratio of 1:1:0.3:0.03;
or, a *p*-toluenesulfonate salt of the compound represented by formula I-3B, wherein *p-*toluenesulfonic acid and the compound represented by formula I-3B have a molar ratio of 1:1;
or, a methanesulfonate salt of the compound represented by formula I-3B, wherein methanesulfonic acid and the compound represented by formula I-3B have a molar ratio of 1:1;
or, an *n*-heptane solvate of a methanesulfonate salt of the compound represented by formula I-3B, wherein methanesulfonic acid, the compound represented by formula I-3B, and *n*-heptane have a molar ratio of 1:1:0.008;
or, a benzenesulfonate salt of the compound represented by formula I-3B, wherein benzenesulfonic acid and the compound represented by formula I-3B have a molar ratio of 1:1;
or, an isopropanol solvate of a benzenesulfonate salt of the compound represented by formula I-3B, wherein benzenesulfonic acid, the compound represented by formula I-3B, and isopropanol have a molar ratio of 1:1:0.01;
or, an oxalate salt of the compound represented by formula I-3B, wherein oxalic acid and the compound represented by formula I-3B have a molar ratio of 1:1.

4. A crystal form, which is a crystal form A of a compound represented by formula I-3B, a crystal form B of a compound represented by formula I-3B, a crystal form of a hydrochloride salt of a compound represented by formula I-3B, a crystal form of a phosphate salt of a compound represented by formula I-3B, a crystal form of a fumarate complex of a compound represented by formula I-3B, a crystal form of an L-tartrate salt of a compound represented by formula I-3B, a crystal form of a *p*-toluenesulfonate salt of a compound represented by formula I-3B, a crystal form of an L-malate salt of a compound represented by formula I-3B, a crystal form A of an ethanedisulfonate salt of a compound represented by formula I-3B, a crystal form B of an ethanedisulfonate salt of a compound represented by formula I-3B, a crystal form C of an ethanedisulfonate salt of a compound represented by formula I-3B, a crystal form of a methanesulfonate salt of a compound represented by formula I-3B, a crystal form of a benzenesulfonate salt of a compound represented by formula I-3B, or a crystal form of an oxalate salt of a compound represented by formula I-3B;
wherein the compound represented by formula I-3B has a structure of 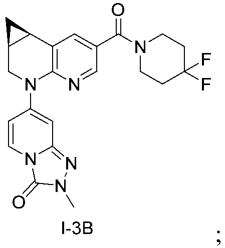
the crystal form A of the compound represented by formula I-3B has an X-ray powder diffraction pattern using Cu-Kα radiation and expressed by 20 angles comprising diffraction peaks at 15.2 ± 0.2°, 22.8 ± 0.2°, 19.1 ± 0.2°, 18.6 ± 0.2°, and 21.6 ± 0.2°;
the crystal form B of the compound represented by formula I-3B has an X-ray powder diffraction pattern using Cu-Kα radiation and expressed by 20 angles comprising diffraction peaks at 18.5 ± 0.2°, 24.8 ± 0.2°, 21.6 ± 0.2°, 20.9 ± 0.2°, and 14.4 ± 0.2°;
the crystal form of the hydrochloride salt of the compound represented by formula I-3B has an X-ray powder diffraction pattern using Cu-Kα radiation and expressed by 20 angles comprising diffraction peaks at 9.5 ± 0.2°, 24.8 ± 0.2°, 17.3 ± 0.2°, 19.3 ± 0.2°, and 11.7 ± 0.2°;
the crystal form of the phosphate salt of the compound represented by formula I-3B has an X-ray powder diffraction pattern using Cu-Kα radiation and expressed by 2θ angles comprising diffraction peaks at 16.7 ± 0.2°, 16.3 ± 0.2°, 8.3 ± 0.2°, 21.1 ± 0.2°, and 11.5 ± 0.2°;
the crystal form of the fumarate complex of the compound represented by formula I-3B has an X-ray powder diffraction pattern using Cu-Kα radiation and expressed by 20 angles comprising diffraction peaks at 20.5 ± 0.2°, 15.9 ± 0.2°, 26.6 ± 0.2°, 18.9 ± 0.2°, and 22.5 ± 0.2°;
the crystal form of the L-tartrate salt of the compound represented by formula I-3B has an X-ray powder diffraction pattern using Cu-Kα radiation and expressed by 2θ angles comprising diffraction peaks at 8.2 ± 0.2°, 20.7 ± 0.2°, 20.1 ± 0.2°, 15.7 ± 0.2°, and 23.3 ± 0.2°;
the crystal form of the p-toluenesulfonate salt of the compound represented by formula I-3B has an X-ray powder diffraction pattern using Cu-Kα radiation and expressed by 2θ angles comprising a diffraction peak at 6.6 ± 0.2°;
the crystal form of the L-malate salt of the compound represented by formula I-3B has an X-ray powder diffraction pattern using Cu-Kα radiation and expressed by 2θ angles comprising diffraction peaks at 15.8 ± 0.2°, 20.3 ± 0.2°, 18.8 ± 0.2°, 21.8 ± 0.2°, and 22.0 ± 0.2°;
the crystal form A of the ethanedisulfonate salt of the compound represented by formula I-3B has an X-ray powder diffraction pattern using Cu-Kα radiation and expressed by 2θ angles comprising diffraction peaks at 5.3 ± 0.2° and 18.9 ± 0.2°;
the crystal form B of the ethanedisulfonate salt of the compound represented by formula I-3B has an X-ray powder diffraction pattern using Cu-Kα radiation and expressed by 2θ angles comprising diffraction peaks at 18.6 ± 0.2°, 19.4 ± 0.2°, and 21.6 ± 0.2°;
the crystal form C of the ethanedisulfonate salt of the compound represented by formula I-3B has an X-ray powder diffraction pattern using Cu-Kα radiation and expressed by 2θ angles comprising diffraction peaks at 20.3 ± 0.2°, 19.6 ± 0.2°, 11.6 ± 0.2°, 24.0 ± 0.2°, and 23.0 ± 0.2°;
the crystal form of the methanesulfonate salt of the compound represented by formula I-3B has an X-ray powder diffraction pattern using Cu-Kα radiation and expressed by 2θ angles comprising diffraction peaks at 9.4 ± 0.2°, 6.5 ± 0.2°, 19.9 ± 0.2°, 18.2 ± 0.2°, and 21.8 ± 0.2°;
the crystal form of the benzenesulfonate salt of the compound represented by formula I-3B has an X-ray powder diffraction pattern using Cu-Kα radiation and expressed by 2θ angles comprising diffraction peaks at 21.4 ± 0.2°, 20.0 ± 0.2°, 19.6 ± 0.2°, 15.1 ± 0.2°, and 21.0 ± 0.2°;
the crystal form of the oxalate salt of the compound represented by formula I-3B has an X-ray powder diffraction pattern using Cu-Kα radiation and expressed by 2θ angles comprising diffraction peaks at 27.7 ± 0.2°, 7.8 ± 0.2°, 15.7 ± 0.2°, 17.1 ± 0.2°, and 16.5 ± 0.2°.

5. The crystal form according to claim 4, wherein the crystal form satisfies one or more of the following conditions:
(1) the X-ray powder diffraction pattern for the crystal form A of the compound represented by formula I-3B, using Cu-Kα radiation and expressed by 2θ angles, further comprises diffraction peaks at one or more of 23.1 ± 0.2°, 17.6 ± 0.2°, 11.9 ± 0.2°, 24.9 ± 0.2°, and 20.1 ± 0.2°;
for example, the X-ray powder diffraction pattern for the crystal form A of the compound represented by formula I-3B, using Cu-Kα radiation and expressed by 2θ angles, comprises diffraction peaks at 15.2 ± 0.2°, 22.8 ± 0.2°, 19.1 ± 0.2°, 17.6 ± 0.2°, 21.6 ± 0.2°, 18.6 ± 0.2°, and 11.9 ± 0.2°;
for another example, the X-ray powder diffraction pattern for the crystal form A of the compound represented by formula I-3B, using Cu-Kα radiation and expressed by 2θ angles, comprises diffraction peaks at 15.2 ± 0.2°, 22.8 ± 0.2°, 19.1 ± 0.2°, 17.6 ± 0.2°, 21.6 ± 0.2°, 23.1 ± 0.2°, 18.6 ± 0.2°, 11.9 ± 0.2°, 24.9 ± 0.2°, and 20.1 ± 0.2°;
(2) the X-ray powder diffraction pattern for the crystal form B of the compound represented by formula I-3B, using Cu-Kα radiation and expressed by 2θ angles, further comprises diffraction peaks at one or more of 14.9 ± 0.2°, 19.2 ± 0.2°, 17.7 ± 0.2°, 17.1 ± 0.2°, and 15.2 ± 0.2°;
for example, the X-ray powder diffraction pattern for the crystal form B of the compound represented by formula I-3B, using Cu-Kα radiation and expressed by 2θ angles, comprises diffraction peaks at 18.5 ± 0.2°, 24.8 ± 0.2°, 21.6 ± 0.2°, 20.9 ± 0.2°, 14.4 ± 0.2°, 14.9 ± 0.2°, and 19.2 ± 0.2°;
for another example, the X-ray powder diffraction pattern for the crystal form B of the compound represented by formula I-3B, using Cu-Kα radiation and expressed by 20 angles, comprises diffraction peaks at 18.5 ± 0.2°, 24.8 ± 0.2°, 21.6 ± 0.2°, 20.9 ± 0.2°, 14.4 ± 0.2°, 14.9 ± 0.2°, 19.2 ± 0.2°, 17.7 ± 0.2°, 17.1 ± 0.2°, and 15.2 ± 0.2°;
(3) in the crystal form of the hydrochloride salt of the compound represented by formula I-3B, the compound represented by formula I-3B and hydrochloric acid have a molar ratio of 1:0.9;
or, the crystal form of the hydrochloride salt of the compound represented by formula I-3B is a crystal form of the acetone solvate of the hydrochloride salt of the compound represented by formula I-3B, wherein the compound represented by formula I-3B, hydrochloric acid, and acetone have a molar ratio of 1:0.9:0.9;
(4) the X-ray powder diffraction pattern for the crystal form of the hydrochloride salt of the compound represented by formula I-3B, using Cu-Kα radiation and expressed by 2θ angles, further comprises diffraction peaks at one or more of 20.7 ± 0.2°, 18.9 ± 0.2°, 22.8 ± 0.2°, 6.6 ± 0.2°, and 22.7 ± 0.2°;
for example, the X-ray powder diffraction pattern for the crystal form of the hydrochloride salt of the compound represented by formula I-3B, using Cu-Kα radiation and expressed by 2θ angles, comprises diffraction peaks at 9.5 ± 0.2°, 24.8 ± 0.2°, 17.3 ± 0.2°, 19.3 ± 0.2°, 11.7 ± 0.2°, and 20.7 ± 0.2°;
for another example, the X-ray powder diffraction pattern for the crystal form of the hydrochloride salt of the compound represented by formula I-3B, using Cu-Kα radiation and expressed by 2θ angles, comprises diffraction peaks at 9.5 ± 0.2°, 24.8 ± 0.2°, 17.3 ± 0.2°, 19.3 ± 0.2°, 11.7 ± 0.2°, 20.7 ± 0.2°, 18.9 ± 0.2°, 22.8 ± 0.2°, 6.6 ± 0.2°, and 22.7 ± 0.2°;
(5) in the crystal form of the phosphate salt of the compound represented by formula I-3B, the compound represented by formula I-3B and phosphoric acid have a molar ratio of 1:0.9;
or, the crystal form of the phosphate salt of the compound represented by formula I-3B is a crystal form of the acetone solvate of the phosphate salt of the compound represented by formula I-3B, wherein the compound represented by formula I-3B, phosphoric acid, and acetone have a molar ratio of 1:0.9:0.05;
(6) the X-ray powder diffraction pattern for the crystal form of the phosphate salt of the compound represented by formula I-3B, using Cu-Kα radiation and expressed by 2θ angles, further comprises diffraction peaks at one or more of 23.2 ± 0.2°, 19.2 ± 0.2°, 20.1 ± 0.2°, 22.3 ± 0.2°, and 10.2 ± 0.2°;
for example, the X-ray powder diffraction pattern for the crystal form of the phosphate salt of the compound represented by formula I-3B, using Cu-Kα radiation and expressed by 2θ angles, comprises diffraction peaks at 16.7 ± 0.2°, 16.3 ± 0.2°, 8.3 ± 0.2°, 21.1 ± 0.2°, 11.5 ± 0.2°, 23.2 ± 0.2°, and 19.2 ± 0.2°;
for another example, the X-ray powder diffraction pattern for the crystal form of the phosphate salt of the compound represented by formula I-3B, using Cu-Kα radiation and expressed by 2θ angles, comprises diffraction peaks at 16.7 ± 0.2°, 16.3 ± 0.2°, 8.3 ± 0.2°, 21.1 ± 0.2°, 11.5 ± 0.2°, 23.2 ± 0.2°, 19.2 ± 0.2°, 20.1 ± 0.2°, 22.3 ± 0.2°, and 10.2 ± 0.2°;
(7) the X-ray powder diffraction pattern for the crystal form of the fumarate complex of the compound represented by formula I-3B, using Cu-Kα radiation and expressed by 2θ angles, further comprises diffraction peaks at one or more of 22.3 ± 0.2°, 26.4 ± 0.2°, 10.8 ± 0.2°, 17.4 ± 0.2°, and 17.5 ± 0.2°;
for example, the X-ray powder diffraction pattern for the crystal form of the fumarate complex of the compound represented by formula I-3B, using Cu-Kα radiation and expressed by 2θ angles, comprises diffraction peaks at 20.5 ± 0.2°, 15.9 ± 0.2°, 26.6 ± 0.2°, 18.9 ± 0.2°, 22.5 ± 0.2°, 22.3 ± 0.2°, and 26.4 ± 0.2°;
for another example, the X-ray powder diffraction pattern for the crystal form of the fumarate complex of the compound represented by formula I-3B, using Cu-Kα radiation and expressed by 2θ angles, comprises diffraction peaks at 20.5 ± 0.2°, 15.9 ± 0.2°, 26.6 ± 0.2°, 18.9 ± 0.2°, 22.5 ± 0.2°, 22.3 ± 0.2°, 26.4 ± 0.2°, 10.8 ± 0.2°, 17.4 ± 0.2°, and 17.5 ± 0.2°;
(8) the X-ray powder diffraction pattern for the crystal form of the L-tartrate salt of the compound represented by formula I-3B, using Cu-Kα radiation and expressed by 2θ angles, further comprises diffraction peaks at one or more of 18.4 ± 0.2°, 9.6 ± 0.2°, 17.6 ± 0.2°, 21.3 ± 0.2°, and 23.0 ± 0.2°;
for example, the X-ray powder diffraction pattern for the crystal form of the L-tartrate salt of the compound represented by formula I-3B, using Cu-Kα radiation and expressed by 2θ angles, comprises diffraction peaks at 8.2 ± 0.2°, 20.7 ± 0.2°, 20.1 ± 0.2°, 15.7 ± 0.2°, 23.3 ± 0.2°, and 18.4 ± 0.2°;
for another example, the X-ray powder diffraction pattern for the crystal form of the L-tartrate salt of the compound represented by formula I-3B, using Cu-Kα radiation and expressed by 2θ angles, comprises diffraction peaks at 8.2 ± 0.2°, 20.7 ± 0.2°, 20.1 ± 0.2°, 15.7 ± 0.2°, 23.3 ± 0.2°, 18.4 ± 0.2°, 9.6 ± 0.2°, 17.6 ± 0.2°, 21.3 ± 0.2°, and 23.0 ± 0.2°;
(9) the X-ray powder diffraction pattern for the crystal form of the *p*-toluenesulfonate salt of the compound represented by formula I-3B, using Cu-Kα radiation and expressed by 2θ angles, further comprises diffraction peaks at one or more of 19.8 ± 0.2°, 18.5 ± 0.2°, 19.7 ± 0.2°, 20.4 ± 0.2°, 9.8 ± 0.2°, 11.4 ± 0.2°, 13.2 ± 0.2°, 22.3 ± 0.2°, and 8.1 ± 0.2°;
for example, the X-ray powder diffraction pattern for the crystal form of the *p-*toluenesulfonate salt of the compound represented by formula I-3B, using Cu-Kα radiation and expressed by 2θ angles, comprises diffraction peaks at 6.6 ± 0.2°, 19.8 ± 0.2°, 18.5 ± 0.2°, 19.7 ± 0.2°, and 20.4 ± 0.2°;
for another example, the X-ray powder diffraction pattern for the crystal form of the *p-*toluenesulfonate salt of the compound represented by formula I-3B, using Cu-Kα radiation and expressed by 2θ angles, comprises diffraction peaks at 6.6 ± 0.2°, 19.8 ± 0.2°, 18.5 ± 0.2°, 19.7 ± 0.2°, 20.4 ± 0.2°, 9.8 ± 0.2°, 11.4 ± 0.2°, 13.2 ± 0.2°, 22.3 ± 0.2°, and 8.1 ± 0.2°;
(10) the X-ray powder diffraction pattern for the crystal form of the L-malate salt of the compound represented by formula I-3B, using Cu-Kα radiation and expressed by 2θ angles, further comprises diffraction peaks at one or more of 20.6 ± 0.2°, 25.5 ± 0.2°, 17.2 ± 0.2°, 16.1 ± 0.2°, and 17.6 ± 0.2°;
for example, the X-ray powder diffraction pattern for the crystal form of the L-malate salt of the compound represented by formula I-3B, using Cu-Kα radiation and expressed by 2θ angles, comprises diffraction peaks at 15.8 ± 0.2°, 20.3 ± 0.2°, 18.8 ± 0.2°, 21.8 ± 0.2°, 22.0 ± 0.2°, 20.6 ± 0.2°, 25.5 ± 0.2°, and 17.2 ± 0.2°;
for another example, the X-ray powder diffraction pattern for the crystal form of the L-malate salt of the compound represented by formula I-3B, using Cu-Kα radiation and expressed by 2θ angles, comprises diffraction peaks at 15.8 ± 0.2°, 20.3 ± 0.2°, 18.8 ± 0.2°, 21.8 ± 0.2°, 22.0 ± 0.2°, 20.6 ± 0.2°, 25.5 ± 0.2°, 17.2 ± 0.2°, 16.1 ± 0.2°, and 17.6 ± 0.2°;
(11) in the crystal form A of the ethanedisulfonate salt of the compound represented by formula I-3B, the compound represented by formula I-3B and ethanedisulfonic acid have a molar ratio of 1:0.5;
or, the crystal form A of the ethanedisulfonate salt of the compound represented by formula I-3B is a crystal form of the isopropanol solvate of the ethanedisulfonate salt of the compound represented by formula I-3B, wherein the compound represented by formula I-3B, ethanedisulfonic acid, and isopropanol have a molar ratio of 1:0.5:0.5;
(12) the X-ray powder diffraction pattern for the crystal form A of the ethanedisulfonate salt of the compound represented by formula I-3B, using Cu-Kα radiation and expressed by 2θ angles, further comprises diffraction peaks at one or more of 20.1 ± 0.2°, 21.0 ± 0.2°, 18.2 ± 0.2°, 21.6 ± 0.2°, 17.4 ± 0.2°, 22.3 ± 0.2°, and 11.5 ± 0.2°;
for example, the X-ray powder diffraction pattern for the crystal form A of the ethanedisulfonate salt of the compound represented by formula I-3B, using Cu-Kα radiation and expressed by 2θ angles, comprises diffraction peaks at 5.3 ± 0.2°, 18.9 ± 0.2°, 20.1 ± 0.2°, 21.0 ± 0.2°, 18.2 ± 0.2°, and 21.6 ± 0.2°;
for another example, the X-ray powder diffraction pattern for the crystal form A of the ethanedisulfonate salt of the compound represented by formula I-3B, using Cu-Kα radiation and expressed by 2θ angles, comprises diffraction peaks at 5.3 ± 0.2°, 18.9 ± 0.2°, 20.1 ± 0.2°, 21.0 ± 0.2°, 18.2 ± 0.2°, 21.6 ± 0.2°, 17.4 ± 0.2°, 22.3 ± 0.2°, and 11.5 ± 0.2°;
(13) the X-ray powder diffraction pattern for the crystal form B of the ethanedisulfonate salt of the compound represented by formula I-3B, using Cu-Kα radiation and expressed by 2θ angles, further comprises diffraction peaks at one or more of 17.7 ± 0.2°, 22.1 ± 0.2°, 15.1 ± 0.2°, 6.3 ± 0.2°, 20.8 ± 0.2°, 20.4 ± 0.2°, and 11.0 ± 0.2°;
for example, the X-ray powder diffraction pattern for the crystal form B of the ethanedisulfonate salt of the compound represented by formula I-3B, using Cu-Kα radiation and expressed by 2θ angles, comprises diffraction peaks at 18.6 ± 0.2°, 19.4 ± 0.2°, 21.6 ± 0.2°, 17.7 ± 0.2°, 22.1 ± 0.2°, and 15.1 ± 0.2°;
for another example, the X-ray powder diffraction pattern for the crystal form B of the ethanedisulfonate salt of the compound represented by formula I-3B, using Cu-Kα radiation and expressed by 2θ angles, comprises diffraction peaks at 18.6 ± 0.2°, 19.4 ± 0.2°, 21.6 ± 0.2°, 17.7 ± 0.2°, 22.1 ± 0.2°, 15.1 ± 0.2°, 6.3 ± 0.2°, 20.8 ± 0.2°, 20.4 ± 0.2°, and 11.0 ± 0.2°;
(14) in the crystal form C of the ethanedisulfonate salt of the compound represented by formula I-3B, the compound represented by formula I-3B and ethanedisulfonic acid have a molar ratio of 1:1;
or, the crystal form C of the ethanedisulfonate salt of the compound represented by formula I-3B is a crystal form of the acetone/*n*-heptane solvate of the ethanedisulfonate salt of the compound represented by formula I-3B, wherein the compound represented by formula I-3B, ethanedisulfonic acid, acetone, and *n*-heptane have a molar ratio of 1:1:0.3:0.03;
(15) the X-ray powder diffraction pattern for the crystal form C of the ethanedisulfonate salt of the compound represented by formula I-3B, using Cu-Kα radiation and expressed by 2θ angles, further comprises diffraction peaks at one or more of 7.3 ± 0.2°, 23.2 ± 0.2°, 13.0 ± 0.2°, 16.4 ± 0.2°, and 15.4 ± 0.2°;
for example, the X-ray powder diffraction pattern for the crystal form C of the ethanedisulfonate salt of the compound represented by formula I-3B, using Cu-Kα radiation and expressed by 2θ angles, comprises diffraction peaks at 20.3 ± 0.2°, 19.6 ± 0.2°, 11.6 ± 0.2°, 24.0 ± 0.2°, 23.0 ± 0.2°, and 17.3 ± 0.2°;
for another example, the X-ray powder diffraction pattern for the crystal form C of the ethanedisulfonate salt of the compound represented by formula I-3B, using Cu-Kα radiation and expressed by 2θ angles, comprises diffraction peaks at 20.3 ± 0.2°, 19.6 ± 0.2°, 11.6 ± 0.2°, 24.0 ± 0.2°, 23.0 ± 0.2°, 17.3 ± 0.2°, 23.2 ± 0.2°, 13.0 ± 0.2°, 16.4 ± 0.2°, and 15.4 ± 0.2°;
(16) in the crystal form of the methanesulfonate salt of the compound represented by formula I-3B, the compound represented by formula I-3B and methanesulfonic acid have a molar ratio of 1:1;
or, the crystal form of the methanesulfonate salt of the compound represented by formula I-3B is a crystal form of the *n*-heptane solvate of the methanesulfonate salt of the compound represented by formula I-3B, wherein the compound represented by formula I-3B, methanesulfonic acid, and *n*-heptane have a molar ratio of 1:1:0.008;
(17) the X-ray powder diffraction pattern for the crystal form of the methanesulfonate salt of the compound represented by formula I-3B, using Cu-Kα radiation and expressed by 2θ angles, further comprises diffraction peaks at one or more of 18.6 ± 0.2°, 15.6 ± 0.2°, 7.6 ± 0.2°, 22.2 ± 0.2°, and 16.9 ± 0.2°;
for example, the X-ray powder diffraction pattern for the crystal form of the methanesulfonate salt of the compound represented by formula I-3B, using Cu-Kα radiation and expressed by 2θ angles, comprises diffraction peaks at 9.4 ± 0.2°, 6.5 ± 0.2°, 19.9 ± 0.2°, 18.2 ± 0.2°, 21.8 ± 0.2°, 18.6 ± 0.2°, and 15.6 ± 0.2°;
for another example, the X-ray powder diffraction pattern for the crystal form of the methanesulfonate salt of the compound represented by formula I-3B, using Cu-Kα radiation and expressed by 2θ angles, comprises diffraction peaks at 9.4 ± 0.2°, 6.5 ± 0.2°, 19.9 ± 0.2°, 18.2 ± 0.2°, 21.8 ± 0.2°, 18.6 ± 0.2°, 15.6 ± 0.2°, 7.6 ± 0.2°, 22.2 ± 0.2°, and 16.9 ± 0.2°;
(18) in the crystal form of the benzenesulfonate salt of the compound represented by formula I-3B, the compound represented by formula I-3B and benzenesulfonic acid have a molar ratio of 1:1;
or, the crystal form of the benzenesulfonate salt of the compound represented by formula I-3B is a crystal form of the isopropanol solvate of the benzenesulfonate salt of the compound represented by formula I-3B, wherein the compound represented by formula I-3B, benzenesulfonic acid, and isopropanol have a molar ratio of 1:1:0.01;
(19) the X-ray powder diffraction pattern for the crystal form of the benzenesulfonate salt of the compound represented by formula I-3B, using Cu-Kα radiation and expressed by 2θ angles, further comprises diffraction peaks at one or more of 20.5 ± 0.2°, 19.1 ± 0.2°, 21.2 ± 0.2°, 6.9 ± 0.2°, and 10.6 ± 0.2°;
for example, the X-ray powder diffraction pattern for the crystal form of the benzenesulfonate salt of the compound represented by formula I-3B, using Cu-Kα radiation and expressed by 2θ angles, comprises diffraction peaks at 21.4 ± 0.2°, 20.0 ± 0.2°, 19.6 ± 0.2°, 15.1 ± 0.2°, 21.0 ± 0.2°, 20.5 ± 0.2°, and 19.1 ± 0.2°;
for another example, the X-ray powder diffraction pattern for the crystal form of the benzenesulfonate salt of the compound represented by formula I-3B, using Cu-Kα radiation and expressed by 2θ angles, comprises diffraction peaks at 21.4 ± 0.2°, 20.0 ± 0.2°, 19.6 ± 0.2°, 15.1 ± 0.2°, 21.0 ± 0.2°, 20.5 ± 0.2°, 19.1 ± 0.2°, 21.2 ± 0.2°, 6.9 ± 0.2°, and 10.6 ± 0.2°;
(20) the X-ray powder diffraction pattern for the crystal form of the oxalate salt of the compound represented by formula I-3B, using Cu-Kα radiation and expressed by 2θ angles, further comprises diffraction peaks at one or more of 19.2 ± 0.2°, 15.4 ± 0.2°, 23.6 ± 0.2°, and 22.4 ± 0.2°;
for example, the X-ray powder diffraction pattern for the crystal form of the oxalate salt of the compound represented by formula I-3B, using Cu-Kα radiation and expressed by 2θ angles, comprises diffraction peaks at 27.7 ± 0.2°, 7.8 ± 0.2°, 15.7 ± 0.2°, 17.1 ± 0.2°, 16.5 ± 0.2°, 19.2 ± 0.2°, and 15.4 ± 0.2°;
for another example, the X-ray powder diffraction pattern for the crystal form of the oxalate salt of the compound represented by formula I-3B, using Cu-Kα radiation and expressed by 2θ angles, comprises diffraction peaks at 27.7 ± 0.2°, 7.8 ± 0.2°, 15.7 ± 0.2°, 17.1 ± 0.2°, 16.5 ± 0.2°, 19.2 ± 0.2°, 15.4 ± 0.2°, 23.6 ± 0.2°, and 22.4 ± 0.2°;
(21) the crystal form A of the compound represented by formula I-3B is a crystal form of an isopropyl acetate solvate of the compound represented by formula I-3B, wherein the compound represented by formula I-3B and the isopropyl acetate have a molar ratio of 1:0.04;
(22) in the crystal form of the fumarate complex of the compound represented by formula I-3B, the compound represented by formula I-3B and fumaric acid have a molar ratio of 1:1;
preferably, the crystal form of the fumarate complex of the compound represented by formula I-3B is a co-crystal of the compound represented by formula I-3B with fumaric acid;
(23) in the crystal form of the L-tartrate salt of the compound represented by formula I-3B, the compound represented by formula I-3B and L-tartaric acid have a molar ratio of 1:1;
(24) in the crystal form of the *p*-toluenesulfonate salt of the compound represented by formula I-3B, the compound represented by formula I-3B and *p*-toluenesulfonic acid have a molar ratio of 1:1;
(25) in the crystal form of the L-malate salt of the compound represented by formula I-3B, the compound represented by formula I-3B and L-malic acid have a molar ratio of 1:1;
(26) in the crystal form B of the ethanedisulfonate salt of the compound represented by formula I-3B, the compound represented by formula I-3B and ethanedisulfonic acid have a molar ratio of 1:0.5; and
(27) in the crystal form of the oxalate salt of the compound represented by formula I-3B, the compound represented by formula I-3B and oxalic acid have a molar ratio of 1:1.

6. The crystal form according to claim 4, wherein the crystal form satisfies one or more of the following conditions:
(1) the X-ray powder diffraction pattern for the crystal form A of the compound represented by formula I-3B, using Cu-Kα radiation and expressed by 2θ angles, comprises diffraction peaks shown in Table 1:
**Table 1**
| **Position 2θ (°)** | **Interplanar spacing d (Å)** | **Relative intensity (%)** |
|---|---|---|
| 6.14 | 14.39 | 3.71 |
| 8.04 | 10.99 | 6.06 |
| 11.91 | 7.43 | 49.01 |
| 14.92 | 5.94 | 37.01 |
| 15.24 | 5.81 | 100.00 |
| 16.16 | 5.49 | 37.26 |
| 16.67 | 5.32 | 37.25 |
| 17.58 | 5.05 | 60.25 |
| 18.59 | 4.77 | 98.08 |
| 19.11 | 4.65 | 71.94 |
| 20.06 | 4.43 | 38.44 |
| 20.81 | 4.27 | 23.77 |
| 21.55 | 4.12 | 60.76 |
| 22.04 | 4.03 | 29.34 |
| 22.81 | 3.90 | 80.16 |
| 23.12 | 3.85 | 46.89 |
| 23.92 | 3.72 | 15.01 |
| 24.87 | 3.58 | 38.17 |
| 26.31 | 3.39 | 7.03 |
| 27.50 | 3.24 | 16.40 |
| 28.28 | 3.16 | 20.42 |
| 29.10 | 3.07 | 16.35 |
| 30.07 | 2.97 | 9.08 |
| 30.46 | 2.93 | 8.08 |
| 31.20 | 2.87 | 3.62 |
| 32.79 | 2.73 | 4.15 |
| 34.29 | 2.62 | 5.74 |
| 34.78 | 2.58 | 5.55 |
| 35.39 | 2.54 | 5.04 |
| 36.02 | 2.49 | 3.52 |
| 37.71 | 2.39 | 1.22 |
;
for example, the X-ray powder diffraction pattern for the crystal form A of the compound represented by formula I-3B using Cu-Kα radiation is substantially shown in FIG. 1;
(2) the crystal form A of the compound represented by formula I-3B has a differential scanning calorimetry curve comprising an endothermic peak with an onset temperature at 160.9 ± 3°C, and/or the crystal form A of the compound represented by formula I-3B has a differential scanning calorimetry curve comprising an endothermic peak with a peak temperature at 168.4 ± 3°C;
for example, the differential scanning calorimetry curve for the crystal form A of the compound represented by formula I-3B is substantially shown in FIG. 2;
(3) the crystal form A of the compound represented by formula I-3B has a thermogravimetric analysis curve with a weight loss of approximately 0.67% in the temperature range of 14.8 ± 3°C to 120 ± 3°C;
for example, the thermogravimetric analysis curve for the crystal form A of the compound represented by formula I-3B is substantially shown in FIG. 2;
(4) the X-ray powder diffraction pattern for the crystal form B of the compound represented by formula I-3B, using Cu-Kα radiation and expressed by 2θ angles, comprises diffraction peaks shown in Table 2:
**Table 2**
| **Position 2θ (°)** | **Interplanar spacing d (Å)** | **Relative intensity (%)** |
|---|---|---|
| 6.15 | 14.37 | 3.97 |
| 11.88 | 7.45 | 7.22 |
| 14.41 | 6.15 | 30.17 |
| 14.92 | 5.94 | 24.38 |
| 15.22 | 5.82 | 12.40 |
| 16.15 | 5.49 | 9.96 |
| 17.13 | 5.18 | 18.22 |
| 17.68 | 5.02 | 19.97 |
| 18.54 | 4.79 | 100.00 |
| 19.20 | 4.62 | 22.51 |
| 20.88 | 4.25 | 30.48 |
| 21.61 | 4.11 | 31.37 |
| 22.12 | 4.02 | 10.83 |
| 22.90 | 3.88 | 7.05 |
| 23.22 | 3.83 | 5.82 |
| 24.79 | 3.59 | 39.54 |
| 25.92 | 3.44 | 2.22 |
| 26.38 | 3.38 | 7.73 |
| 27.12 | 3.29 | 2.20 |
| 28.33 | 3.15 | 8.37 |
| 29.10 | 3.07 | 11.50 |
| 30.07 | 2.97 | 11.74 |
| 31.19 | 2.87 | 1.88 |
| 32.84 | 2.73 | 3.38 |
| 35.44 | 2.53 | 2.18 |
| 37.55 | 2.40 | 2.27 |
| 38.42 | 2.34 | 1.32 |
;
for example, the X-ray powder diffraction pattern for the crystal form B of the compound represented by formula I-3B using Cu-Kα radiation is substantially shown in FIG. 4;
(5) the crystal form B of the compound represented by formula I-3B has a differential scanning calorimetry curve comprising an endothermic peak with an onset temperature at approximately 164.2 ± 3°C, and/or the crystal form B of the compound represented by formula I-3B has a differential scanning calorimetry curve comprising an endothermic peak with a peak temperature at approximately 171.7 ± 3°C;
for example, the differential scanning calorimetry curve for the crystal form B of the compound represented by formula I-3B is substantially shown in FIG. 5;
(6) the crystal form B of the compound represented by formula I-3B has a thermogravimetric analysis curve with a weight loss of approximately 1.61% in the temperature range of 26.6 ± 3°C to 120 ± 3°C;
for example, the thermogravimetric analysis curve for the crystal form B of the compound represented by formula I-3B is substantially shown in FIG. 5;
(7) the X-ray powder diffraction pattern for the crystal form of the hydrochloride salt of the compound represented by formula I-3B, using Cu-Kα radiation and expressed by 2θ angles, comprises diffraction peaks shown in Table 3:
**Table 3**
| **Position 2θ (°)** | **Interplanar spacing d (Å)** | **Relative intensity (%)** |
|---|---|---|
| 6.63 | 13.33 | 37.55 |
| 9.47 | 9.34 | 100.00 |
| 11.73 | 7.54 | 63.89 |
| 12.36 | 7.16 | 16.20 |
| 15.30 | 5.79 | 8.06 |
| 16.01 | 5.54 | 30.30 |
| 16.31 | 5.44 | 18.97 |
| 16.55 | 5.36 | 19.67 |
| 17.29 | 5.13 | 66.41 |
| 17.76 | 5.00 | 19.17 |
| 18.87 | 4.70 | 43.17 |
| 19.31 | 4.60 | 66.26 |
| 19.85 | 4.47 | 27.36 |
| 20.69 | 4.29 | 48.38 |
| 21.96 | 4.05 | 23.61 |
| 22.66 | 3.92 | 32.60 |
| 22.82 | 3.90 | 41.01 |
| 23.11 | 3.85 | 24.05 |
| 24.79 | 3.59 | 75.90 |
| 26.03 | 3.42 | 9.46 |
| 26.33 | 3.38 | 9.22 |
| 27.18 | 3.28 | 18.90 |
| 27.72 | 3.22 | 11.75 |
| 28.02 | 3.18 | 14.19 |
| 28.59 | 3.12 | 7.60 |
| 29.25 | 3.05 | 4.86 |
| 29.71 | 3.01 | 14.70 |
| 29.94 | 2.98 | 24.07 |
| 31.39 | 2.85 | 6.62 |
| 34.33 | 2.61 | 4.43 |
| 35.75 | 2.51 | 6.95 |
| 36.55 | 2.46 | 2.45 |
| 38.52 | 2.34 | 4.06 |
;
for example, the X-ray powder diffraction pattern for the crystal form of the hydrochloride salt of the compound represented by formula I-3B using Cu-Kα radiation is substantially shown in FIG. 7;
(8) the crystal form of the hydrochloride salt of the compound represented by formula I-3B has a differential scanning calorimetry curve comprising endothermic peaks with peak temperatures at 122.1 ± 3°C and 199.6 ± 3°C;
for example, the differential scanning calorimetry curve for the crystal form of the hydrochloride salt of the compound represented by formula I-3B is substantially shown in FIG. 8;
(9) the crystal form of the hydrochloride salt of the compound represented by formula I-3B has a thermogravimetric analysis curve with a weight loss of approximately 13.39% in the temperature range of 17.7 ± 3°C to 100 ± 3°C and a weight loss of approximately 4.54% in the temperature range of 100 ± 3°C to 150 ± 3°C;
for example, the thermogravimetric analysis curve for the crystal form of the hydrochloride salt of the compound represented by formula I-3B is substantially shown in FIG. 8;
(10) the X-ray powder diffraction pattern for the crystal form of the phosphate salt of the compound represented by formula I-3B, using Cu-Kα radiation and expressed by 2θ angles, comprises diffraction peaks shown in Table 4:
**Table 4**
| **Position 2θ (°)** | **Interplanar spacing d (Å)** | **Relative intensity (%)** |
|---|---|---|
| 8.31 | 10.64 | 37.46 |
| 10.16 | 8.70 | 9.93 |
| 11.55 | 7.66 | 33.58 |
| 16.25 | 5.45 | 43.24 |
| 16.65 | 5.32 | 100.00 |
| 19.21 | 4.62 | 29.94 |
| 20.09 | 4.42 | 16.37 |
| 21.08 | 4.22 | 34.82 |
| 22.29 | 3.99 | 11.90 |
| 23.20 | 3.83 | 29.96 |
| 24.43 | 3.64 | 6.64 |
| 25.20 | 3.53 | 7.81 |
| 26.49 | 3.36 | 6.33 |
;
for example, the X-ray powder diffraction pattern for the crystal form of the phosphate salt of the compound represented by formula I-3B using Cu-Kα radiation is substantially shown in FIG. 9;
(11) the crystal form of the phosphate salt of the compound represented by formula I-3B has a differential scanning calorimetry curve comprising an endothermic peak with a peak temperature at 158.5 ± 3°C;
for example, the differential scanning calorimetry curve for the crystal form of the phosphate salt of the compound represented by formula I-3B is substantially shown in FIG. 10;
(12) the crystal form of the phosphate salt of the compound represented by formula I-3B has a thermogravimetric analysis curve with a weight loss of approximately 2.07% in the temperature range of 27.4 ± 3°C to 120 ± 3°C;
for example, the thermogravimetric analysis curve for the crystal form of the phosphate salt of the compound represented by formula I-3B is substantially shown in FIG. 10;
(13) the X-ray powder diffraction pattern for the crystal form of the fumarate complex of the compound represented by formula I-3B, using Cu-Kα radiation and expressed by 2θ angles, comprises diffraction peaks shown in Table 5:
**Table 5**
| **Position 2θ (°)** | **Interplanar spacing d (Å)** | **Relative intensity (%)** |
|---|---|---|
| 7.87 | 11.23 | 9.15 |
| 9.10 | 9.71 | 16.03 |
| 10.86 | 8.15 | 45.45 |
| 14.72 | 6.02 | 13.65 |
| 15.87 | 5.58 | 89.76 |
| 16.49 | 5.37 | 12.73 |
| 16.84 | 5.26 | 18.86 |
| 17.36 | 5.11 | 35.75 |
| 17.54 | 5.06 | 30.19 |
| 18.40 | 4.82 | 3.74 |
| 18.89 | 4.70 | 80.22 |
| 20.52 | 4.33 | 100.00 |
| 21.05 | 4.22 | 25.20 |
| 21.79 | 4.08 | 5.77 |
| 22.33 | 3.98 | 52.76 |
| 22.52 | 3.95 | 65.05 |
| 22.90 | 3.88 | 8.22 |
| 23.84 | 3.73 | 11.46 |
| 24.33 | 3.66 | 23.71 |
| 24.75 | 3.60 | 6.20 |
| 25.07 | 3.55 | 10.79 |
| 25.37 | 3.51 | 6.04 |
| 26.44 | 3.37 | 50.87 |
| 26.63 | 3.35 | 82.72 |
| 27.61 | 3.23 | 11.00 |
| 28.15 | 3.17 | 3.20 |
| 28.81 | 3.10 | 20.52 |
| 29.47 | 3.03 | 13.22 |
| 30.29 | 2.95 | 8.79 |
| 30.66 | 2.92 | 1.26 |
| 30.99 | 2.89 | 7.39 |
| 31.58 | 2.83 | 2.70 |
| 31.97 | 2.80 | 5.06 |
| 32.97 | 2.72 | 3.13 |
| 33.39 | 2.68 | 3.93 |
| 34.02 | 2.63 | 4.10 |
| 35.89 | 2.50 | 3.79 |
| 38.38 | 2.35 | 4.03 |
;
for example, the X-ray powder diffraction pattern for the crystal form of the fumarate complex of the compound represented by formula I-3B using Cu-Kα radiation is substantially shown in FIG. 11;
(14) the crystal form of the fumarate complex of the compound represented by formula I-3B has a differential scanning calorimetry curve comprising an endothermic peak with an onset temperature at 165.2 ± 3°C, and/or the crystal form of the fumarate complex of the compound represented by formula I-3B has a differential scanning calorimetry curve comprising an endothermic peak with a peak temperature at 167.2 ± 3°C;
for example, the differential scanning calorimetry curve for the crystal form of the fumarate complex of the compound represented by formula I-3B is substantially shown in FIG. 12;
(15) the crystal form of the fumarate complex of the compound represented by formula I-3B has a thermogravimetric analysis curve with a weight loss of approximately 0.36% in the temperature range of 26.2 ± 3°C to 120 ± 3°C;
for example, the thermogravimetric analysis curve for the crystal form of the fumarate complex of the compound represented by formula I-3B is substantially shown in FIG. 12;
(16) the X-ray powder diffraction pattern for the crystal form of the L-tartrate salt of the compound represented by formula I-3B, using Cu-Kα radiation and expressed by 2θ angles, comprises diffraction peaks shown in Table 6:
**Table 6**
| **Position 2θ (°)** | **Interplanar spacing d (Å)** | **Relative intensity (%)** |
|---|---|---|
| 4.80 | 18.39 | 16.15 |
| 8.21 | 10.77 | 100.00 |
| 9.58 | 9.23 | 41.59 |
| 12.37 | 7.15 | 9.37 |
| 14.81 | 5.98 | 4.17 |
| 15.74 | 5.63 | 62.64 |
| 16.44 | 5.39 | 10.08 |
| 16.99 | 5.22 | 14.58 |
| 17.61 | 5.04 | 37.38 |
| 18.42 | 4.82 | 45.16 |
| 18.73 | 4.74 | 10.46 |
| 19.18 | 4.63 | 10.38 |
| 20.10 | 4.42 | 93.66 |
| 20.74 | 4.28 | 95.51 |
| 21.32 | 4.17 | 37.28 |
| 21.61 | 4.11 | 31.13 |
| 22.25 | 4.00 | 5.53 |
| 22.99 | 3.87 | 33.19 |
| 23.30 | 3.82 | 54.10 |
| 24.04 | 3.70 | 15.95 |
| 25.10 | 3.55 | 16.33 |
| 25.61 | 3.48 | 11.18 |
| 26.57 | 3.36 | 5.64 |
| 27.06 | 3.30 | 12.24 |
| 27.96 | 3.19 | 15.84 |
| 28.61 | 3.12 | 7.39 |
| 29.03 | 3.08 | 16.42 |
| 31.11 | 2.87 | 6.12 |
| 31.78 | 2.82 | 3.72 |
| 32.46 | 2.76 | 9.00 |
| 33.23 | 2.70 | 6.73 |
| 33.90 | 2.64 | 4.81 |
| 35.31 | 2.54 | 3.69 |
;
for example, the X-ray powder diffraction pattern for the crystal form of the L-tartrate salt of the compound represented by formula I-3B using Cu-Kα radiation is substantially shown in FIG. 13;
(17) the crystal form of the L-tartrate salt of the compound represented by formula I-3B has a differential scanning calorimetry curve comprising an endothermic peak with an onset temperature at 162.4 ± 3°C, and/or the crystal form of the L-tartrate salt of the compound represented by formula I-3B has a differential scanning calorimetry curve comprising an endothermic peak with a peak temperature at 165.7 ± 3°C;
for example, the differential scanning calorimetry curve for the crystal form of the L-tartrate salt of the compound represented by formula I-3B is substantially shown in FIG. 14;
(18) the crystal form of the L-tartrate salt of the compound represented by formula I-3B has a thermogravimetric analysis curve with a weight loss of approximately 0.57% in the temperature range of 25.9 ± 3°C to 120 ± 3°C;
for example, the thermogravimetric analysis curve for the crystal form of the L-tartrate salt of the compound represented by formula I-3B is substantially shown in FIG. 14;
(19) the X-ray powder diffraction pattern for the crystal form of the p-toluenesulfonate salt of the compound represented by formula I-3B, using Cu-Kα radiation and expressed by 2θ angles, comprises diffraction peaks shown in Table 7:
**Table 7**
| **Position 2θ (°)** | **Interplanar spacing d (Å)** | **Relative intensity (%)** |
|---|---|---|
| 6.59 | 13.41 | 100.00 |
| 8.06 | 10.97 | 4.95 |
| 9.80 | 9.02 | 8.34 |
| 11.40 | 7.77 | 6.24 |
| 12.69 | 6.98 | 2.00 |
| 13.19 | 6.71 | 6.24 |
| 13.81 | 6.41 | 4.74 |
| 13.99 | 6.33 | 3.32 |
| 15.30 | 5.79 | 2.39 |
| 16.20 | 5.47 | 2.83 |
| 16.42 | 5.40 | 2.80 |
| 17.32 | 5.12 | 3.01 |
| 17.95 | 4.94 | 1.81 |
| 18.52 | 4.79 | 15.73 |
| 19.34 | 4.59 | 4.02 |
| 19.67 | 4.51 | 12.69 |
| 19.85 | 4.47 | 16.75 |
| 20.39 | 4.36 | 11.96 |
| 20.97 | 4.24 | 4.56 |
| 21.61 | 4.11 | 2.27 |
| 21.80 | 4.08 | 2.77 |
| 22.29 | 3.99 | 5.39 |
| 23.13 | 3.85 | 1.51 |
| 23.83 | 3.73 | 0.55 |
| 24.31 | 3.66 | 2.87 |
| 24.89 | 3.58 | 1.41 |
| 25.84 | 3.45 | 2.32 |
| 26.15 | 3.41 | 2.00 |
| 26.38 | 3.38 | 2.03 |
| 26.74 | 3.33 | 1.41 |
| 27.33 | 3.26 | 1.68 |
| 28.10 | 3.18 | 2.39 |
| 29.33 | 3.05 | 0.86 |
| 30.33 | 2.95 | 1.11 |
| 30.83 | 2.90 | 1.75 |
| 31.89 | 2.81 | 1.33 |
| 32.27 | 2.77 | 1.20 |
| 33.20 | 2.70 | 0.73 |
| 33.65 | 2.66 | 0.80 |
| 37.24 | 2.41 | 0.63 |
| 38.04 | 2.37 | 0.77 |
| 38.33 | 2.35 | 0.41 |
;
for example, the X-ray powder diffraction pattern for the crystal form of the *p-*toluenesulfonate salt of the compound represented by formula I-3B using Cu-Kα radiation is substantially shown in FIG. 15;
(20) the crystal form of the p-toluenesulfonate salt of the compound represented by formula I-3B has a differential scanning calorimetry curve comprising an endothermic peak with a peak temperature at 278.9 ± 3°C;
for example, the differential scanning calorimetry curve for the crystal form of the *p-*toluenesulfonate salt of the compound represented by formula I-3B is shown in FIG. 16;
(21) the crystal form of the p-toluenesulfonate salt of the compound represented by formula I-3B has a thermogravimetric analysis curve with a weight loss of approximately 0.89% in the temperature range of 25.9 ± 3°C to 150 ± 3°C;
for example, the thermogravimetric analysis curve for the crystal form of the *p-*toluenesulfonate salt of the compound represented by formula I-3B is shown in FIG. 16;
(22) the X-ray powder diffraction pattern for the crystal form of the L-malate salt of the compound represented by formula I-3B, using Cu-Kα radiation and expressed by 2θ angles, comprises diffraction peaks shown in Table 8:
**Table 8**
| **Position 2θ (°)** | **Interplanar spacing d (Å)** | **Relative intensity (%)** |
|---|---|---|
| 8.12 | 10.89 | 15.08 |
| 10.15 | 8.72 | 6.65 |
| 10.83 | 8.17 | 23.00 |
| 14.74 | 6.01 | 11.51 |
| 15.81 | 5.60 | 100.00 |
| 16.06 | 5.52 | 36.81 |
| 16.66 | 5.32 | 19.86 |
| 17.20 | 5.16 | 38.24 |
| 17.60 | 5.04 | 32.06 |
| 18.03 | 4.92 | 4.64 |
| 18.85 | 4.71 | 62.30 |
| 19.38 | 4.58 | 4.10 |
| 19.99 | 4.44 | 9.39 |
| 20.34 | 4.37 | 67.92 |
| 20.62 | 4.31 | 44.60 |
| 20.93 | 4.24 | 6.93 |
| 21.77 | 4.08 | 50.66 |
| 22.04 | 4.03 | 47.94 |
| 23.61 | 3.77 | 5.80 |
| 23.88 | 3.73 | 12.69 |
| 24.19 | 3.68 | 10.90 |
| 24.40 | 3.65 | 12.88 |
| 24.67 | 3.61 | 7.51 |
| 25.47 | 3.50 | 38.90 |
| 25.86 | 3.45 | 24.01 |
| 26.65 | 3.34 | 3.19 |
| 27.45 | 3.25 | 13.63 |
| 27.86 | 3.20 | 8.01 |
| 28.34 | 3.15 | 13.20 |
| 29.11 | 3.07 | 20.41 |
| 29.95 | 2.98 | 5.19 |
| 30.71 | 2.91 | 16.36 |
| 31.12 | 2.87 | 3.23 |
| 31.77 | 2.82 | 4.40 |
| 32.40 | 2.76 | 5.70 |
| 32.90 | 2.72 | 5.86 |
| 33.64 | 2.66 | 3.17 |
| 34.14 | 2.63 | 6.57 |
| 35.61 | 2.52 | 3.62 |
| 36.93 | 2.43 | 2.46 |
;
for example, the X-ray powder diffraction pattern for the crystal form of the L-malate salt of the compound represented by formula I-3B using Cu-Kα radiation is substantially shown in FIG. 17;
(23) the crystal form of the L-malate salt of the compound represented by formula I-3B has a differential scanning calorimetry curve comprising an endothermic peak with an onset temperature at 120.4 ± 3°C, and/or the crystal form of the L-malate salt of the compound represented by formula I-3B has a differential scanning calorimetry curve comprising endothermic peaks with peak temperatures at 128.1 ± 3°C and 213.6 ± 3°C;
for example, the differential scanning calorimetry curve for the crystal form of the L-malate salt of the compound represented by formula I-3B is substantially shown in FIG. 18;
(24) the crystal form of the L-malate salt of the compound represented by formula I-3B has a thermogravimetric analysis curve with a weight loss of approximately 1.31% in the temperature range of 27.5 ± 3°C to 100 ± 3°C;
for example, the thermogravimetric analysis curve for the crystal form of the L-malate salt of the compound represented by formula I-3B is substantially shown in FIG. 18;
(25) the X-ray powder diffraction pattern for the crystal form A of the ethanedisulfonate salt of the compound represented by formula I-3B, using Cu-Kα radiation and expressed by 2θ angles, comprises diffraction peaks shown in Table 9:
**Table 9**
| **Position 2θ (°)** | **Interplanar spacing d (Å)** | **Relative intensity (%)** |
|---|---|---|
| 5.30 | 16.67 | 100.00 |
| 11.50 | 7.70 | 19.80 |
| 17.43 | 5.09 | 32.90 |
| 18.19 | 4.88 | 37.80 |
| 18.85 | 4.71 | 50.78 |
| 20.14 | 4.41 | 49.08 |
| 20.98 | 4.23 | 45.49 |
| 21.60 | 4.11 | 34.00 |
| 22.28 | 3.99 | 23.67 |
;
for example, the X-ray powder diffraction pattern for the crystal form A of the ethanedisulfonate salt of the compound represented by formula I-3B using Cu-Kα radiation is substantially shown in FIG. 19;
(26) the crystal form A of the ethanedisulfonate salt of the compound represented by formula I-3B has a differential scanning calorimetry curve comprising endothermic peaks with peak temperatures at 162.8 ± 3°C and 239.7 ± 3°C and an exothermic peak with a peak temperature at 198.8 ± 3°C;
for example, the differential scanning calorimetry curve for the crystal form A of the ethanedisulfonate salt of the compound represented by formula I-3B is substantially shown in FIG. 20;
(27) the crystal form A of the ethanedisulfonate salt of the compound represented by formula I-3B has a thermogravimetric analysis curve with a weight loss of approximately 9.08% in the temperature range of 25.2 ± 3°C to 170 ± 3°C;
for example, the thermogravimetric analysis curve for the crystal form A of the ethanedisulfonate salt of the compound represented by formula I-3B is substantially shown in FIG. 20;
(28) the X-ray powder diffraction pattern for the crystal form B of the ethanedisulfonate salt of the compound represented by formula I-3B, using Cu-Kα radiation and expressed by 2θ angles, comprises diffraction peaks shown in Table 10:
**Table 10**
| **Position 2θ (°)** | **Interplanar spacing d (Å)** | **Relative intensity (%)** |
|---|---|---|
| 6.29 | 14.05 | 28.10 |
| 10.95 | 8.08 | 19.58 |
| 12.59 | 7.03 | 12.58 |
| 13.39 | 6.61 | 11.12 |
| 15.08 | 5.88 | 29.77 |
| 17.03 | 5.21 | 15.46 |
| 17.68 | 5.02 | 36.27 |
| 18.56 | 4.78 | 100.00 |
| 19.41 | 4.57 | 74.23 |
| 20.37 | 4.36 | 21.88 |
| 20.81 | 4.27 | 26.86 |
| 21.64 | 4.11 | 56.26 |
| 22.11 | 4.02 | 34.78 |
| 23.30 | 3.82 | 16.52 |
| 23.73 | 3.75 | 9.86 |
| 25.01 | 3.56 | 6.66 |
| 27.35 | 3.26 | 8.72 |
| 27.95 | 3.19 | 11.98 |
| 29.21 | 3.06 | 3.18 |
| 38.73 | 2.32 | 3.42 |
for example, the X-ray powder diffraction pattern for the crystal form B of the ethanedisulfonate salt of the compound represented by formula I-3B using Cu-Kα radiation is substantially shown in FIG. 21;
(29) the crystal form B of the ethanedisulfonate salt of the compound represented by formula I-3B has a differential scanning calorimetry curve comprising an endothermic peak with an onset temperature at 235.8 ± 3°C, and/or the crystal form B of the ethanedisulfonate salt of the compound represented by formula I-3B has a differential scanning calorimetry curve comprising an endothermic peak with a peak temperature at 242.4 ± 3°C;
for example, the differential scanning calorimetry curve for the crystal form B of the ethanedisulfonate salt of the compound represented by formula I-3B is substantially shown in FIG. 22;
(30) the crystal form B of the ethanedisulfonate salt of the compound represented by formula I-3B has a thermogravimetric analysis curve with a weight loss of approximately 1.84% in the temperature range of 28.4 ± 3°C to 150 ± 3°C;
for example, the thermogravimetric analysis curve for the crystal form B of the ethanedisulfonate salt of the compound represented by formula I-3B is substantially shown in FIG. 22;
(31) the X-ray powder diffraction pattern for the crystal form C of the ethanedisulfonate salt of the compound represented by formula I-3B, using Cu-Kα radiation and expressed by 2θ angles, comprises diffraction peaks shown in Table 11:
**Table 11**
| **Position 2θ (°)** | **Interplanar spacing d (Å)** | **Relative intensity (%)** |
|---|---|---|
| 6.52 | 13.56 | 17.64 |
| 11.64 | 7.60 | 67.92 |
| 13.03 | 6.79 | 32.18 |
| 13.85 | 6.39 | 16.55 |
| 15.40 | 5.75 | 25.64 |
| 16.37 | 5.42 | 25.77 |
| 17.32 | 5.12 | 38.26 |
| 18.12 | 4.90 | 6.64 |
| 19.57 | 4.54 | 70.18 |
| 20.31 | 4.37 | 100.00 |
| 20.79 | 4.27 | 22.82 |
| 21.65 | 4.11 | 12.59 |
| 22.96 | 3.87 | 41.32 |
| 23.19 | 3.84 | 35.61 |
| 24.01 | 3.71 | 56.79 |
| 24.87 | 3.58 | 12.62 |
| 27.89 | 3.20 | 12.78 |
| 28.21 | 3.16 | 16.17 |
| 30.31 | 2.95 | 9.50 |
| 36.48 | 2.46 | 6.31 |
| 38.05 | 2.37 | 6.96 |
;
for example, the X-ray powder diffraction pattern for the crystal form C of the ethanedisulfonate salt of the compound represented by formula I-3B using Cu-Kα radiation is substantially shown in FIG. 23;
(32) the crystal form C of the ethanedisulfonate salt of the compound represented by formula I-3B has a differential scanning calorimetry curve comprising endothermic peaks with peak temperatures at 109.5 ± 3°C and 243.6 ± 3°C;
for example, the differential scanning calorimetry curve for the crystal form C of the ethanedisulfonate salt of the compound represented by formula I-3B is substantially shown in FIG. 24;
(33) the crystal form C of the ethanedisulfonate salt of the compound represented by formula I-3B has a thermogravimetric analysis curve with a weight loss of approximately 7.28% in the temperature range of 28.6 ± 3°C to 150 ± 3°C;
for example, the thermogravimetric analysis curve for the crystal form C of the ethanedisulfonate salt of the compound represented by formula I-3B is substantially shown in FIG. 24;
(34) the X-ray powder diffraction pattern for the crystal form of the methanesulfonate salt of the compound represented by formula I-3B, using Cu-Kα radiation and expressed by 2θ angles, comprises diffraction peaks shown in Table 12:
**Table 12**
| **Position 2θ (°)** | **Interplanar spacing d (Å)** | **Relative intensity (%)** |
|---|---|---|
| 6.49 | 13.62 | 83.98 |
| 7.90 | 11.19 | 31.16 |
| 8.40 | 10.53 | 13.41 |
| 9.35 | 9.46 | 100.00 |
| 10.49 | 8.43 | 14.90 |
| 12.21 | 7.25 | 18.51 |
| 12.94 | 6.84 | 11.36 |
| 15.27 | 5.80 | 28.32 |
| 15.60 | 5.68 | 32.77 |
| 16.40 | 5.41 | 19.92 |
| 16.89 | 5.25 | 29.66 |
| 18.20 | 4.88 | 41.42 |
| 18.60 | 4.77 | 34.88 |
| 19.90 | 4.46 | 44.96 |
| 20.52 | 4.33 | 26.59 |
| 21.83 | 4.07 | 36.70 |
| 22.20 | 4.00 | 30.38 |
| 23.82 | 3.74 | 10.75 |
| 24.90 | 3.58 | 8.82 |
| 25.95 | 3.43 | 9.43 |
| 27.21 | 3.28 | 11.58 |
| 29.70 | 3.01 | 5.24 |
| 30.52 | 2.93 | 7.31 |
;
for example, the X-ray powder diffraction pattern for the crystal form of the methanesulfonate salt of the compound represented by formula I-3B using Cu-Kα radiation is substantially shown in FIG. 25;
(35) the crystal form of the methanesulfonate salt of the compound represented by formula I-3B has a differential scanning calorimetry curve comprising an endothermic peak with an onset temperature at 116.3 ± 3°C, and/or the crystal form of the methanesulfonate salt of the compound represented by formula I-3B has a differential scanning calorimetry curve comprising an endothermic peak with a peak temperature at 124.3 ± 3°C;
for example, the differential scanning calorimetry curve for the crystal form of the methanesulfonate salt of the compound represented by formula I-3B is substantially shown in FIG. 26;
(36) the crystal form of the methanesulfonate salt of the compound represented by formula I-3B has a thermogravimetric analysis curve with a weight loss of approximately 4.53% in the temperature range of 28.4 ± 3°C to 80 ± 3°C;
for example, the thermogravimetric analysis curve for the crystal form of the methanesulfonate salt of the compound represented by formula I-3B is substantially shown in FIG. 26;
(37) the X-ray powder diffraction pattern for the crystal form of the benzenesulfonate salt of the compound represented by formula I-3B, using Cu-Kα radiation and expressed by 2θ angles, comprises diffraction peaks shown in Table 13:
**Table 13**
| **Position 2θ (°)** | **Interplanar spacing d (Å)** | **Relative intensity (%)** |
|---|---|---|
| 6.92 | 12.78 | 35.99 |
| 8.18 | 10.81 | 16.47 |
| 9.65 | 9.17 | 17.40 |
| 10.62 | 8.33 | 33.24 |
| 13.00 | 6.81 | 16.03 |
| 14.50 | 6.11 | 21.94 |
| 15.09 | 5.87 | 58.73 |
| 15.71 | 5.64 | 12.49 |
| 16.36 | 5.42 | 15.53 |
| 16.95 | 5.23 | 23.13 |
| 17.21 | 5.15 | 29.90 |
| 17.98 | 4.93 | 27.37 |
| 19.11 | 4.64 | 42.35 |
| 19.56 | 4.54 | 94.95 |
| 20.00 | 4.44 | 95.03 |
| 20.51 | 4.33 | 44.87 |
| 20.99 | 4.23 | 46.25 |
| 21.22 | 4.19 | 36.03 |
| 21.44 | 4.14 | 100.00 |
| 21.70 | 4.10 | 26.40 |
| 23.61 | 3.77 | 6.59 |
| 24.05 | 3.70 | 11.46 |
| 24.67 | 3.61 | 13.94 |
| 25.23 | 3.53 | 6.69 |
| 26.73 | 3.34 | 18.64 |
| 27.83 | 3.21 | 12.55 |
| 29.19 | 3.06 | 7.02 |
| 30.35 | 2.95 | 14.88 |
| 31.43 | 2.85 | 18.39 |
| 35.15 | 2.55 | 5.18 |
;
for example, the X-ray powder diffraction pattern for the crystal form of the benzenesulfonate salt of the compound represented by formula I-3B using Cu-Kα radiation is substantially shown in FIG. 27;
(38) the crystal form of the benzenesulfonate salt of the compound represented by formula I-3B has a differential scanning calorimetry curve comprising an endothermic peak with a peak temperature at 254.1 ± 3°C;
for example, the differential scanning calorimetry curve for the crystal form of the benzenesulfonate salt of the compound represented by formula I-3B is substantially shown in FIG. 28;
(39) the crystal form of the benzenesulfonate salt of the compound represented by formula I-3B has a thermogravimetric analysis curve with a weight loss of approximately 1.32% in the temperature range of 28.4 ± 3°C to 150 ± 3°C;
for example, the thermogravimetric analysis curve for the crystal form of the benzenesulfonate salt of the compound represented by formula I-3B is substantially shown in FIG. 28;
(40) the X-ray powder diffraction pattern for the crystal form of the oxalate salt of the compound represented by formula I-3B, using Cu-Kα radiation and expressed by 2θ angles, comprises diffraction peaks shown in Table 14:
**Table 14**
| **Position 2θ (°)** | **Interplanar spacing d (Å)** | **Relative intensity (%)** |
|---|---|---|
| 7.83 | 11.29 | 89.35 |
| 12.56 | 7.05 | 24.30 |
| 13.34 | 6.64 | 26.91 |
| 13.98 | 6.33 | 24.80 |
| 14.48 | 6.12 | 29.01 |
| 15.42 | 5.75 | 41.11 |
| 15.66 | 5.66 | 66.85 |
| 16.53 | 5.36 | 57.44 |
| 17.14 | 5.17 | 62.59 |
| 18.07 | 4.91 | 17.71 |
| 18.40 | 4.82 | 27.36 |
| 19.17 | 4.63 | 55.05 |
| 20.75 | 4.28 | 3.42 |
| 21.67 | 4.10 | 23.11 |
| 21.89 | 4.06 | 19.06 |
| 22.36 | 3.98 | 29.14 |
| 23.57 | 3.78 | 30.85 |
| 25.24 | 3.53 | 12.16 |
| 25.66 | 3.47 | 21.27 |
| 26.60 | 3.35 | 21.81 |
| 27.15 | 3.28 | 16.50 |
| 27.68 | 3.22 | 100.00 |
| 28.52 | 3.13 | 29.11 |
| 29.28 | 3.05 | 9.20 |
| 30.81 | 2.90 | 9.13 |
| 31.57 | 2.83 | 24.52 |
| 33.05 | 2.71 | 4.44 |
| 36.84 | 2.44 | 4.95 |
;
for example, the X-ray powder diffraction pattern for the crystal form of the oxalate salt of the compound represented by formula I-3B using Cu-Kα radiation is substantially shown in FIG. 29;
(41) the crystal form of the oxalate salt of the compound represented by formula I-3B has a differential scanning calorimetry curve comprising endothermic peaks with peak temperatures at 129.7 ± 3°C and 198.2 ± 3°C;
for example, the differential scanning calorimetry curve for the crystal form of the oxalate salt of the compound represented by formula I-3B is substantially shown in FIG. 30;
(42) the crystal form of the oxalate salt of the compound represented by formula I-3B has a thermogravimetric analysis curve with a weight loss of approximately 2.27% in the temperature range of 28.4 ± 3°C to 100 ± 3°C;
for example, the thermogravimetric analysis curve for the crystal form of the oxalate salt of the compound represented by formula I-3B is substantially shown in FIG. 30.

7. The crystal form according to claim 4, wherein the crystal form of the fumarate complex of the compound represented by formula I-3B has the following unit cell parameters: orthorhombic, space group of P2₁2₁2₁; a = 6.4400(4) Å, α = 90°, b = 11.9376(8) Å, β = 90°, c = 33.139(2) Å, γ = 90°, unit cell volume = 2547.7(3) Å³, number of asymmetric units in the unit cell Z = 4, crystal density of 1.451 mg/m³.

8. □ preparation method for the crystal form as defined in any one of claims 4 to 6, wherein the preparation method is method 1 or method 2:
method 1: the preparation method comprises the following steps: crystallizing a mixture of the compound represented by formula I-3B and a solvent to obtain a crystal form of the compound represented by formula I-3B or a solvate thereof;
in method 1, when the crystal form □ of the compound represented by formula I-3B is obtained, the solvent is isopropyl acetate;
when the crystal form B of the compound represented by formula I-3B is obtained, the solvent is one or more of methanol, water, acetonitrile, and methyl *tert*-butyl ether;
method 2: the preparation method comprises the following steps: crystallizing a mixture of the compound represented by formula I-3B, an acid, and a solvent;
in method 2, when the crystal form of the hydrochloride salt of the compound represented by formula I-3B is obtained, the acid is hydrochloric acid, and the solvent is acetone;
when the crystal form of the phosphate salt of the compound represented by formula I-3B is obtained, the acid is phosphoric acid, and the solvent is a mixture of acetone and *n*-heptane;
when the crystal form of the fumarate complex of the compound represented by formula I-3B is obtained, the acid is fumaric acid, and the solvent is a mixture of acetone and *n*-heptane;
when the crystal form of the L-tartrate salt of the compound represented by formula I-3B is obtained, the acid is tartaric acid, and the solvent is a mixture of acetone and *n*-heptane;
when the crystal form of the *p*-toluenesulfonate salt of the compound represented by formula I-3B is obtained, the acid is *p*-toluenesulfonic acid, and the solvent is a mixture of acetone and *n*-heptane;
when the crystal form of the L-malate salt of the compound represented by formula I-3B is obtained, the acid is malic acid, and the solvent is a mixture of acetone and *n*-heptane;
when the crystal form A of the ethanedisulfonate salt of the compound represented by formula I-3B is obtained, the acid is ethanedisulfonic acid, and the solvent is isopropanol;
when the crystal form B of the ethanedisulfonate salt of the compound represented by formula I-3B is obtained, the acid is ethanedisulfonic acid, and the solvent is isopropyl acetate;
when the crystal form C of the ethanedisulfonate salt of the compound represented by formula I-3B is obtained, the acid is ethanedisulfonic acid, and the solvent is a mixture of acetone and *n*-heptane;
when the crystal form of the methanesulfonate salt of the compound represented by formula I-3B is obtained, the acid is methanesulfonic acid, and the solvent is a mixture of acetone and *n*-heptane;
when the crystal form of the benzenesulfonate salt of the compound represented by formula I-3B is obtained, the acid is benzenesulfonic acid, and the solvent is isopropanol;
when the crystal form of the oxalate salt of the compound represented by formula I-3B is obtained, the acid is oxalic acid, and the solvent is a mixture of acetone and *n*-heptane;
preferably, the preparation method satisfies one or more of the following conditions:
(1) in the preparation method for the crystal form of the compound represented by formula I-3B, when the crystal form B of the compound represented by formula I-3B is obtained, the solvent is a mixture of methanol and water or a mixture of acetonitrile and methyl *tert*-butyl ether, preferably a mixture of methanol and water with a volume ratio of 1:5;
or, in the preparation method for the crystal form of the compound represented by formula I-3B, when the crystal form A of the compound represented by formula I-3B is obtained, the crystallizing involves cooling a mixture of isopropyl acetate and the compound represented by formula I-3B to room temperature, followed by drying to obtain the crystal form A of the compound represented by formula I-3B;
(2) in the preparation method for the crystal form of the compound represented by formula I-3B, when the crystal form B of the compound represented by formula I-3B is obtained, the crystallizing involves crystallizing a mixture of the compound represented by formula I-3B with methanol and water at 50°C, followed by drying to obtain the crystal form B of the compound represented by formula I-3B;
(3) in the preparation method, when the solvent is a mixture of acetone and n-heptane, the acetone and *n*-heptane have a volume ratio of 1:1;
(4) in the preparation method, the compound represented by formula I-3B and the acid have a molar ratio of 1:1; and
(5) the preparation method comprises the following steps: suspending the compound represented by formula I-3B, an acid, and a solvent to obtain a mixture, followed by crystallizing to obtain a crystal form of a pharmaceutically acceptable salt of the compound represented by formula I-3B;
preferably, in the preparation method, when the crystal form of the hydrochloride salt of the compound represented by formula I-3B, the crystal form of the L-malate salt of the compound represented by formula I-3B, the crystal form B of the ethanedisulfonate salt of the compound represented by formula I-3B, the crystal form C of the ethanedisulfonate salt of the compound represented by formula I-3B, the crystal form of the methanesulfonate salt of the compound represented by formula I-3B, or the crystal form of the benzenesulfonate salt of the compound represented by formula I-3B is obtained, the crystallizing comprises the following steps: subjecting the mixture of the compound represented by formula I-3B, the acid, and the solvent to temperature cycling, followed by drying;
or, in the preparation method, when the crystal form of the phosphate salt of the compound represented by formula I-3B, the crystal form of the fumarate complex of the compound represented by formula I-3B, the crystal form of the L-tartrate salt of the compound represented by formula I-3B, the crystal form of the *p*-toluenesulfonate salt of the compound represented by formula I-3B, the crystal form A of the ethanedisulfonate salt of the compound represented by formula I-3B, or the crystal form of the oxalate salt of the compound represented by formula I-3B is obtained, the crystallizing comprises the following steps: subjecting the mixture of the compound represented by formula I-3B, the acid, and the solvent to drying.

9. A pharmaceutical composition, wherein the pharmaceutical composition is any one of the following schemes:
scheme 1:
the pharmaceutical composition comprises the pharmaceutically acceptable salt of the heterocyclic compound or the solvate thereof as defined in any one of claims 1 to 3, and a pharmaceutically acceptable carrier;
scheme 2:
the pharmaceutical composition comprises a substance X and a pharmaceutically acceptable carrier, wherein the substance X is the crystal form A of the compound represented by formula I-3B, the crystal form B of the compound represented by formula I-3B, the crystal form of the hydrochloride salt of the compound represented by formula I-3B, the crystal form of the phosphate salt of the compound represented by formula I-3B, the crystal form of the fumarate complex of the compound represented by formula I-3B, the crystal form of the L-tartrate salt of the compound represented by formula I-3B, the crystal form of the *p-*toluenesulfonate salt of the compound represented by formula I-3B, the crystal form of the L-malate salt of the compound represented by formula I-3B, the crystal form A of the ethanedisulfonate salt of the compound represented by formula I-3B, the crystal form B of the ethanedisulfonate salt of the compound represented by formula I-3B, the crystal form C of the ethanedisulfonate salt of the compound represented by formula I-3B, the crystal form of the methanesulfonate salt of the compound represented by formula I-3B, the crystal form of the benzenesulfonate salt of the compound represented by formula I-3B, or the crystal form of the oxalate salt of the compound represented by formula I-3B as defined in any one of claims 4 to 7.

10. A use of a substance, wherein the substance is the pharmaceutically acceptable salt of the heterocyclic compound or the solvate thereof as defined in any one of claims 1 to 3, the pharmaceutical composition as defined in claim 9, or a substance X;
the substance X is the crystal form A of the compound represented by formula I-3B, the crystal form B of the compound represented by formula I-3B, the crystal form of the hydrochloride salt of the compound represented by formula I-3B, the crystal form of the phosphate salt of the compound represented by formula I-3B, the crystal form of the fumarate complex of the compound represented by formula I-3B, the crystal form of the L-tartrate salt of the compound represented by formula I-3B, the crystal form of the *p*-toluenesulfonate salt of the compound represented by formula I-3B, the crystal form of the L-malate salt of the compound represented by formula I-3B, the crystal form A of the ethanedisulfonate salt of the compound represented by formula I-3B, the crystal form B of the ethanedisulfonate salt of the compound represented by formula I-3B, the crystal form C of the ethanedisulfonate salt of the compound represented by formula I-3B, the crystal form of the methanesulfonate salt of the compound represented by formula I-3B, the crystal form of the benzenesulfonate salt of the compound represented by formula I-3B, or the crystal form of the oxalate salt of the compound represented by formula I-3B as defined in any one of claims 4 to 7;
the use comprises: preparing a 15-PGDH inhibitor; and/or preparing a medicament, pharmaceutical composition, or formulation for preventing and/or treating a disease related to 15-PGDH.

11. The use of the substance according to claim 10, wherein the disease related to 15-PGDH is one, two, or more of fibrotic disease, inflammatory disease, cardiovascular disease, trauma, autoimmune disease, graft-versus-host disease, hair growth, osteoporosis, ear disease, eye disease, neutropenia, diabetes, underactive bladder, implant promotion in stem cell or bone marrow transplantation or organ transplantation, neurogenesis and neuronal cell death, hematopoietic reconstruction, tissue injury, cervical disease, or kidney disease; preferably one or more of fibrotic disease, inflammatory disease, or tissue injury;
preferably, the disease related to 15-PGDH further satisfies one or more of the following conditions:
(1) the fibrotic disease is one or more of pulmonary fibrosis, liver fibrosis, renal fibrosis, myocardial fibrosis, scleroderma, or myelofibrosis, preferably pulmonary fibrosis and/or liver fibrosis, such as pulmonary fibrosis; for example, the pulmonary fibrosis may be idiopathic pulmonary fibrosis;
(2) the inflammatory disease is one or more of chronic obstructive pulmonary disease, acute lung injury, sepsis, exacerbation of asthma and pulmonary disease, inflammatory bowel disease, peptic ulcer, autoinflammatory disease, vasculitis syndrome, acute liver injury, acute kidney injury, non-alcoholic fatty liver disease, atopic dermatitis, psoriasis, interstitial cystitis, or prostatitis syndrome, preferably inflammatory bowel disease; for example, the inflammatory bowel disease may be ulcerative colitis and/or Crohn's disease; the peptic ulcer may be NSAID-induced ulcer; the autoinflammatory disease may be Behcet's disease; the prostatitis syndrome may be chronic prostatitis and/or chronic pelvic pain syndrome;
(3) the cardiovascular disease is one or more of pulmonary hypertension, angina, myocardial infarction, heart failure, ischemic heart disease, stroke, or peripheral circulatory disorder;
(4) the trauma is one or more of diabetic ulcer, burn, pressure ulcer, acute mucosal injury, including Stevens-Johnson syndrome, mucosal injury, injury related to an anticancer chemotherapeutic agent, or injury related to antimetabolites, cellular or humoral immunotherapy or radiation; for example, the anticancer chemotherapeutic agent may be one or more of an alkylating agent, a DNA synthesis inhibitor, or a DNA gyrase inhibitor;
(5) the autoimmune disease is multiple sclerosis and/or rheumatoid arthritis;
(6) the ear disease is one or more of hearing loss, tinnitus, vertigo, or balance disorder;
(7) the eye disease is glaucoma and/or dry eye;
(8) the neurogenesis and neuronal cell death is one or more of neuropsychiatric disorder, neuropathy, neurotoxic disease, neuropathic pain, or neurodegenerative disease;
(9) the tissue injury is liver injury and/or muscle injury; the muscle injury is preferably muscle atrophy and/or muscular dystrophy; and
(10) the kidney disease is chronic kidney disease and/or renal failure;
further preferably, the disease related to 15-PGDH is idiopathic pulmonary fibrosis, liver regeneration, liver injury, or inflammatory bowel disease.

12. A use of a substance, wherein the substance is the pharmaceutically acceptable salt of the heterocyclic compound or the solvate thereof as defined in any one of claims 1 to 3, the pharmaceutical composition as defined in claim 9, or a substance X;
the substance X is the crystal form A of the compound represented by formula I-3B, the crystal form B of the compound represented by formula I-3B, the crystal form of the hydrochloride salt of the compound represented by formula I-3B, the crystal form of the phosphate salt of the compound represented by formula I-3B, the crystal form of the fumarate complex of the compound represented by formula I-3B, the crystal form of the L-tartrate salt of the compound represented by formula I-3B, the crystal form of the *p*-toluenesulfonate salt of the compound represented by formula I-3B, the crystal form of the L-malate salt of the compound represented by formula I-3B, the crystal form A of the ethanedisulfonate salt of the compound represented by formula I-3B, the crystal form B of the ethanedisulfonate salt of the compound represented by formula I-3B, the crystal form C of the ethanedisulfonate salt of the compound represented by formula I-3B, the crystal form of the methanesulfonate salt of the compound represented by formula I-3B, the crystal form of the benzenesulfonate salt of the compound represented by formula I-3B, or the crystal form of the oxalate salt of the compound represented by formula I-3B as defined in any one of claims 4 to 7;
the use is for preparing a medicament for preventing or treating a disease as follows; the disease is one or more of fibrotic disease, inflammatory disease, or tissue injury;
for example, the fibrotic disease, the inflammatory disease, and the tissue injury are as described in claim 11.
